(19)

Europäisches Patentamt
European Patent Office
Office européen des brevets

(11) **EP 4 298 239 B1**

(12) **EUROPEAN PATENT SPECIFICATION**

(45) Date of publication and mention of the grant of the patent:
**05.11.2025 Bulletin 2025/45**

(21) Application number: **22713488.9**

(22) Date of filing: **23.02.2022**

(51) International Patent Classification (IPC):
**C12Q 1/6806** (2018.01)     **C12N 15/10** (2006.01)
**C40B 20/04** (2006.01)

(52) Cooperative Patent Classification (CPC):
(C-Sets available)
**C40B 20/04; C12N 15/1075; C12Q 1/6806**   (Cont.)

(86) International application number:
**PCT/US2022/017558**

(87) International publication number:
**WO 2022/182785 (01.09.2022 Gazette 2022/35)**

(54) **DRUG SCREENING METHODS**

ARZNEISTOFF-SCREENING-VERFAHREN

PROCÉDÉS DE CRIBLAGE DE MÉDICAMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.02.2021   US 202163152561 P**
**06.08.2021   US 202163230650 P**
**09.08.2021   US 202163231082 P**
**26.10.2021   US 202163271950 P**

(43) Date of publication of application:
**03.01.2024   Bulletin 2024/01**

(73) Proprietor: **10X Genomics, Inc.**
**Pleasanton, CA 94588-3260 (US)**

(72) Inventors:
• **WALTER, Dagmar**
  **Pleasanton, California 94588-3260 (US)**
• **TSAI, FuNien**
  **Pleasanton, California 94588-3260 (US)**
• **PHAM, Kristen Nguyen**
  **Pleasanton, California 94588-3260 (US)**

(74) Representative: **J A Kemp LLP**
**80 Turnmill Street**
**London EC1M 5QU (GB)**

(56) References cited:
**WO-A1-2019/113533     US-A1- 2020 002 764**

• **JAMES R. HEATH ET AL: "Single-cell analysis tools for drug discovery and development", NATURE REVIEWS DRUG DISCOVERY, vol. 15, no. 3, 16 December 2015 (2015-12-16), GB, pages 204 - 216, XP055556579, ISSN: 1474-1776, DOI: 10.1038/nrd.2015.16**
• **NACHIKET SHEMBEKAR ET AL: "Droplet-based microfluidics in drug discovery, transcriptomics and high-throughput molecular genetics", LAB ON A CHIP, vol. 16, no. 8, 1 January 2016 (2016-01-01), UK, pages 1314 - 1331, XP055451515, ISSN: 1473-0197, DOI: 10.1039/ C6LC00249H**

EP 4 298 239 B1

**(Cont. next page)**

(52) Cooperative Patent Classification (CPC): (Cont.)

C-Sets
**C12N 15/1075, C12Q 2535/122, C12Q 2537/159,
C12Q 2563/159, C12Q 2563/179, C12Q 2565/514;
C12Q 1/6806, C12Q 2535/122, C12Q 2537/159,
C12Q 2563/159, C12Q 2563/179, C12Q 2565/514**

## Description

### BACKGROUND

A sample may be processed for various purposes, such as identification of a type of moiety within the sample. The sample may be a biological sample. Biological samples may be processed, such as for detection of a disease (e.g., cancer) or identification of a particular species. There are various approaches for processing samples, such as polymerase chain reaction (PCR) and sequencing.

Biological samples may be processed within various reaction environments, such as partitions. Partitions may be wells or droplets. Droplets or wells may be employed to process biological samples in a manner that enables the biological samples to be partitioned and processed separately. For example, such droplets may be fluidically isolated from other droplets, enabling accurate control of respective environments in the droplets.

Biological samples in partitions may be subjected to various processes, such as chemical processes or physical processes. Samples in partitions may be subjected to heating or cooling, or chemical reactions, such as to yield species that may be qualitatively or quantitatively processed.

Biological molecules, such as nucleic acids and proteins, within biological samples may be probed and/or processed for quantitative or qualitative assessment.

US 2020/002764 discloses methods and systems for processing polynucleotides. WO 2019/113533 discloses methods and compositions for labelling cells. Heath et al. 2015 (Nature Reviews Drug Discovery Vol. 15, No. 3, 16, pages 204-216) discloses single-cell analysis tools for drug discovery and development. Shembekar et al. 2016 (Lab On A Chip, Vol. 16, No. 8, January, pages 1313-1331) discloses droplet-based microfluidics in drug discovery, transcriptomics and high-throughput molecular genetics.

### SUMMARY OF THE INVENTION

The present disclosure provides methods for use in sample processing and analysis.

The present invention provides a method for single cell drug screening comprising: (a) contacting a first sample of cells in a first partition with a first drug to provide first treated cells; (b) contacting a second sample of cells in a second partition with the first drug to provide second treated cells; (c) contacting the first treated cells with first labelling molecules to generate first labelled treated cells in the first partition, wherein the first labelling molecules comprise a plurality of first barcode sequences; (d) contacting the second treated cells with the first labelling molecules to generate second labelled treated cells in the second partition, wherein the first labelled treated cells in the first partition and the second labelled treated cells in the second partition comprise (i) a first barcode sequence of the plurality of first barcode sequences and (ii) a first plurality of cellular analytes; (e) contacting a third sample of cells in a third partition with a second drug to provide third treated cells; (f) contacting a fourth sample of cells in a fourth partition with the second drug to provide fourth treated cells; (g) contacting the third treated cells with second labelling molecules to generate third labelled treated cells in the third partition, wherein the second labelling molecules comprise a plurality of second barcode sequences; (h) contacting the fourth treated cells with the second labelling molecules to generate fourth labelled treated cells in the fourth partition, wherein the third labelled treated cells in the third partition and the fourth labelled treated cells in the fourth partition comprise (i) a second barcode sequence of the plurality of second barcode sequences and (ii) a second plurality of cellular analytes; (i) removing the first labelled treated cells from the first partition and the third labelled treated cells from the third partition at a first time point; (j) removing the second labelled treated cells from the second partition and the fourth labelled treated cells from the fourth partition at a second time point; (k) pooling the first labelled treated cells from the first partition and the third labelled treated cells from the third partition to provide a first pooled sample; and (l) pooling the second labelled treated cells from the second and the fourth labelled treated cells from the fourth partition to provide a second pooled sample.

In additional embodiments, the first plurality of cellular analytes and the second plurality of cellular analytes comprise macromolecular constituents. In additional embodiments, macromolecular constituents the first plurality of cellular analytes and the second plurality of cellular analytes comprise nucleic acid analytes. In additional embodiments, the nucleic acid analytes comprise ribonucleic acid (RNA) analytes. In additional embodiments, said RNA analytes comprise messenger RNA (mRNA) analytes.

In additional embodiments, the first labelling molecules comprise a lipophilic moiety. In additional embodiments, the first labelling molecules comprise a fluorophore, a cell-penetrating peptide, or a dye.

In additional embodiments, the first pooled sample comprises (i) a first labelled treated cell from the first partition, wherein the first labelled treated cell comprises the first barcode sequence and (ii) a third labelled treated cell from the third partition, wherein the third labelled treated cell comprises the second barcode sequence. In additional embodiments, the second pooled sample comprises (i) a second labelled treated cell from the second partition, wherein the second cell comprises the first barcode sequence and (ii) a fourth labelled treated cell from the fourth partition, wherein the fourth cell comprises the second barcode sequence. In additional embodiments, the first drug comprises a single drug or a combination of drugs. In additional embodiments, the second drug comprises a single drug or a combination of drugs.

In additional embodiments, the method further comprises contacting a fifth sample of cells in a fifth partition with a third drug to provide fifth treated cells and contacting a sixth sample of cells in a sixth partition with the third drug to provide sixth treated cells. In additional embodiments, the method further comprises contacting the fifth treated cells and the sixth treated cells with third labelling molecules to generate fifth labelled treated cells in the fifth partition and sixth labelled treated cells in the sixth partition, wherein the third labelling molecules comprise a plurality of third barcode sequences, and wherein said fifth labelled treated cells in the fifth partition and the sixth labelled cells in the sixth partition comprise (i) a third barcode sequence of the plurality of third barcode sequences and (ii) a third plurality of cellular analytes. In additional embodiments, the method further comprises removing the fifth labelled treated cells from the fifth partition and the sixth labelled treated cells from the sixth partition at a third time point. In additional embodiments, the method further comprises pooling the fifth labelled treated cells from the fifth partition with the first pooled sample. In additional embodiments, the method further comprises pooling the sixth labelled treated cells from the sixth partition with the second pooled sample.

In additional embodiments, any one or all of said first partition, second partition, third partition, or fourth partition is a well. In additional embodiments, the method further comprises partitioning (i) labelled treated cells from the first pooled sample and (ii) labelled treated cells from the second pooled sample into a plurality of partitions. In additional embodiments, the partitioning comprises partitioning into a plurality of droplets. In additional embodiments, the partitioning comprises partitioning into a plurality of wells. In additional embodiments, the plurality of partitions comprises labelled treated cells and nucleic acid barcode molecules.

In additional embodiments, a partition of the plurality of partitions comprises a particle comprising a plurality of nucleic acid barcode molecules and a labelled treated cell. In additional embodiments, said particle is a bead. In additional embodiments, the bead is a gel bead. In additional embodiments, the plurality of nucleic acid barcode molecules comprises capture sequences configured to couple to cellular analytes. In additional embodiments, the plurality of nucleic acid barcode molecules comprises capture sequences configured to generate barcoded nucleic acid molecules from the cellular analytes. In additional embodiments, said plurality of nucleic acid barcode molecules comprises partition barcode sequences specific for the partition.

In additional embodiments, the method further comprises generating a plurality of barcoded nucleic acid molecules, wherein a barcoded nucleic acid molecule of the plurality of barcoded nucleic acid molecules comprises: (i) a sequence corresponding to a cellular analyte, a partition barcode sequence and the first barcode sequence, thereby indicating that the labelled treated cell originates from the first partition or the second partition; or (ii) a sequence corresponding to a cellular analyte, a partition barcode sequence and the second barcode sequence, thereby indicating that the labelled treated cell originates from the third partition or the fourth partition.

In additional embodiments, the first sample of cells and/or the second sample of cells comprise perturbed cells. In additional embodiments, said perturbed cells comprise one or more edited genes. In additional embodiments, said one or more edited genes were edited by a CRISPR-Cas system.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

To the extent publications and patents or patent applications referenced contradict the disclosure contained in the specification, the specification is intended to supersede and/or take precedence over any such contradictory material.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

FIG. 1 shows an example of a microfluidic channel structure for partitioning individual biological particles.
FIG. 2 shows an example of a microfluidic channel structure for the controlled partitioning of beads into discrete droplets.
FIG. 3 illustrates an example of a barcode carrying bead.
FIG. 4 illustrates another example of a barcode carrying bead.
FIG. 5 schematically illustrates an example microwell array.
FIG. 6 schematically illustrates an example microwell array workflow for processing nucleic acid molecules.
FIG. 7 schematically illustrates example labelling agents with nucleic acid molecules attached thereto.

FIG. 8 schematically shows another example of a barcode-carrying bead.

FIG. 9A schematically shows an example of labelling agents. FIG. 9B schematically shows another example workflow for processing nucleic acid molecules. FIG. 9C schematically shows another example workflow for processing nucleic acid molecules.

FIG. 10 shows a computer system that is programmed or otherwise configured to implement methods provided herein.

FIG. 11 depicts the design of a single cell drug screening experiment.

FIG. 12 shows gene expression of various genes in response to drug treatment.

FIG. 13 depicts the design of a high-throughput single cell drug screening experiment

FIG. 14 shows gene expression of various genes in response to drug treatment.

FIG. 15 depicts a sample preparation approach based on a 96-well plate for biological particles, e.g., single cells or single nuclei. A sample of biological particles can be added to each well in a row wherein each row represents a different experimental condition.

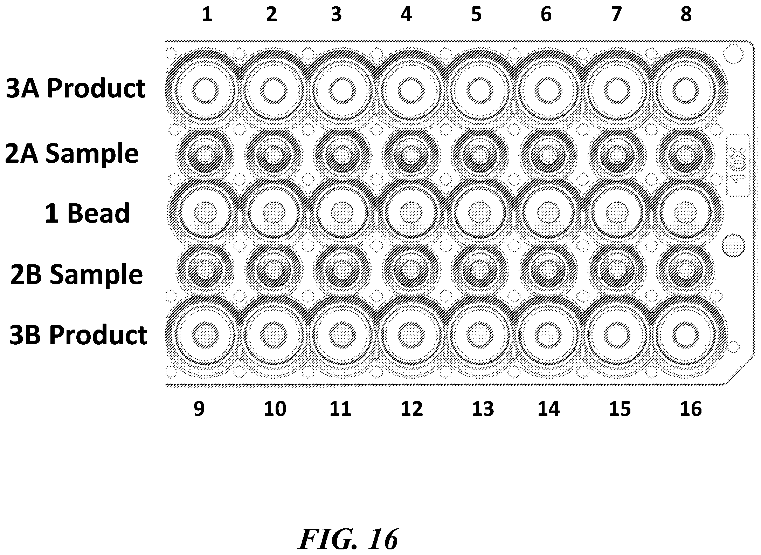

FIG. 16 depicts an example of a microfluidic chip for single cell or single nucleus analysis using barcode nucleic acid workflows. The chip has 16 different input wells for loading of up to 16 different samples (labeled Sample). Barcoded nucleic acid molecules, e.g., provided in droplets in an emulsion, generated from a sample loaded in the top left-most input well can be collected from the top left-most product well following an assay run, barcoded nucleic acid molecules, e.g., provided in droplets in an emulsion, generated from a sample loaded in the bottom left-most input well can be collected from the bottom left-most product well following an assay run, etc. The chip has 16 different product wells (labeled Product) for collection of barcoded nucleic acid molecules, e.g., provided in droplets in an emulsion.

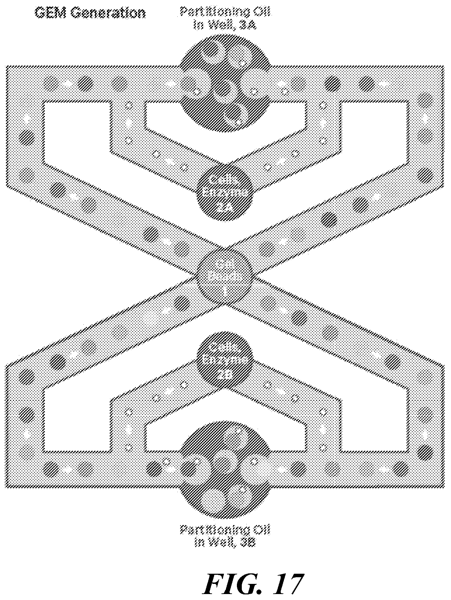

FIG. 17 depicts a exemplary channel architecture associated with a vertical column of wells from the microfluidic chip shown in FIG. 16. In particular, the channel paths associated with 1, 2A, 2B, 3A, and 3B are depicted.

FIG. 18 depicts an approach for removing products (e.g., barcoded nucleic acid molecules in a droplet in an emulsion) generated in wells from rows 3A and 3B of the microfluidic chip depicted in FIG. 16.

FIGs. 19A and 19B illustrate the responsiveness of A549 cells to drug treatments in the cell cycle and DNA repair pathways, respectively.

FIG. 20 illustrates gene expression patterns for different pathways.

FIG. 21 highlights gene expression for drug-treated samples.

FIG. 22 depicts cell clustering information for drug treated samples.

FIG. 23 shows gene expression for drug-treated samples.

FIG. 24A-B show dot plots for gene set enrichment using high-throughput chips and reagents (FIG. 24A) and standard chips and reagents (FIG. 24B). Standard chips and reagents are able to process 4-fold fewer cells compared to HT assays.

FIG. 25 shows a workflow for generating high-throughput drug screen data for 96 samples. Cells can be treated with single or combinatorial drugs for 4, 16, or 24 hours.

FIG. 26 shows a multiomic approach using Feature Barcode and CellPlex technology with single cell gene expression.

FIG. 27A-D demonstrate the different ways of clustering cell library data. Cells were clustered based on oligo labeling (FIG. 27A), cell type (FIG. 27B), donor (FIG. 27C), and gene expression data (FIG. 27D).

FIG. 28A-B show the difference between high-throughput methods (FIG. 28A) and standard assay methods (FIG. 28B).

FIG. 29 shows that high-throughput assays enable the detection of rare tumor populations.

FIG. 30A-C shows the difference between standard assays (FIG. 30C) and high-throughput assays (FIG. 30B) when attempting to identify rare tumor clusters. FIG. 30A is an aggregation of the high throughput and standard assay data.

FIG. 31A-B shows the difference between clustering based on treatment condition (FIG. 31A) and k-means clustering (FIG. 31B).

## DETAILED DESCRIPTION OF THE DISCLOSURE

The technical information set out below may in some respects go beyond the disclosure of the invention, which is defined by the appended claims. The additional technical information is provided to place the invention in a broader technical context and to illustrate possible related technical developments. Such additional technical information which does not fall within the scope of the appended claims is not part of the invention. Accordingly, any aspects, embodiments and examples of the present disclosure which do not fall under the scope of the appended claims do not form part of the invention and are merely provided for illustrative purposes. While various embodiments of the invention have been shown and described herein, it will be obvious to those skilled in the art that such embodiments are provided by way of example only. Numerous variations, changes, and substitutions may occur to those skilled in the art. It should be understood that various alternatives to the embodiments of the invention described herein may be employed.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

The terms "a," "an," and "the," as used herein, generally refers to singular and plural references unless the context clearly dictates otherwise.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

The term "barcode," as used herein, generally refers to a label, or identifier, that conveys or is capable of conveying information about an analyte. A barcode can be part of an analyte. A barcode can be independent of an analyte. A barcode can be a tag attached to an analyte (e.g., nucleic acid molecule) or a combination of the tag in addition to an endogenous characteristic of the analyte (e.g., size of the analyte or end sequence(s)). A barcode may be unique. Barcodes can have a variety of different formats. For example, barcodes can include: polynucleotide barcodes; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads.

The term "real time," as used herein, can refer to a response time of less than about 1 second, a tenth of a second, a hundredth of a second, a millisecond, or less. The response time may be greater than 1 second. Real time may refer to simultaneous or substantially simultaneous processing, detection or identification.

The term "subject," as used herein, generally refers to an animal, such as a mammal (e.g., human) or avian (e.g., bird), or other organism, such as a plant. For example, the subject can be a vertebrate, a mammal, a rodent (e.g., a mouse), a primate, a simian or a human. Animals may include, but are not limited to, farm animals, sport animals, and pets. A subject can be a healthy or asymptomatic individual, an individual that has or is suspected of having a disease (e.g., cancer) or a pre-disposition to the disease, and/or an individual that is in need of therapy or suspected of needing therapy. A subject can be a patient. A subject can be a microorganism or microbe (e.g., bacteria, fungi, archaea, viruses).

The term "genome," as used herein, generally refers to genomic information from a subject, which may be, for example, at least a portion or an entirety of a subject's hereditary information. A genome can be encoded either in DNA or in RNA. A genome can comprise coding regions (e.g., that code for proteins) as well as non-coding regions. A genome can include the sequence of all chromosomes together in an organism. For example, the human genome ordinarily has a total of 46 chromosomes. The sequence of all of these together may constitute a human genome.

The terms "adaptor(s)", "adapter(s)" and "tag(s)" may be used synonymously. An adaptor or tag can be coupled to a polynucleotide sequence to be "tagged" by any approach, including ligation, hybridization, or other approaches.

The term "sequencing," as used herein, generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (e.g., single stranded DNA). Sequencing can be performed by various systems currently available, such as, without limitation, a sequencing system by Illumina®, Pacific Biosciences (PacBio®), Oxford Nanopore®, or Life Technologies (Ion Torrent®). Alternatively or in addition, sequencing may be performed using nucleic acid amplification, polymerase chain reaction (PCR) (e.g., digital PCR, quantitative PCR, or real time PCR), or isothermal amplification. Such systems may provide a plurality of raw genetic data corresponding to the genetic information of a subject (e.g., human), as generated by the systems from a sample provided by the subject. In some examples, such systems provide sequencing reads (also "reads" herein). A read may include a string of nucleic acid bases corresponding to a sequence of a nucleic acid molecule that has been sequenced. In some situations, systems and methods provided herein may be used with proteomic information.

The term "bead," as used herein, generally refers to a particle. The bead may be a solid or semi-solid particle. The bead may be a gel bead. The gel bead may include a polymer matrix (e.g., matrix formed by polymerization or cross-linking). The polymer matrix may include one or more polymers (e.g., polymers having different functional groups or repeat units). Polymers in the polymer matrix may be randomly arranged, such as in random copolymers, and/or have ordered structures, such as in block copolymers. Cross-linking can be via covalent, ionic, or inductive, interactions, or physical entanglement. The bead may be a macromolecule. The bead may be formed of nucleic acid molecules bound together. The bead may be formed via covalent or non-covalent assembly of molecules (e.g., macromolecules), such as monomers or polymers. Such polymers or monomers may be natural or synthetic. Such polymers or monomers may be or include, for example, nucleic acid molecules (e.g., DNA or RNA). The bead may be formed of a polymeric material. The bead may be magnetic or non-magnetic. The bead may be rigid. The bead may be flexible and/or compressible. The bead may be disruptable or dissolvable. The bead may be a solid particle (e.g., a metal-based particle including but not limited to iron oxide, gold or silver) covered with a coating comprising one or more polymers. Such coating may be disruptable or dissolvable.

As used herein, the term "barcoded nucleic acid molecule" generally refers to a nucleic acid molecule that results from, for example, the processing of a nucleic acid barcode molecule with a nucleic acid sequence (e.g., nucleic acid sequence complementary to a nucleic acid primer sequence encompassed by the nucleic acid barcode molecule). The nucleic acid sequence may be a targeted sequence or a non-targeted sequence. For example, in the methods and systems described herein, hybridization and reverse transcription of a nucleic acid molecule (e.g., a messenger RNA (mRNA) molecule) of a cell with a nucleic acid barcode molecule (e.g., a nucleic acid barcode molecule containing a barcode sequence and a nucleic acid primer sequence complementary to a nucleic acid sequence of the mRNA molecule) results in a barcoded nucleic acid molecule that has a sequence corresponding to the nucleic acid sequence of the mRNA and the barcode sequence (or a reverse complement thereof). A barcoded nucleic acid molecule may serve as a template, such as a template polynucleotide, that can be further processed (e.g., amplified) and sequenced to obtain the target nucleic acid sequence. For example, in the methods and systems described herein, a barcoded nucleic acid molecule may be further processed (e.g., amplified) and sequenced to obtain the nucleic acid sequence of the mRNA.

The term "sample," as used herein, generally refers to a biological sample of a subject. The biological sample may comprise any number of macromolecules, for example, cellular macromolecules. The sample may be a cell sample. The sample may be a cell line or cell culture sample. The sample can include one or more cells. The sample can include one or more microbes. The biological sample may be a nucleic acid sample or protein sample. The biological sample may also be a carbohydrate sample or a lipid sample. The biological sample may be derived from another sample. The sample may be a tissue sample, such as a biopsy, core biopsy, needle aspirate, or fine needle aspirate. The sample may be a fluid sample, such as a blood sample, urine sample, or saliva sample. The sample may be a skin sample. The sample may be a cheek swab. The sample may be a plasma or serum sample. The sample may be a cell-free or cell free sample. A cell-free sample may include extracellular polynucleotides. Extracellular polynucleotides may be isolated from a bodily sample that may be selected from the group consisting of blood, plasma, serum, urine, saliva, mucosal excretions, sputum, stool and tears.

The term "biological particle," as used herein, generally refers to a discrete biological system derived from a biological sample. The biological particle may be a macromolecule. The biological particle may be a small molecule. The biological particle may be a virus. The biological particle may be a cell or derivative of a cell. The biological particle may be an organelle. The biological particle may be a rare cell from a population of cells. The biological particle may be any type of cell, including without limitation prokaryotic cells, eukaryotic cells, bacterial, fungal, plant, mammalian, or other animal cell type, mycoplasmas, normal tissue cells, tumor cells, or any other cell type, whether derived from single cell or multicellular organisms. The biological particle may be a constituent of a cell. The biological particle may be or may include DNA, RNA, organelles, proteins, or any combination thereof. The biological particle may be or may include a matrix (e.g., a gel or polymer matrix) comprising a cell or one or more constituents from a cell (e.g., cell bead), such as DNA, RNA, organelles, proteins, or any combination thereof, from the cell. The biological particle may be obtained from a tissue of a subject. The biological particle may be a hardened cell. Such hardened cell may or may not include a cell wall or cell membrane. The biological particle may include one or more constituents of a cell, but may not include other constituents of the cell. An example of such constituents is a nucleus or an organelle. A cell may be a live cell. The live cell may be capable of being cultured, for example, being cultured when enclosed in a gel or polymer matrix, or cultured when comprising a gel or polymer matrix. The biological particle may be an analyte carrier.

The term "macromolecular constituent," as used herein, generally refers to a macromolecule contained within or from a biological particle. The macromolecular constituent may comprise a nucleic acid. In some cases, the biological particle may be a macromolecule. The macromolecular constituent may comprise DNA. The macromolecular constituent may comprise RNA. The RNA may be coding or non-coding. The RNA may be messenger RNA (mRNA), ribosomal RNA (rRNA) or transfer RNA (tRNA), for example. The RNA may be a transcript. The RNA may be small RNA that are less than 200 nucleic acid bases in length, or large RNA that are greater than 200 nucleic acid bases in length. Small RNAs may include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA may be double-stranded RNA or single-stranded RNA. The RNA may be circular RNA. The macromolecular constituent may comprise a protein. The macromolecular constituent may comprise a peptide. The macromolecular constituent may comprise a polypeptide.

The term "molecular tag," as used herein, generally refers to a molecule capable of binding to a macromolecular constituent. The molecular tag may bind to the macromolecular constituent with high affinity. The molecular tag may bind to the macromolecular constituent with high specificity. The molecular tag may comprise a nucleotide sequence. The molecular tag may comprise a nucleic acid sequence. The nucleic acid sequence may be at least a portion or an entirety of the molecular tag. The molecular tag may be a nucleic acid molecule or may be part of a nucleic acid molecule. The molecular tag may be an oligonucleotide or a polypeptide. The molecular tag may comprise a DNA aptamer. The molecular tag may be or comprise a primer. The molecular tag may be, or comprise, a protein. The molecular tag may comprise a polypeptide. The molecular tag may be a barcode.

The term "partition," as used herein, generally, refers to a space or volume that may be suitable to contain one or more species or conduct one or more reactions. A partition may be a physical compartment, such as a droplet or well. The partition may isolate space or volume from another space or volume. The droplet may be a first phase (e.g., aqueous phase) in a second phase (e.g., oil) immiscible with the first phase. The droplet may be a first phase in a second phase that does not phase separate from the first phase, such as, for example, a capsule or liposome in an aqueous phase. A partition may comprise one or more other (inner) partitions. In some cases, a partition may be a virtual compartment that can be defined and identified by an index (e.g., indexed libraries) across multiple and/or remote physical compartments. For example, a physical compartment may comprise a plurality of virtual compartments.

The nucleic acid molecule or a derivative thereof or a cell comprising the nucleic acid molecule or a derivative thereof (e.g., a cell bead) may be provided within a partition such as a well or droplet, e.g., as described herein. One or more reagents may be co-partitioned with a nucleic acid molecule or a derivative thereof or a cell comprising the nucleic acid molecule or a derivative thereof. For example, a nucleic acid molecule or a derivative thereof or a cell comprising the nucleic acid molecule or a derivative thereof may be co-partitioned with one or more reagents selected from the group consisting of lysis agents or buffers, permeabilizing agents, enzymes (e.g., enzymes capable of digesting one or more RNA molecules, extending one or more nucleic acid molecules, reverse transcribing an RNA molecule, permeabilizing or lysing a cell, or carrying out other actions), fluorophores, oligonucleotides, primers, probes, barcodes, nucleic acid barcode molecules (e.g., nucleic acid barcode molecules comprising one or more barcode sequences), buffers, deoxynucleotide triphosphates, detergents, reducing agents, chelating agents, oxidizing agents, nanoparticles, beads, and antibodies. In some cases, a nucleic acid molecule or a derivative thereof, or a cell comprising the nucleic acid molecule or a derivative thereof (e.g., a cell bead), may be co-partitioned with one or more reagents selected from the group consisting of temperature-sensitive enzymes, pH-sensitive enzymes, light-sensitive enzymes, reverse transcriptases, proteases, ligase, polymerases, restriction enzymes, nucleases, protease inhibitors, exonucleases, and nuclease inhibitors. For example, a nucleic acid molecule or a derivative thereof or a cell comprising the nucleic acid molecule or a derivative thereof may be co-partitioned with a polymerase and nucleotide molecules. Partitioning a nucleic acid molecule or a derivative thereof or a cell comprising the nucleic acid molecule or a derivative thereof and one or more reagents may comprise flowing a first phase comprising an aqueous fluid, the cell, and the one or more reagents and a second phase comprising a fluid that is immiscible with the aqueous fluid toward a junction. Upon interaction of the first and second phases, a discrete droplet of the first phase comprising the nucleic acid molecule or a derivative thereof or a cell comprising the nucleic acid molecule or a derivative thereof (e.g., a cell bead) and the one or more reagents may be formed. In some cases, the partition may comprise a single cell. The cell may be lysed or permeabilized within the partition (e.g., droplet) to provide access to the nucleic acid molecule of the cell.

Similarly, the nucleic acid molecule or the cell or cell bead comprising the nucleic acid molecule, or derivatives thereof may be released from a partition at any useful stage of the method. Duplication and/or amplification of the extended nucleic acid molecule may be carried out within the partition or in bulk, e.g., within a solution. In some cases, the solution may comprise additional extended nucleic acid molecules generated through the same process carried out in different partitions. Each extended nucleic acid molecule may comprise a different barcode sequence, and the barcode sequence may be useful in identifying the partition or cell from whence the extended nucleic acid molecules originated. In such cases, the solution may comprise a pooled mixture comprising the contents of two or more partitions (e.g., droplets).

Additional processes or operations may be performed within a partition, including, but not limited to: lysis, permeabilization, denaturation, hybridization, extension, duplication, and amplification of one or more components of a sample. In some cases, multiple processes are carried out within a partition.

Following partition-based barcoding, the contents of the partitions may be pooled and the barcoded molecules may be duplicated or amplified by, for example, one or more amplification reactions, which may be isothermal. The amplification reactions may comprise polymerase chain reactions (PCR) and may involve the use of one or more primers or polymerases. The one or more primers may comprise one or more functional sequences (e.g., a primer sequence/primer binding sequence, a sequencing primer sequence (e.g., R1 or R2), a partial sequencing primer sequence (e.g., partial R1 or partial R2), a sequence configured to attach to the flow cell of a sequencer (e.g., P5 or P7, or partial sequences thereof), etc.) and may facilitate addition of said one or more functional sequences to the extended nucleic acid molecule. The barcoded molecules, or derivatives thereof, may be detected via nucleic acid sequencing (e.g., as described herein).

Disclosed herein are systems useful for barcoding nucleic acid molecules. The systems may comprise any of the components described herein, e.g., a plurality of partitions (e.g., droplets, wells), which may be provided in any useful format, e.g., a microfluidic device, a multi-well array or plate, etc. The systems may include nucleic acid barcode molecules, optionally coupled to microcapsules and/or supports (e.g., particles, beads, gel beads, etc.). The systems may comprise any of the nucleic acid barcode molecules described herein, and any useful reaction components (e.g., for performing a nucleic acid reaction, e.g., extension, ligation, amplification, etc.). Such useful reaction components can include, in non-limiting examples, enzymes (e.g., ligases, polymerases, reverse transcriptases, restriction enzymes, etc.),

nucleotides bases, etc.

Also disclosed herein are compositions useful for systems and methods for barcoding nucleic acid molecules. A composition may comprise any of the nucleic acid barcode molecules described herein. A composition may comprise nucleic acid barcode molecules, which may optionally be provided coupled to a microcapsule and/or support (e.g., particle, bead). A composition may be a part of or comprise a reaction mixture, which can include reaction components or reagents, e.g., enzymes, nucleotide bases, catalysts, etc.

## Multiplexed analysis of nucleic acids and proteins

The present disclosure provides methods for performing multiplexed assays. Such a multiplexed assay may comprise assaying or analyzing one or more biomolecules (e.g., nucleic acid molecules, proteins, lipids, carbohydrates, etc.). A method may comprise using one or more macromolecular constituents described herein and a nucleic acid barcode molecule to barcode a nucleic acid molecule of a cell, thereby generating a first barcoded nucleic acid molecule. One or more operations may be performed within a partition (e.g., droplet or well).

The methods described herein may facilitate profiling of one or more biomolecules with single-cell resolution, using, for example, feature binding groups (e.g., antibodies, antibody fragments, epitope-binding groups, etc.), barcoding, amplification, and sequencing. The methods may be useful in providing genomic, transcriptomic, proteomic, exomic, or other "-omic" information from a single cell. As described herein, the methods may be used to analyze a pre-determined panel of target genes and a pre-determined panel of target features (e.g., proteins, peptides, or other biomolecules) in a sensitive and accurate manner.

The feature binding group may comprise a labelling agent, as described elsewhere herein. Accordingly, the feature binding group may comprise, in some examples, an antibody or antibody fragment, an epitope binding moiety, a protein, a peptide, a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof.

In addition to barcoding of nucleic acid molecules, the present disclosure provides for methods of multiplexed analysis, e.g., processing of additional biomolecule types, such as proteins and peptides. The method may comprise providing a feature-binding group (e.g., antibody, protein, binding moiety, etc.), which may couple to or bind to a feature (e.g., protein, peptide) of a cell or cell bead. Such a method may comprise providing a cell or cell bead having a feature of interest (e.g., protein) and contacting the cell or cell bead with the feature-binding group. The feature-binding group may couple to the feature of interest. The feature-binding group may comprise a reporter oligonucleotide comprising a reporter sequence coupled thereto, which may be specific for a particular feature and thus be used to identify the feature. For example, the feature-binding group may be an antibody and the reporter oligonucleotide may comprise a reporter sequence that identifies the antigen or binding moiety (e.g., epitope, epitope fragment) to which the antibody couples or binds.

As described herein, one or more processes described herein may be performed in a cell or cell bead. For example, a plurality of cells or cell beads may comprise a plurality of nucleic acid molecules and features. The cells or cell beads may be alive or fixed and/or permeabilized. The cells or cell beads may be contacted with a feature binding group comprising a reporter oligonucleotide, which comprises a reporter sequence. Subsequently, the cells or cell beads comprising the macromolecular constituents may be partitioned into a plurality of separate partitions, where at least a subset of the plurality of separate partitions comprises a single cell. Barcoding may be performed within the separate partitions. The nucleic acid barcode molecules provided within each partition of the plurality of separate partitions may be provided attached to beads. As described elsewhere herein, the nucleic acid barcode molecule may be releasably attached to a bead (e.g., via a labile bond). Each partition (or a subset of partitions) of the plurality of separate partitions may comprise a bead comprising a plurality of nucleic acid barcode molecules attached thereto (e.g., as described herein). The plurality of nucleic acid barcode molecules attached to each bead may comprise a unique barcode sequence, such that each partition of the plurality of separate partitions comprises a different barcode sequence. Upon release of components from the plurality of different partitions of the plurality of separate partitions (e.g., following barcoding), the barcoded molecules arising from a single cell may have a same barcode sequence (e.g., a common barcode sequence), such that each barcoded nucleic acid molecule may be traced to a given partition and/or, a given cell or cell bead. The released components may then be partitioned, as described herein, in a second set of partitions comprising capture molecules with a second barcode sequence, such that different partitions of the second set of partitions have a unique second barcode sequence.

Any number of barcoding operations may be performed for a given nucleic acid molecule and/or feature binding group. As described herein, additional barcoding operations may be useful, for example, in indexing nucleic acid molecules and features (e.g., proteins) to a cell, a sample, a partition, or a plurality of partitions. Such indexing may be useful in situations when a single partition is occupied by multiple cells. It may be beneficial to overload partitions such that a partition comprises more than a cell or cell bead; for example, it may be useful in certain situations to overload partitions, e.g., to overcome Poisson loading statistics in partitions and/or to prevent reagent waste (e.g., from unoccupied partitions). Accordingly, such indexing may be useful in attributing (i) nucleic acid molecules and (ii) features (e.g., proteins) in multiply-occupied partitions to the originating cell, partition, sample, etc., as is described elsewhere herein.

Following partition-based barcoding, the contents of the partitions may be pooled and the barcoded molecules may be duplicated or amplified by, for example, one or more amplification reactions, which may be isothermal. The amplification reactions may comprise polymerase chain reactions (PCR) and may involve the use of one or more primers or polymerases. The one or more primers may comprise one or more functional sequences (e.g., a primer sequence/-primer binding sequence, a sequencing primer sequence (e.g., R1 or R2), a partial sequencing primer sequence (e.g., partial R1 or partial R2), a sequence configured to attach to the flow cell of a sequencer (e.g., P5 or P7, or partial sequences thereof), etc.) and may facilitate addition of said one or more functional sequences to the extended nucleic acid molecule. The barcoded molecules, or derivatives thereof, may be detected via nucleic acid sequencing (e.g., as described herein).

Disclosed herein are systems useful for barcoding nucleic acid molecules. The systems may comprise any of the components described herein, e.g., a plurality of partitions (e.g., droplets, wells), which may be provided in any useful format, e.g., a microfluidic device, a multi-well array or plate, etc. The system may comprise a first set of partitions and a second set of partitions. The first set of partitions may be the same or different types of partitions as the second set of partitions. For example, the first set of partitions may comprise microwells and the second set of partitions may comprise droplets. As another example, both the first set of partitions and the second set of partitions may comprise droplets. The systems may include nucleic acid barcode molecules, optionally coupled to microcapsules and/or supports (e.g., particles, beads, gel beads, etc.). The systems may comprise one or more feature-binding groups. The feature binding groups may be the same or different across partitions; for example, the feature binding groups may comprise a variety of antibodies that bind to different epitopes within a single partition, or the partitions may comprise different feature binding groups that bind to different epitopes or moieties. The systems may include reaction components that are useful, such as, in non-limiting examples, enzymes (e.g., ligases, polymerases, reverse transcriptases, restriction enzymes, etc.), nucleotides bases, etc.

## Systems and methods for sample compartmentalization

The systems and methods described herein provide for the compartmentalization, depositing, or partitioning of one or more particles (e.g., biological particles, analyte carriers, macromolecular constituents of biological particles, beads, reagents, etc.) into discrete compartments or partitions (referred to interchangeably herein as partitions), where each partition maintains separation of its own contents from the contents of other partitions. The partition can be a droplet in an emulsion or a well. A partition may comprise one or more other partitions.

A partition may include one or more particles. A partition may include one or more types of particles. For example, a partition of the present disclosure may comprise one or more biological particles and/or macromolecular constituents thereof. A partition may comprise one or more beads. A partition may comprise one or more gel beads. A partition may comprise one or more cell beads. A partition may include a single gel bead, a single cell bead, or both a single cell bead and single gel bead. A partition may include one or more reagents. Alternatively, a partition may be unoccupied. For example, a partition may not comprise a bead. A cell bead can be a biological particle and/or one or more of its macromolecular constituents encased inside of a gel or polymer matrix, such as via polymerization of a droplet containing the analyte carrier and/or biological particle and precursors capable of being polymerized or gelled. Unique identifiers, such as barcodes, may be injected into the droplets previous to, subsequent to, or concurrently with droplet generation, such as via a microcapsule and/or support (e.g., bead), as described elsewhere herein.

The methods and systems of the present disclosure may comprise methods and systems for generating one or more partitions such as droplets. The droplets may comprise a plurality of droplets in an emulsion. In some examples, the droplets may comprise droplets in a colloid. In some cases, the emulsion may comprise a microemulsion or a nanoemulsion. In some examples, the droplets may be generated with aid of a microfluidic device and/or by subjecting a mixture of immiscible phases to agitation (e.g., in a container). In some cases, a combination of the mentioned methods may be used for droplet and/or emulsion formation.

Droplets can be formed by creating an emulsion by mixing and/or agitating immiscible phases. Mixing or agitation may comprise various agitation techniques, such as vortexing, pipetting, tube flicking, or other agitation techniques. In some cases, mixing or agitation may be performed without using a microfluidic device. In some examples, the droplets may be formed by exposing a mixture to ultrasound or sonication. Systems and methods for droplet and/or emulsion generation by agitation are described in WO2020/167862A1.

Microfluidic devices or platforms comprising microfluidic channel networks (e.g., on a chip) can be utilized to generate partitions such as droplets and/or emulsions as described herein. Methods and systems for generating partitions such as droplets, methods of encapsulating analyte carriers and/or biological particles, methods of increasing the throughput of droplet generation, and various geometries, architectures, and configurations of microfluidic devices and channels are described in U.S. Patent Publication Nos. 2019/0367997 and 2019/0064173.

In some examples, individual particles can be partitioned to discrete partitions by introducing a flowing stream of particles in an aqueous fluid into a flowing stream or reservoir of a non-aqueous fluid, such that droplets may be generated at the junction of the two streams/reservoir, such as at the junction of a microfluidic device provided elsewhere herein.

The methods of the present disclosure may comprise generating partitions and/or encapsulating particles, such as analyte carriers and/or biological particles, in some cases, individual biological particles such as single cells. In some examples, reagents may be encapsulated and/or partitioned (e.g., co-partitioned with analyte carriers and/or biological particles) in the partitions. Various mechanisms may be employed in the partitioning of individual particles. An example may comprise porous membranes through which aqueous mixtures of cells may be extruded into fluids (e.g., non-aqueous fluids).

The partitions can be flowable within fluid streams. The partitions may comprise, for example, micro-vesicles that have an outer barrier surrounding an inner fluid center or core. In some cases, the partitions may comprise a porous matrix that is capable of entraining and/or retaining materials within its matrix. The partitions can be droplets of a first phase within a second phase, wherein the first and second phases are immiscible. For example, the partitions can be droplets of aqueous fluid within a non-aqueous continuous phase (e.g., oil phase). In another example, the partitions can be droplets of a non-aqueous fluid within an aqueous phase. In some examples, the partitions may be provided in a water-in-oil emulsion or oil-in-water emulsion. A variety of different vessels are described in, for example, U.S. Patent Application Publication No. 2014/0155295. Emulsion systems for creating stable droplets in non-aqueous or oil continuous phases are described in, for example, U.S. Patent Application Publication No. 2010/0105112.

Fluid properties (e.g., fluid flow rates, fluid viscosities, etc.), particle properties (e.g., volume fraction, particle size, particle concentration, etc.), microfluidic architectures (e.g., channel geometry, etc.), and other parameters may be adjusted to control the occupancy of the resulting partitions (e.g., number of analyte carriers and/or biological particles per partition, number of beads per partition, etc.). For example, partition occupancy can be controlled by providing the aqueous stream at a certain concentration and/or flow rate of particles. To generate single analyte carrier and/or biological particle partitions, the relative flow rates of the immiscible fluids can be selected such that, on average, the partitions may contain less than one analyte carrier and/or biological particle per partition in order to ensure that those partitions that are occupied are primarily singly occupied. In some cases, partitions among a plurality of partitions may contain at most one analyte carrier or biological particle (e.g., bead, DNA, cell or cellular material). The various parameters (e.g., fluid properties, particle properties, microfluidic architectures, etc.) may be selected or adjusted such that a majority of partitions are occupied, for example, allowing for only a small percentage of unoccupied partitions. The flows and channel architectures can be controlled as to ensure a given number of singly occupied partitions, less than a certain level of unoccupied partitions and/or less than a certain level of multiply occupied partitions.

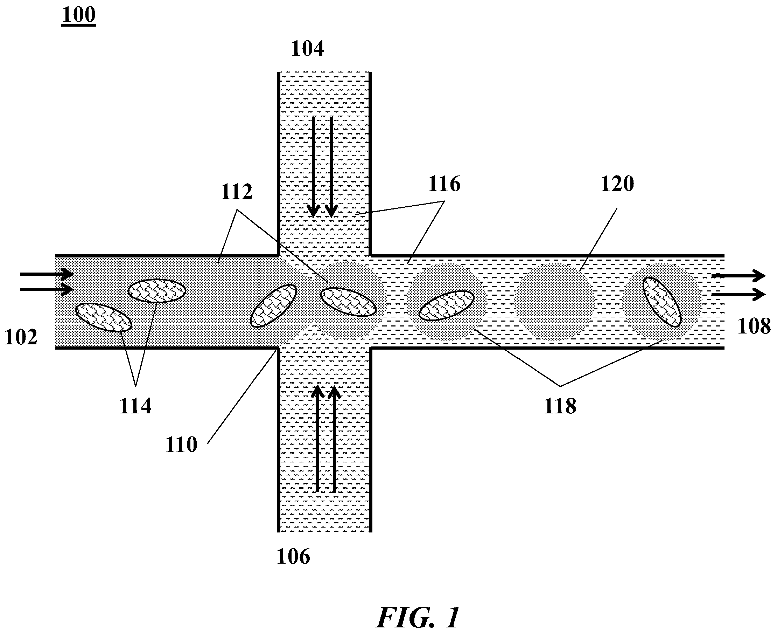

FIG. 1 shows an example of a microfluidic channel structure 100 for partitioning individual analyte carriers and/or biological particles. The channel structure 100 can include channel segments 102, 104, 106 and 108 communicating at a channel junction 110. In operation, a first aqueous fluid 112 that includes suspended analyte carriers and/or biological particles (or cells) 114 may be transported along channel segment 102 into junction 110, while a second fluid 116 that is immiscible with the aqueous fluid 112 is delivered to the junction 110 from each of channel segments 104 and 106 to create discrete droplets 118, 120 of the first aqueous fluid 112 flowing into channel segment 108, and flowing away from junction 110. The channel segment 108 may be fluidically coupled to an outlet reservoir where the discrete droplets can be stored and/or harvested. A discrete droplet generated may include an individual analyte carrier and/or biological particle 114 (such as droplets 118). A discrete droplet generated may include more than one individual analyte carrier and/or biological particle 114 (not shown in FIG. 1). A discrete droplet may contain no analyte carrier and/or biological particle 114 (such as droplet 120). Each discrete partition may maintain separation of its own contents (e.g., individual biological particle 114) from the contents of other partitions.

The second fluid 116 can comprise an oil, such as a fluorinated oil, that includes a fluorosurfactant for stabilizing the resulting droplets, for example, inhibiting subsequent coalescence of the resulting droplets 118, 120. Examples of particularly useful partitioning fluids and fluorosurfactants are described, for example, in U.S. Patent Application Publication No. 2010/0105112.

The second fluid 116 can comprise an oil, such as a fluorinated oil, that includes a fluorosurfactant for stabilizing the resulting droplets, for example, inhibiting subsequent coalescence of the resulting droplets 118, 120. Examples of particularly useful partitioning fluids and fluorosurfactants are described, for example, in U.S. Patent Application Publication No. 2010/0105112.

As will be appreciated, the channel segments described herein may be coupled to any of a variety of different fluid sources or receiving components, including reservoirs, tubing, manifolds, or fluidic components of other systems. As will be appreciated, the microfluidic channel structure 100 may have other geometries. For example, a microfluidic channel structure can have more than one channel junction. For example, a microfluidic channel structure can have 2, 3, 4, or 5 channel segments each carrying particles (e.g., analyte carriers, biological particles, cell beads, and/or gel beads) that meet at a channel junction. Fluid may be directed to flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

The generated droplets may comprise two subsets of droplets: (1) occupied droplets **118,** containing one or more analyte carriers and/or biological particles **114,** and (2) unoccupied droplets **120,** not containing any analyte carriers and/or biological particles **114.** Occupied droplets **118** may comprise singly occupied droplets (having one analyte carrier or biological particle) and multiply occupied droplets (having more than one analyte carrier and/or biological particle). As described elsewhere herein, in some cases, the majority of occupied partitions can include no more than one analyte carrier and/or biological particle per occupied partition and some of the generated partitions can be unoccupied (of any analyte carrier and/or biological particle). In some cases, though, some of the occupied partitions may include more than one analyte carrier and/or biological particle. In some cases, the partitioning process may be controlled such that fewer than about 25% of the occupied partitions contain more than one analyte carrier and/or biological particle, and in many cases, fewer than about 20% of the occupied partitions have more than one analyte carrier and/or biological particle, while in some cases, fewer than about 10% or even fewer than about 5% of the occupied partitions include more than one analyte carrier and/or biological particle per partition.

In some cases, it may be desirable to minimize the creation of excessive numbers of empty partitions, such as to reduce costs and/or increase efficiency. While this minimization may be achieved by providing a sufficient number of analyte carrier and/or biological particles (e.g., biological particles **114**) at the partitioning junction **110,** such as to ensure that at least one analyte carrier and/or biological particle is encapsulated in a partition, the Poissonian distribution may expectedly increase the number of partitions that include multiple analyte carriers and/or biological particles. As such, where singly occupied partitions are to be obtained, at most about 95%, 90%, 85%, 80%, 75%, 70%, 65%, 60%, 55%, 50%, 45%, 40%, 35%, 30%, 25%, 20%, 15%, 10%, 5% or less of the generated partitions can be unoccupied.

In some cases, the flow of one or more of the analyte carriers and/or biological particles (e.g., in channel segment **102),** or other fluids directed into the partitioning junction (e.g., in channel segments **104, 106)** can be controlled such that, in many cases, no more than about 50% of the generated partitions, no more than about 25% of the generated partitions, or no more than about 10% of the generated partitions are unoccupied. These flows can be controlled so as to present a non-Poissonian distribution of single-occupied partitions while providing lower levels of unoccupied partitions. The above noted ranges of unoccupied partitions can be achieved while still providing any of the single occupancy rates described above. For example, in many cases, the use of the systems and methods described herein can create resulting partitions that have multiple occupancy rates of less than about 25%, less than about 20%, less than about 15%, less than about 10%, and in many cases, less than about 5%, while having unoccupied partitions of less than about 50%, less than about 40%, less than about 30%, less than about 20%, less than about 10%, less than about 5%, or less.

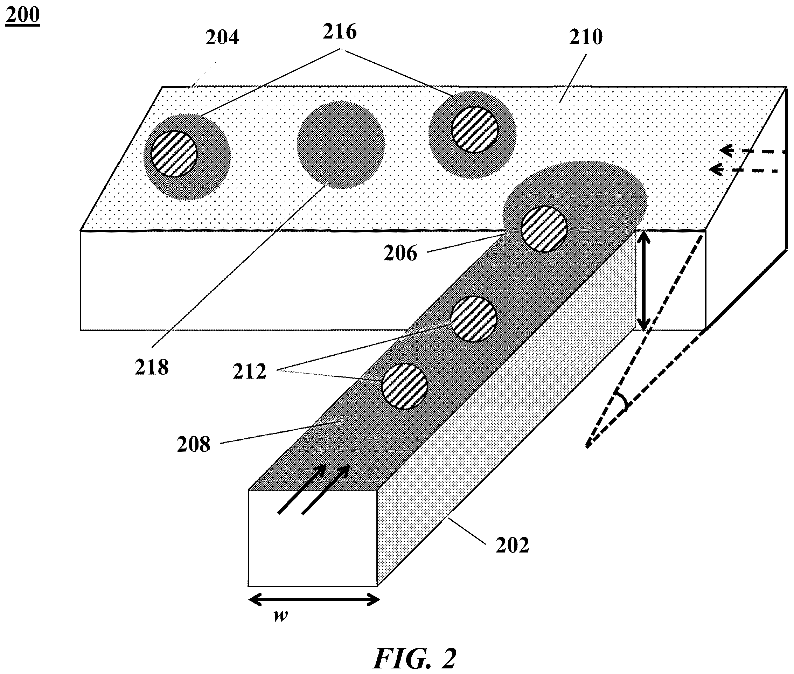

As will be appreciated, the above-described occupancy rates are also applicable to partitions that include both analyte carriers and/or biological particles and additional reagents, including, but not limited to, microcapsules and/or supports such as beads (e.g., gel beads) carrying barcoded nucleic acid molecules (e.g., oligonucleotides) (described in relation to **FIG. 2).** The occupied partitions (e.g., at least about 10%, 20%, 30%, 40%, 50%, 60%, 70%, 80%, 90%, 95%, or 99% of the occupied partitions) can include both a microcapsule and/or support (e.g., bead) comprising barcoded nucleic acid molecules and a analyte carrier and/or biological particle.

In addition to or as an alternative to droplet based partitioning, analyte carriers and/or biological particles may be encapsulated within a microcapsule and/or support that comprises an outer shell, layer or porous matrix in which is entrained one or more individual analyte carriers and/or biological particles or small groups of analyte carriers and/or biological particles. The microcapsule may include other reagents. Encapsulation of analyte carriers and/or biological particles may be performed by a variety of processes. Such processes may combine an aqueous fluid containing the analyte carriers and/or biological particles with a polymeric precursor material that may be capable of being formed into a gel or other solid or semi-solid matrix upon application of a particular stimulus to the polymer precursor. Such stimuli can include, for example, thermal stimuli (e.g., either heating or cooling), photo-stimuli (e.g., through photocuring), chemical stimuli (e.g., through crosslinking, polymerization initiation of the precursor (e.g., through added initiators)), mechanical stimuli, or a combination thereof.

Preparation of microcapsules comprising analyte carriers and/or biological particles (e.g., cells) may be performed by a variety of methods. For example, air knife droplet or aerosol generators may be used to dispense droplets of precursor fluids into gelling solutions in order to form beads (e.g., gel beads) that include individual analyte carriers and/or biological particles or small groups of analyte carriers and/or biological particles. Likewise, membrane-based encapsulation systems may be used to generate beads comprising encapsulated analyte carriers and/or biological particles as described herein. Microfluidic systems of the present disclosure, such as that shown in **FIG. 1,** may be readily used in encapsulating analyte carriers and/or biological particles (e.g., cells) as described herein. In particular, and with reference to **FIG. 1,** the aqueous fluid **112** comprising (i) the analyte carrier sand/or biological particles **114** and (ii) the polymer precursor material (not shown) is flowed into channel junction **110,** where it is partitioned into droplets **118, 120** through the flow of non-aqueous fluid **116.** In the case of encapsulation methods, non-aqueous fluid **116** may also include an initiator (not shown) to cause polymerization and/or crosslinking of the polymer precursor to form the porous matrix that includes the entrained analyte carriers and/or biological particles. Examples of polymer precursor/initiator pairs include those described in U.S. Patent Application Publication No. 2014/0378345.

For example, in the case where the polymer precursor material comprises a linear polymer material, such as a linear polyacrylamide, PEG, or other linear polymeric material, the activation agent may comprise a cross-linking agent, or a chemical that activates a cross-linking agent within the formed droplets. Likewise, for polymer precursors that comprise polymerizable monomers, the activation agent may comprise a polymerization initiator. For example, in certain cases, where the polymer precursor comprises a mixture of acrylamide monomer with a N,N'-bis-(acryloyl)cystamine (BAC) comonomer, an agent such as tetraethylmethylenediamine (TEMED) may be provided within the second fluid streams 116 in channel segments 104 and 106, which can initiate the copolymerization of the acrylamide and BAC into a cross-linked polymer network, or hydrogel.

Upon contact of the second fluid stream 116 with the first fluid stream 112 at junction 110, during formation of droplets, the TEMED may diffuse from the second fluid 116 into the aqueous fluid 112 comprising the linear polyacrylamide, which will activate the crosslinking of the polyacrylamide within the droplets 118, 120, resulting in the formation of gel (e.g., hydrogel) microcapsules, as solid or semi-solid beads or particles entraining the cells 114. Although described in terms of polyacrylamide encapsulation, other 'activatable' encapsulation compositions may also be employed in the context of the methods and compositions described herein. For example, formation of alginate droplets followed by exposure to divalent metal ions (e.g., Ca2+ ions), can be used as an encapsulation process using the described processes. Likewise, agarose droplets may also be transformed into capsules through temperature based gelling (e.g., upon cooling, etc.).

In some cases, encapsulated analyte carriers and/or biological particles can be selectively releasable from the microcapsule, such as through passage of time or upon application of a particular stimulus, that degrades the microcapsule sufficiently to allow the analyte carriers and/or biological particles (e.g., cell), or its other contents to be released from the microcapsule and/or support, such as into a partition (e.g., droplet). For example, in the case of the polyacrylamide polymer described above, degradation of the microcapsule may be accomplished through the introduction of an appropriate reducing agent, such as DTT or the like, to cleave disulfide bonds that cross-link the polymer matrix. See, for example, U.S. Patent Application Publication No. 2014/0378345.

The analyte carrier and/or biological particle can be subjected to other conditions sufficient to polymerize or gel the precursors. The conditions sufficient to polymerize or gel the precursors may comprise exposure to heating, cooling, electromagnetic radiation, and/or light. The conditions sufficient to polymerize or gel the precursors may comprise any conditions sufficient to polymerize or gel the precursors. Following polymerization or gelling, a polymer or gel may be formed around the analyte carrier and/or biological particle. The polymer or gel may be diffusively permeable to chemical or biochemical reagents. The polymer or gel may be diffusively impermeable to macromolecular constituents of the analyte carrier and/or biological particle. In this manner, the polymer or gel may act to allow the analyte carrier and/or biological particle to be subjected to chemical or biochemical operations while spatially confining the macromolecular constituents to a region of the droplet defined by the polymer or gel. The polymer or gel may include one or more of disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin. The polymer or gel may comprise any other polymer or gel.

The polymer or gel may be functionalized to bind to targeted analytes, such as nucleic acids, proteins, carbohydrates, lipids or other analytes. The polymer or gel may be polymerized or gelled via a passive mechanism. The polymer or gel may be stable in alkaline conditions or at elevated temperature. The polymer or gel may have mechanical properties similar to the mechanical properties of the bead. The polymer or gel may be of a similar size to the bead. The polymer or gel may have a mechanical strength (e.g. tensile strength) similar to that of the bead. The polymer or gel may be of a lower density than an oil. The polymer or gel may be of a density that is roughly similar to that of a buffer. The polymer or gel may have a tunable pore size. The pore size may be chosen to retain denatured nucleic acids. The pore size may be chosen to maintain diffusive permeability to exogenous chemicals such as sodium hydroxide (NaOH) and/or endogenous chemicals such as inhibitors. The polymer or gel may be biocompatible. The polymer or gel may maintain or enhance cell viability. The polymer or gel may be biochemically compatible. The polymer or gel may be polymerized and/or depolymerized thermally, chemically, enzymatically, and/or optically.

The polymer may comprise poly(acrylamide-co-acrylic acid) crosslinked with disulfide linkages. The preparation of the polymer may comprise a two-step reaction. In the first activation step, poly(acrylamide-co-acrylic acid) may be exposed to an acylating agent to convert carboxylic acids to esters. The poly(acrylamide-co-acrylic acid) may be exposed to 4-(4,6-dimethoxy-1,3,5-triazin-2-yl)-4-methylmorpholinium chloride (DMTMM). The polyacrylamide-co-acrylic acid may be exposed to other salts of 4-(4,6-dimethoxy-1,3,5-triazin-2-yl)-4-methylmorpholinium. In the second cross-linking step, the ester formed in the first step may be exposed to a disulfide crosslinking agent. The ester may be exposed to cystamine (2,2'-dithiobis(ethylamine)). Following the two steps, the analyte carrier and/or biological particle may be surrounded by polyacrylamide strands linked together by disulfide bridges. In this manner, the analyte carrier and/or biological particle may be encased inside of or comprise a gel or matrix (e.g., polymer matrix) to form a "cell bead."

A cell bead can contain analyte carriers and/or biological particles (e.g., a cell) or macromolecular constituents (e.g., RNA, DNA, proteins, etc.) of analyte carriers and/or biological particles. A cell bead may include a single cell or multiple cells, or a derivative of the single cell or multiple cells. For example after lysing and washing the cells, inhibitory components from cell lysates can be washed away and the macromolecular constituents can be bound as cell beads. Systems and methods disclosed herein can be applicable to both cell beads (and/or droplets or other partitions) containing analyte carriers and/or biological particles and cell beads (and/or droplets or other partitions) containing macromolecular constituents of analyte carriers and/or biological particles. Cell beads may be or include a cell, cell derivative, cellular material and/or material derived from the cell in, within, or encased in a matrix, such as a polymeric matrix. In some cases, a cell bead may comprise a live cell. The live cell may be capable of being cultured when enclosed in a gel or polymer matrix, or of being cultured when comprising a gel or polymer matrix. The polymer or gel may be diffusively permeable to certain components and diffusively impermeable to other components (e.g., macromolecular constituents).

Encapsulated analyte carriers and/or biological particles can provide certain potential advantages of being more storable and more portable than droplet-based partitioned analyte carrier and/or biological particles. Furthermore, in some cases, it may be desirable to allow analyte carriers and/or biological particles to incubate for a select period of time before analysis, such as in order to characterize changes in such analyte carriers and/or biological particles over time, either in the presence or absence of different stimuli (or reagents). In such cases, encapsulation may allow for longer incubation than partitioning in emulsion droplets, although in some cases, droplet partitioned analyte carriers and/or biological particles may also be incubated for different periods of time, e.g., at least 10 seconds, at least 30 seconds, at least 1 minute, at least 5 minutes, at least 10 minutes, at least 30 minutes, at least 1 hour, at least 2 hours, at least 5 hours, or at least 10 hours or more. The encapsulation of analyte carriers and/or biological particles may constitute the partitioning of the analyte carriers and/or biological particles into which other reagents are co-partitioned. Alternatively or in addition, encapsulated analyte carriers and/or biological particles may be readily deposited into other partitions (e.g., droplets) as described above.

## Wells

As described herein, one or more processes may be performed in a partition, which may be a well. The well may be a well of a plurality of wells of a substrate, such as a microwell of a microwell array or plate, or the well may be a microwell or microchamber of a device (e.g., microfluidic device) comprising a substrate. The well may be a well of a well array or plate, or the well may be a well or chamber of a device (e.g., fluidic device). Accordingly, the wells or microwells may assume an "open" configuration, in which the wells or microwells are exposed to the environment (e.g., contain an open surface) and are accessible on one planar face of the substrate, or the wells or microwells may assume a "closed" or "sealed" configuration, in which the microwells are not accessible on a planar face of the substrate. The wells or microwells may be configured to toggle between "open" and "closed" configurations. An "open" microwell or set of microwells may be "closed" or "sealed" using a membrane (e.g., semi-permeable membrane), an oil (e.g., fluorinated oil to cover an aqueous solution), or a lid, as described elsewhere herein.

The well may have a volume of less than 1 milliliter (mL). The well may be configured to hold a volume of at most 1000 microliters (µL), at most 100 µL, at most 10 µL, at most 1 µL, at most 100 nanoliters (nL), at most 10 nL, at most 1 nL, at most 100 picoliters (pL), at most 10 (pL), or less. The well may be configured to hold a volume of about 1000 µL, about 100 µL, about 10 µL, about 1 µL, about 100 nL, about 10 nL, about 1 nL, about 100 pL, about 10 pL, etc. The well may be configured to hold a volume of at least 10 pL, at least 100 pL, at least 1 nL, at least 10 nL, at least 100 nL, at least 1 µL, at least 10 µL, at least 100 µL, at least 1000 µL, or more. The well may be configured to hold a volume in a range of volumes listed herein, for example, from about 5 nL to about 20 nL, from about 1 nL to about 100 nL, from about 500 pL to about 100 µL, etc. The well may be of a plurality of wells that have varying volumes and may be configured to hold a volume appropriate to accommodate any of the partition volumes described herein.

A microwell array or plate may comprise a single variety of microwells. A microwell array or plate may comprise a variety of microwells. The microwell array or plate may comprise one or more types of microwells within a single microwell array or plate. The types of microwells may have different dimensions (e.g., length, width, diameter, depth, cross-sectional area, etc.), shapes (e.g., circular, triangular, square, rectangular, pentagonal, hexagonal, heptagonal, octagonal, nonagonal, decagonal, etc.), aspect ratios, or other physical characteristics. The microwell array or plate may comprise any number of different types of microwells. For example, the microwell array or plate may comprise 1, 2, 3, 4, 5, 6, 7, 8, 9, 10, 20, 30, 40, 50, 60, 70, 80, 90, 100, 200, 300, 400, 500, 600, 700, 800, 900, 1000 or more different types of microwells. A well may have any dimension (e.g., length, width, diameter, depth, cross-sectional area, volume, etc.), shape (e.g., circular, triangular, square, rectangular, pentagonal, hexagonal, heptagonal, octagonal, nonagonal, decagonal, other polygonal, etc.), aspect ratios, or other physical characteristics described herein with respect to any well.

The microwell array or plate may comprise different types of microwells that are located adjacent to one another within the array or plate. A microwell with one set of dimensions may be located adjacent to and in contact with another microwell with a different set of dimensions. Similarly, microwells of different geometries may be placed adjacent to or in contact with one another. The adjacent microwells may be configured to hold different articles; for example, one microwell may be used to contain a cell, cell bead, or other sample (e.g., cellular components, nucleic acid molecules, etc.) while the adjacent microwell may be used to contain a microcapsule and/or support (e.g., a bead such as a gel bead), droplet, or other reagent. In some cases, the adjacent microwells may be configured to merge the contents held within, e.g., upon application of a stimulus, or spontaneously, upon contact of the articles in each microwell.

As is described elsewhere herein, a plurality of partitions may be used in the systems, compositions, and methods described herein. For example, any suitable number of partitions (e.g., wells or droplets) can be generated or otherwise provided. For example, in the case when wells are used, at least about 1,000 wells, at least about 5,000 wells, at least about 10,000 wells, at least about 50,000 wells, at least about 100,000 wells, at least about 500,000 wells, at least about 1,000,000 wells, at least about 5,000,000 wells at least about 10,000,000 wells, at least about 50,000,000 wells, at least about 100,000,000 wells, at least about 500,000,000 wells, at least about 1,000,000,000 wells, or more wells can be generated or otherwise provided. Moreover, the plurality of wells may comprise both unoccupied wells (e.g., empty wells) and occupied wells.

A well may comprise any of the reagents described herein, or combinations thereof. These reagents may include, for example, barcode molecules, enzymes, adapters, and combinations thereof. The reagents may be physically separated from a sample (e.g., a cell, cell bead, or cellular components, e.g., proteins, nucleic acid molecules, etc.) that is placed in the well. This physical separation may be accomplished by containing the reagents within, or coupling to, a microcapsule and/or support (e.g., a bead such as a gel bead) that is placed within a well. The physical separation may also be accomplished by dispensing the reagents in the well and overlaying the reagents with a layer that is, for example, dissolvable, meltable, or permeable prior to introducing the polynucleotide sample into the well. This layer may be, for example, an oil, wax, membrane (e.g., semi-permeable membrane), or the like. The well may be sealed at any point, for example, after addition of the microcapsule and/or support (e.g., bead), after addition of the reagents, or after addition of either of these components. The sealing of the well may be useful for a variety of purposes, including preventing escape of beads or loaded reagents from the well, permitting select delivery of certain reagents (e.g., via the use of a semi-permeable membrane), for storage of the well prior to or following further processing, etc.

A well may comprise free reagents and/or reagents encapsulated in, or otherwise coupled to or associated with, microcapsules and/or supports (e.g., beads) or droplets. Any of the reagents described in this disclosure may be encapsulated in, or otherwise coupled to, a microcapsule and/or support (e.g., bead) or droplet, with any chemicals, particles, and elements suitable for sample processing reactions involving biomolecules, such as, but not limited to, nucleic acid molecules and proteins. For example, a bead or droplet used in a sample preparation reaction for DNA sequencing may comprise one or more of the following reagents: enzymes, restriction enzymes (e.g., multiple cutters), ligase, polymerase, fluorophores, oligonucleotide barcodes, adapters, buffers, nucleotides (e.g., dNTPs, ddNTPs) and the like.

Additional examples of reagents include, but are not limited to: buffers, acidic solution, basic solution, temperature-sensitive enzymes, pH-sensitive enzymes, light-sensitive enzymes, metals, metal ions, magnesium chloride, sodium chloride, manganese, aqueous buffer, mild buffer, ionic buffer, inhibitor, enzyme, protein, polynucleotide, antibodies, saccharides, lipid, oil, salt, ion, detergents, ionic detergents, non-ionic detergents, oligonucleotides, nucleotides, deoxyribonucleotide triphosphates (dNTPs), dideoxyribonucleotide triphosphates (ddNTPs), DNA, RNA, peptide polynucleotides, complementary DNA (cDNA), double stranded DNA (dsDNA), single stranded DNA (ssDNA), plasmid DNA, cosmid DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA, polymerase, ligase, restriction enzymes, proteases, nucleases, protease inhibitors, nuclease inhibitors, chelating agents, reducing agents, oxidizing agents, fluorophores, probes, chromophores, dyes, organics, emulsifiers, surfactants, stabilizers, polymers, water, small molecules, pharmaceuticals, radioactive molecules, preservatives, antibiotics, aptamers, and pharmaceutical drug compounds. As described herein, one or more reagents in the well may be used to perform one or more reactions, including but not limited to: cell lysis, cell fixation, permeabilization, nucleic acid reactions, e.g., nucleic acid extension reactions, amplification, reverse transcription, transposase reactions (e.g., tagmentation), etc.

The wells may be provided as a part of a kit. For example, a kit may comprise instructions for use, a microwell array or device, and reagents (e.g., beads). The kit may comprise any useful reagents for performing the processes described herein, e.g., nucleic acid reactions, barcoding of nucleic acid molecules, sample processing (e.g., for cell lysis, fixation, and/or permeabilization).

In some cases, a well comprises a microcapsule and/or support (e.g., a bead), or droplet that comprises a set of reagents that has a similar attribute (e.g., a set of enzymes, a set of minerals, a set of oligonucleotides, a mixture of different barcode molecules, a mixture of identical barcode molecules). In other cases, a microcapsule and/or support or droplet comprises a heterogeneous mixture of reagents. In some cases, the heterogeneous mixture of reagents can comprise all components necessary to perform a reaction. In some cases, such mixture can comprise all components necessary to perform a reaction, except for 1, 2, 3, 4, 5, or more components necessary to perform a reaction. In some cases, such additional components are contained within, or otherwise coupled to, a different microcapsule and/or support or droplet, or within a solution within a partition (e.g., microwell) of the system.

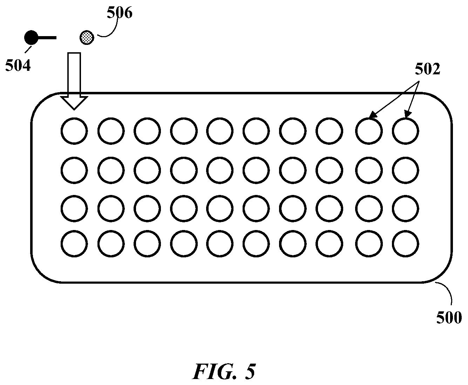

**FIG. 5** schematically illustrates an example of a microwell array. The array can be contained within a substrate **500.** The substrate **500** comprises a plurality of wells **502.** The wells **502** may be of any size or shape, and the spacing between the wells, the number of wells per substrate, as well as the density of the wells on the substrate **500** can be modified, depending on the particular application. In one such example application, a sample molecule **506,** which may comprise a cell or cellular components (e.g., nucleic acid molecules) is co-partitioned with a bead **504,** which may comprise a nucleic acid barcode molecule coupled thereto. The wells **502** may be loaded using gravity or other loading technique (e.g., centrifugation, liquid handler, acoustic loading, optoelectronic, etc.). At least one of the wells **502** may contain a single sample molecule **506** (e.g., cell) and a single bead **504.**

Reagents may be loaded into a well either sequentially or concurrently. In some cases, reagents are introduced to the device either before or after a particular operation. In some cases, reagents (which may be provided in microcapsules and/or supports or droplets) are introduced sequentially such that different reactions or operations occur at different steps. The reagents (or microcapsules, supports, or droplets) may also be loaded at operations interspersed with a reaction or operation step. For example, microcapsules and/or supports (or droplets) comprising reagents for fragmenting polynucleotides (e.g., restriction enzymes) and/or other enzymes (e.g., transposases, ligases, polymerases, etc.) may be loaded into the well or plurality of wells, followed by loading of microcapsules and/or supports or droplets comprising reagents for attaching nucleic acid barcode molecules to a sample nucleic acid molecule. Reagents may be provided concurrently or sequentially with a sample, e.g., a cell or cellular components (e.g., organelles, proteins, nucleic acid molecules, carbohydrates, lipids, etc.). Accordingly, use of wells may be useful in performing multi-step operations or reactions.

As described elsewhere herein, the nucleic acid barcode molecules and other reagents may be contained within a microcapsule and/or support (e.g., a bead), or droplet. These microcapsules and/or supports, or droplets may be loaded into a partition (e.g., a microwell) before, after, or concurrently with the loading of a cell, such that each cell is contacted with a different microcapsule and/or support or droplet. This technique may be used to attach a unique nucleic acid barcode molecule to nucleic acid molecules obtained from each cell. Alternatively or in addition to, the sample nucleic acid molecules may be attached to a microcapsule and/or support. The partition (e.g., microwell) may comprise a bead which has coupled thereto a plurality of nucleic acid barcode molecules. The sample nucleic acid molecules, or derivatives thereof, may couple or attach to the nucleic acid barcode molecules on the microcapsule and/or support. The resulting barcoded nucleic acid molecules may then be removed from the partition, pooled and sequenced. In such cases, the nucleic acid barcode sequences may be used to trace the origin of the sample nucleic acid molecule. For example, polynucleotides with identical barcodes may be determined to originate from the same cell or partition, while polynucleotides with different barcodes may be determined to originate from different cells or partitions.

The samples or reagents may be loaded in the wells or microwells using a variety of approaches. The samples (e.g., a cell, cell bead, or cellular component) or reagents (as described herein) may be loaded into the well or microwell using an external force, e.g., gravitational force, electrical force, magnetic force, or using mechanisms to drive the sample or reagents into the well, e.g., via pressure-driven flow, centrifugation, optoelectronics, acoustic loading, electrokinetic pumping, vacuum, capillary flow, etc. In certain cases, a fluid handling system may be used to load the samples or reagents into the well. The loading of the samples or reagents may follow a Poissonian distribution or a non-Poissonian distribution, e.g., super Poisson or sub-Poisson. The geometry, spacing between wells, density, and size of the microwells may be modified to accommodate a useful sample or reagent distribution; for instance, the size and spacing of the microwells may be adjusted such that the sample or reagents may be distributed in a super-Poissonian fashion.

In one particular non-limiting example, the microwell array or plate comprises pairs of microwells, in which each pair of microwells is configured to hold a droplet (e.g., comprising a single cell) and a single bead (such as those described herein, which may also be encapsulated in a droplet). The droplet and the bead (or droplet containing the bead) may be loaded simultaneously or sequentially, and the droplet and the bead may be merged, e.g., upon contact of the droplet and the bead, or upon application of a stimulus (e.g., external force, agitation, heat, light, magnetic or electric force, etc.). In some cases, the loading of the droplet and the bead is super-Poissonian. In other examples of pairs of microwells, the wells are configured to hold two droplets comprising different reagents and/or samples, which are merged upon contact or upon application of a stimulus. The droplet of one microwell of the pair may comprise reagents that may react with an agent in the droplet of the other microwell of the pair. One droplet may comprise reagents that are configured to release the nucleic acid barcode molecules of a bead contained in another droplet, located in the adjacent microwell. Upon merging of the droplets, the nucleic acid barcode molecules may be released from the bead into the partition (e.g., the microwell or microwell pair that are in contact), and further processing may be performed (e.g., barcoding, nucleic acid reactions, etc.). In cases where intact or live cells are loaded in the microwells, one of the droplets may comprise lysis reagents for lysing the cell upon droplet merging.

A droplet or microcapsule and/or support (e.g., a bead) may be partitioned into a well. The droplets may be selected or subjected to pre-processing prior to loading into a well. The droplets may comprise cells, and only certain droplets, such as those containing a single cell (or at least one cell), may be selected for use in loading of the wells. Such a pre-selection process may be useful in efficient loading of single cells, such as to obtain a non-Poissonian distribution, or to pre-filter cells for a selected characteristic prior to further partitioning in the wells. Additionally, the technique may be useful in obtaining or preventing cell doublet or multiplet formation prior to or during loading of the microwell.

The wells may comprise nucleic acid barcode molecules attached thereto. The nucleic acid barcode molecules may be attached to a surface of the well (e.g., a wall of the well). The nucleic acid barcode molecule (e.g., a partition barcode sequence) of one well may differ from the nucleic acid barcode molecule of another well, which can permit identification of the contents contained with a single partition or well. In some cases, the nucleic acid barcode molecule can comprise a spatial barcode sequence that can identify a spatial coordinate of a well, such as within the well array or well plate. In some cases, the nucleic acid barcode molecule can comprise a unique molecular identifier for individual molecule identification. The nucleic acid barcode molecules may be configured to attach to or capture a nucleic acid molecule within a sample or cell distributed in the well. For example, the nucleic acid barcode molecules may comprise a capture sequence that may be used to capture or hybridize to a nucleic acid molecule (e.g., RNA, DNA) within the sample. The nucleic acid barcode molecules may be releasable from the microwell. The nucleic acid barcode molecules may comprise a chemical cross-linker which may be cleaved upon application of a stimulus (e.g., photo-, magnetic, chemical, biological, stimulus). The released nucleic acid barcode molecules, which may be hybridized or configured to hybridize to a sample nucleic acid molecule, may be collected and pooled for further processing, which can include nucleic acid processing (e.g., amplification, extension, reverse transcription, etc.) and/or characterization (e.g., sequencing). In such cases, the unique partition barcode sequences may be used to identify the cell or partition from which a nucleic acid molecule originated.

Characterization of samples within a well may be performed. Such characterization can include, in non-limiting examples, imaging of the sample (e.g., cell, cell bead, or cellular components) or derivatives thereof. Characterization techniques such as microscopy or imaging may be useful in measuring sample profiles in fixed spatial locations. When cells are partitioned, optionally with beads, imaging of each microwell and the contents contained therein may provide useful information on cell doublet formation (e.g., frequency, spatial locations, etc.), cell-bead pair efficiency, cell viability, cell size, cell morphology, expression level of a biomarker (e.g., a surface marker, a fluorescently labeled molecule therein, etc.), cell or bead loading rate, number of cell-bead pairs, etc. Imaging may be used to characterize live cells in the wells, including, but not limited to: dynamic live-cell tracking, cell-cell interactions (when two or more cells are co-partitioned), cell proliferation, etc. Alternatively or in addition to, imaging may be used to characterize a quantity of amplification products in the well.

In operation, a well may be loaded with a sample and reagents, simultaneously or sequentially. When cells or cell beads are loaded, the well may be subjected to washing, e.g., to remove excess cells from the well, microwell array, or plate. Similarly, washing may be performed to remove excess beads or other reagents from the well, microwell array, or plate. Where live cells are used, the cells may be lysed in the individual partitions to release the intracellular components or cellular analytes. Alternatively, the cells may be fixed or permeabilized in the individual partitions. The intracellular components or cellular analytes may couple to a microcapsule and/or support, e.g., on a surface of the microwell, on a solid support (e.g., bead), or they may be collected for further downstream processing. After cell lysis, the intracellular components or cellular analytes may be transferred to individual droplets or other partitions for barcoding. Alternatively, or in addition to, the intracellular components or cellular analytes (e.g., nucleic acid molecules) may couple to a bead comprising a nucleic acid barcode molecule; subsequently, the bead may be collected and further processed, e.g., subjected to nucleic acid reaction such as reverse transcription, amplification, or extension, and the nucleic acid molecules thereon may be further characterized, e.g., via sequencing. Alternatively, or in addition to, the intracellular components or cellular analytes may be barcoded in the well (e.g., using a bead comprising nucleic acid barcode molecules that are releasable or on a surface of the microwell comprising nucleic acid barcode molecules). The barcoded nucleic acid molecules or analytes may be further processed in the well, or the barcoded nucleic acid molecules or analytes may be collected from the individual partitions and subjected to further processing outside the partition. Further processing can include nucleic acid processing (e.g., performing an amplification, extension) or characterization (e.g., fluorescence monitoring of amplified molecules, sequencing). At any convenient or useful step, the well (or microwell array or plate) may be sealed (e.g., using an oil, membrane, wax, etc.), which enables storage of the assay or selective introduction of additional reagents.

Once sealed, the well may be subjected to conditions for further processing of a analyte carrier and/or biological particle (e.g., a cell or a cell bead) in the well. Reagents in the well may allow further processing of the analyte carrier and/or biological particle, e.g., lysis of the cell, as further described herein. Alternatively, the well (or wells such as those of a well-based array) comprising the analyte carrier and/or biological particle (e.g., cell or cell bead) may be subjected to freeze-thaw cycling to process the analyte carrier(s) and/or biological particle(s), e.g., lysis of a cell. The well containing the analyte carrier and/or biological particle (e.g., cell or cell bead) may be subjected to freezing temperatures (e.g., 0°C, below 0°C, -5°C, -10°C, -15°C, - 20°C, -25°C, -30°C, -35°C, -40°C, -45°C, -50°C, -55°C, -60°C, -65°C, -70°C, -80°C, or -85°C). Freezing may be performed in a suitable manner, e.g., sub-zero freezer or a dry ice/ethanol bath. Following an initial freezing, the well (or wells) comprising the analyte carrier and/or biological particle(s) (e.g., cell(s) or cell bead(s)) may be subjected to freeze thaw cycles to lyse analyte carrier(s) and/or biological particle(s). The initially frozen well (or wells) may be thawed to a temperature above freezing (e.g., room temperature or 25°C). The freezing may be performed for less than 10 minutes (e.g., 5 minutes or 7 minutes) followed by thawing at room temperature for less than 10 minutes (e.g., 5 minutes or 7 minutes). This freeze-thaw cycle may be repeated a number of times, e.g., 2, 3, or 4 times, to obtain lysis of the analyte carrier(s) and/or biological particle(s) (e.g., cell(s) or cell bead(s)) in the well (or wells). The freezing, thawing and/or freeze/thaw cycling may be performed in the absence of a lysis buffer. Additional disclosure related to freeze-thaw cycling is provided in WO2019/165181A1.

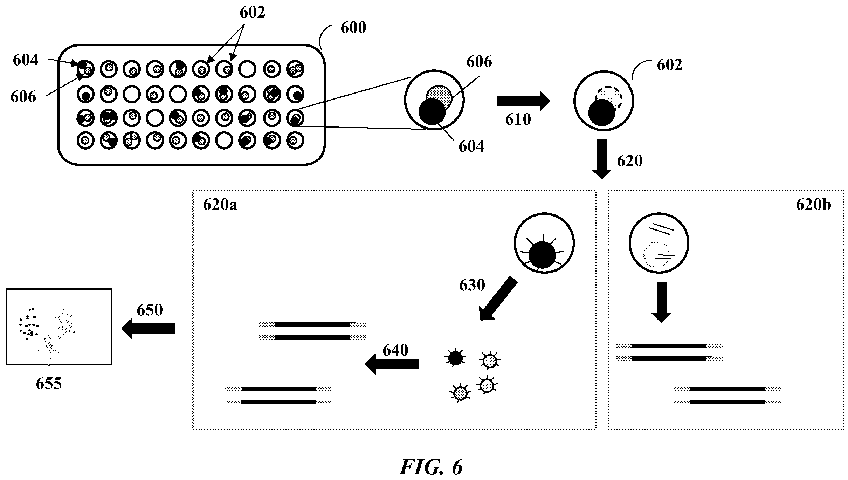

FIG. 6 schematically shows an example workflow for processing nucleic acid molecules within a sample. A substrate **600** comprising a plurality of microwells **602** may be provided. A sample **606** which may comprise a cell, cell bead, cellular components or analytes (e.g., proteins and/or nucleic acid molecules) can be co-partitioned, in a plurality of microwells **602,** with a plurality of beads **604** comprising nucleic acid barcode molecules. During process **610,** the sample **606** may be processed within the partition. In the case of live cells, the cell may be subjected to conditions sufficient to lyse the cells and release the analytes contained therein. In process **620,** the bead **604** may be further processed. By way of example, processes **620a** and **620b** schematically illustrate different workflows, depending on the properties of the bead **604.**

In **620a,** the bead comprises nucleic acid barcode molecules that are attached thereto, and sample nucleic acid molecules (e.g., RNA, DNA) may attach, e.g., via hybridization of ligation, to the nucleic acid barcode molecules. Such attachment may occur on the bead. In process **630,** the beads **604** from multiple wells **602** may be collected and pooled. Further processing may be performed in process **640.** For example, one or more nucleic acid reactions may be performed, such as reverse transcription, nucleic acid extension, amplification, ligation, transposition, etc. One or more reactions may occur on the bead using the sample nucleic acid molecules captured on the bead via the nucleic acid barcode molecules on the bead, e.g., sample nucleic acid molecules hybridized to complementary sequences of the nucleic acid barcode molecules on the bead. Adapter sequences may be ligated to the nucleic acid molecules, or derivatives thereof, as described elsewhere herein. Sequencing primer sequences may be appended to each end of the nucleic acid molecule. In process **650,** further characterization, such as sequencing may be performed to generate sequencing reads. The sequencing reads may yield information on individual cells or populations of cells, which may be represented visually or graphically, e.g., in a plot **655.**

In **620b,** the bead comprises nucleic acid barcode molecules that are releasably attached thereto, as described below. The bead may degrade or otherwise release the nucleic acid barcode molecules into the well **602;** the nucleic acid barcode molecules may then be used to barcode nucleic acid molecules within the well **602.** Further processing may be performed either inside the partition or outside the partition. For example, one or more nucleic acid reactions may be performed, such as reverse transcription, nucleic acid extension, amplification, ligation, transposition, etc. Adapter sequences may be ligated to the nucleic acid molecules, or derivatives thereof, as described elsewhere herein. Sequencing primer sequences may be appended to each end of the nucleic acid molecule. In process **650,** further characterization, such as sequencing may be performed to generate sequencing reads. The sequencing reads may yield information on individual cells or populations of cells, which may be represented visually or graphically, e.g., in a plot **655.**

### Beads

Nucleic acid barcode molecules may be delivered to a partition (e.g., a droplet or well) via a solid microcapsule and/or support or carrier (e.g., a bead). In some cases, nucleic acid barcode molecules are initially associated with the microcapsule and/or solid support and then released from the microcapsule and/or solid support upon application of a stimulus, which allows the nucleic acid barcode molecules to dissociate or to be released from the solid microcapsule and/or support. In specific examples, nucleic acid barcode molecules are initially associated with the solid microcapsule and/or support (e.g., bead) and then released from the solid microcapsule and/or support upon application of a biological stimulus, a chemical stimulus, a thermal stimulus, an electrical stimulus, a magnetic stimulus, and/or a photo stimulus.

A nucleic acid barcode molecule may contain a barcode sequence and a functional sequence, such as a nucleic acid primer sequence or a template switch oligonucleotide (TSO) sequence.

The solid microcapsule and/or support may be a bead. A solid microcapsule and/or support, e.g., a bead, may be porous, non-porous, hollow (e.g., a microcapsule), solid, semi-solid, and/or a combination thereof. Beads may be solid, semi-solid, semi-fluidic, fluidic, and/or a combination thereof. A solid microcapsule and/or support, e.g., a bead, may be at least partially dissolvable, disruptable, and/or degradable. In some cases, a solid microcapsule and/or support, e.g., a bead, may not be degradable. In some cases, the solid microcapsule and/or support, e.g., a bead, may be a gel bead. A gel bead may be a hydrogel bead. A gel bead may be formed from molecular precursors, such as a polymeric or monomeric species. A semi-solid microcapsule and/or support, e.g., a bead, may be a liposomal bead. Solid microcapsules and/or supports, e.g., beads, may comprise metals including iron oxide, gold, and silver. In some cases, the solid microcapsule and/or support, e.g., the bead, may be a silica bead. In some cases, the solid microcapsule and/or support, e.g., a bead, can be rigid. In other cases, the solid microcapsule and/or support, e.g., a bead, may be flexible and/or compressible.

A partition may comprise one or more unique identifiers, such as barcodes. Barcodes may be previously, subsequently or concurrently delivered to the partitions that hold the compartmentalized or partitioned analyte carrier and/or biological particle. For example, barcodes may be injected into droplets or deposited in microwells previous to, subsequent to, or concurrently with droplet generation or providing of reagents in the microwells, respectively. The delivery of the barcodes to a particular partition allows for the later attribution of the characteristics of the individual analyte carrier and/or biological particle to the particular partition. Barcodes may be delivered, for example on a nucleic acid molecule (e.g., an oligonucleotide), to a partition via any suitable mechanism. Barcoded nucleic acid molecules can be delivered to a partition via a microcapsule and/or support (e.g., a bead). A microcapsule and/or support may comprise a bead. Beads are described in further detail below.

In some cases, barcoded nucleic acid molecules can be initially associated with the microcapsule and/or support (e.g., bead) and then released from the microcapsule and/or support. Release of the barcoded nucleic acid molecules can be passive (e.g., by diffusion from or out of the microcapsule and/or support). In addition or alternatively, release from the microcapsule and/or support can be upon application of a stimulus which allows the barcoded nucleic acid nucleic acid molecules to dissociate or to be released from the microcapsule and/or support (e.g., bead). Such stimulus may disrupt the microcapsule and/or support, an interaction that couples the barcoded nucleic acid molecules to or within the microcapsule and/or support, or both. Such stimulus can include, for example, a thermal stimulus, photo-stimulus, chemical stimulus (e.g., change in pH or use of a reducing agent(s)), a mechanical stimulus, a radiation stimulus; a biological stimulus (e.g., enzyme), or any combination thereof. Methods and systems for partitioning barcode carrying beads into droplets are provided in US. Patent Publication Nos. 2019/0367997 and 2019/0064173, and WO2020/167862A1.

In some examples, beads, analyte carriers, biological particles, and droplets may flow along channels (e.g., the channels of a microfluidic device), in some cases at substantially regular flow profiles (e.g., at regular flow rates). Such regular flow profiles may permit a droplet to include a single bead and a single analyte carrier and/or biological particle. Such regular flow profiles may permit the droplets to have an occupancy (e.g., droplets having beads and biological particles) greater than 5%, 10%, 20%, 30%, 40%, 50%, 60%, 70%, 80%, 90%, or 95%. Such regular flow profiles and devices that may be used to provide such regular flow profiles are provided in, for example, U.S. Patent Publication No. 2015/0292988.

A bead may be porous, non-porous, solid, semi-solid, semi-fluidic, fluidic, and/or a combination thereof. A bead may be dissolvable, disruptable, and/or degradable. In some cases, a bead may not be degradable. In some cases, the bead may be a gel bead. A gel bead may be a hydrogel bead. A gel bead may be formed from molecular precursors, such as a polymeric or monomeric species. A semi-solid bead may be a liposomal bead. Solid beads may comprise metals including iron oxide, gold, and silver. In some cases, the bead may be a silica bead. In some cases, the bead can be rigid. In other cases, the bead may be flexible and/or compressible.

A bead may be of any suitable shape. Examples of bead shapes include, but are not limited to, spherical, non-spherical, oval, oblong, amorphous, circular, cylindrical, and variations thereof.

Beads may be of uniform size or heterogeneous size. In some cases, the diameter of a bead may be at least about 10 nanometers (nm), 100 nm, 500 nm, 1 micrometer ($\mu$m), 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or greater. In some cases, a bead may have a diameter of less than about 10 nm, 100 nm, 500 nm, 1$\mu$m, 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or less. In some cases, a bead may have a diameter in the range of about 40-75$\mu$m, 30-75$\mu$m, 20-75$\mu$m, 40-85$\mu$m, 40-95$\mu$m, 20-100$\mu$m, 10-100$\mu$m, 1-100$\mu$m, 20-250$\mu$m, or 20-500$\mu$m.

Beads can be provided as a population or plurality of beads having a relatively monodisperse size distribution. Where it may be desirable to provide relatively consistent amounts of reagents within partitions, maintaining relatively consistent bead characteristics, such as size, can contribute to the overall consistency. In particular, the beads described herein may have size distributions that have a coefficient of variation in their cross-sectional dimensions of less than 50%, less than 40%, less than 30%, less than 20%, and in some cases less than 15%, less than 10%, less than 5%, or less.

A bead may comprise natural and/or synthetic materials. For example, a bead can comprise a natural polymer, a synthetic polymer or both natural and synthetic polymers. Examples of natural polymers include proteins and sugars such as deoxyribonucleic acid, rubber, cellulose, starch (e.g., amylose, amylopectin), proteins, enzymes, polysaccharides, silks, polyhydroxyalkanoates, chitosan, dextran, collagen, carrageenan, ispaghula, acacia, agar, gelatin, shellac, sterculia gum, xanthan gum, Corn sugar gum, guar gum, gum karaya, agarose, alginic acid, alginate, or natural polymers thereof. Examples of synthetic polymers include acrylics, nylons, silicones, spandex, viscose rayon, polycarboxylic acids, polyvinyl acetate, polyacrylamide, polyacrylate, polyethylene glycol, polyurethanes, polylactic acid, silica, polystyrene, polyacrylonitrile, polybutadiene, polycarbonate, polyethylene, polyethylene terephthalate, poly(chlorotrifluoroethylene), poly(ethylene oxide), poly(ethylene terephthalate), polyethylene, polyisobutylene, poly(methyl methacrylate), poly(oxymethylene), polyformaldehyde, polypropylene, polystyrene, poly(tetrafluoroethylene), poly(vinyl acetate), poly(vinyl alcohol), poly(vinyl chloride), poly(vinylidene dichloride), poly(vinylidene difluoride), poly(vinyl fluoride) and/or combinations (e.g., co-polymers) thereof. Beads may also be formed from materials other than polymers, including lipids, micelles, ceramics, glass-ceramics, material composites, metals, other inorganic materials, and others.

The bead may contain molecular precursors (e.g., monomers or polymers), which may form a polymer network via polymerization of the molecular precursors. In some cases, a precursor may be an already polymerized species capable of undergoing further polymerization via, for example, a chemical cross-linkage. In some cases, a precursor can comprise one or more of an acrylamide or a methacrylamide monomer, oligomer, or polymer. In some cases, the bead may comprise prepolymers, which are oligomers capable of further polymerization. For example, polyurethane beads may be prepared using prepolymers. In some cases, the bead may contain individual polymers that may be further polymerized together. In some cases, beads may be generated via polymerization of different precursors, such that they comprise mixed polymers, co-polymers, and/or block co-polymers. In some cases, the bead may comprise covalent or ionic bonds between polymeric precursors (e.g., monomers, oligomers, linear polymers), nucleic acid molecules (e.g., oligonucleotides), primers, and other entities. In some cases, the covalent bonds can be carbon-carbon bonds, thioether bonds, or carbon-heteroatom bonds.

Cross-linking may be permanent or reversible, depending upon the particular cross-linker used. Reversible cross-linking may allow for the polymer to linearize or dissociate under appropriate conditions. In some cases, reversible cross-linking may also allow for reversible attachment of a material bound to the surface of a bead. In some cases, a cross-linker may form disulfide linkages. In some cases, the chemical cross-linker forming disulfide linkages may be cystamine or a modified cystamine.

In some cases, disulfide linkages can be formed between molecular precursor units (e.g., monomers, oligomers, or linear polymers) or precursors incorporated into a bead and nucleic acid molecules (e.g., oligonucleotides). Cystamine (including modified cystamines), for example, is an organic agent comprising a disulfide bond that may be used as a crosslinker agent between individual monomeric or polymeric precursors of a bead. Polyacrylamide may be polymerized in the presence of cystamine or a species comprising cystamine (e.g., a modified cystamine) to generate polyacrylamide gel beads comprising disulfide linkages (e.g., chemically degradable beads comprising chemically-reducible cross-linkers). The disulfide linkages may permit the bead to be degraded (or dissolved) upon exposure of the bead to a reducing agent.

In some cases, chitosan, a linear polysaccharide polymer, may be crosslinked with glutaraldehyde via hydrophilic chains to form a bead. Crosslinking of chitosan polymers may be achieved by chemical reactions that are initiated by heat, pressure, change in pH, and/or radiation.

A bead may comprise an acrydite moiety, which may be used to attach one or more nucleic acid molecules (e.g., barcode sequence, barcoded nucleic acid molecule, barcoded oligonucleotide, primer, or other oligonucleotide) to the bead. In some cases, an acrydite moiety can refer to an acrydite analogue generated from the reaction of acrydite with one or more species, such as, the reaction of acrydite with other monomers and cross-linkers during a polymerization reaction. Acrydite moieties may be modified to form chemical bonds with a species to be attached, such as a nucleic acid molecule (e.g., barcode sequence, barcoded nucleic acid molecule, barcoded oligonucleotide, primer, or other oligonucleotide). Acrydite moieties may be modified with thiol groups capable of forming a disulfide bond or may be modified with groups already comprising a disulfide bond. The thiol or disulfide (via disulfide exchange) may be used as an anchor point for a species to be attached or another part of the acrydite moiety may be used for attachment. In some cases, attachment can be reversible, such that when the disulfide bond is broken (e.g., in the presence of a reducing agent), the attached species is released from the bead. In other cases, an acrydite moiety can comprise a reactive hydroxyl group that may be used for attachment.

Functionalization of beads for attachment of nucleic acid molecules (e.g., oligonucleotides) may be achieved through a wide range of different approaches, including activation of chemical groups within a polymer, incorporation of active or activatable functional groups in the polymer structure, or attachment at the pre-polymer or monomer stage in bead production.

For example, precursors (e.g., monomers, cross-linkers) that are polymerized to form a bead may comprise acrydite moieties, such that when a bead is generated, the bead also comprises acrydite moieties. The acrydite moieties can be attached to a nucleic acid molecule (e.g., oligonucleotide) that comprises one or more functional sequences, such as a TSO sequence or a primer sequence (e.g., a poly T sequence, or a nucleic acid primer sequence complementary to a target nucleic acid sequence and/or for amplifying a target nucleic acid sequence, a random primer, or a primer sequence for messenger RNA) that is useful for incorporation into the bead, etc.) and/or one or more barcode sequences. The one or more barcode sequences may include sequences that are the same for all nucleic acid molecules coupled to a given bead and/or sequences that are different across all nucleic acid molecules coupled to the given bead. The nucleic acid molecule may be incorporated into the bead.

In some cases, the nucleic acid molecule can comprise a functional sequence, for example, for attachment to a sequencing flow cell, such as, for example, a P5 sequence (or a portion thereof) for Illumina® sequencing. In some cases, the nucleic acid molecule or derivative thereof (e.g., oligonucleotide or polynucleotide generated from the nucleic acid molecule) can comprise another functional sequence, such as, for example, a P7 sequence (or a portion thereof) for attachment to a sequencing flow cell for Illumina sequencing. In some cases, the nucleic acid molecule can comprise a barcode sequence. In some cases, the nucleic acid molecule can further comprise a unique molecular identifier (UMI). In some cases, the nucleic acid molecule can comprise an R1 primer sequence for Illumina sequencing. In some cases, the nucleic acid molecule can comprise an R2 primer sequence for Illumina sequencing. Examples of such nucleic acid molecules (e.g., oligonucleotides, polynucleotides, etc.) and uses thereof, as may be used with compositions, devices, methods and systems of the present disclosure, are provided in U.S. Patent Pub. Nos. 2014/0378345 and 2015/0376609.

In some cases, the nucleic acid molecule can comprise one or more functional sequences. For example, a functional sequence can comprise a sequence for attachment to a sequencing flow cell, such as, for example, a P5 sequence for Illumina® sequencing. In some cases, the nucleic acid molecule or derivative thereof (e.g., oligonucleotide or polynucleotide generated from the nucleic acid molecule) can comprise another functional sequence, such as, for example, a P7 sequence for attachment to a sequencing flow cell for Illumina sequencing. In some cases, the functional sequence can comprise a barcode sequence or multiple barcode sequences. In some cases, the functional sequence can comprise a unique molecular identifier (UMI). In some cases, the functional sequence can comprise a primer sequence (e.g., an R1 primer sequence for Illumina sequencing, an R2 primer sequence for Illumina sequencing, etc.). In some cases, a functional sequence can comprise a partial sequence, such as a partial barcode sequence, partial anchoring sequence, partial sequencing primer sequence (e.g., partial R1 sequence, partial R2 sequence, etc.), a partial sequence configured to attach to the flow cell of a sequencer (e.g., partial P5 sequence, partial P7 sequence, etc.), or a partial sequence of any other type of sequence described elsewhere herein. A partial sequence may contain a contiguous or continuous portion or segment, but not all, of a full sequence, for example. In some cases, a downstream procedure may extend the partial sequence, or derivative thereof, to achieve a full sequence of the partial sequence, or derivative thereof.

Examples of such nucleic acid molecules (e.g., oligonucleotides, polynucleotides, etc.) and uses thereof, as may be used with compositions, devices, methods and systems of the present disclosure, are provided in U.S. Patent Pub. Nos. 2014/0378345 and 2015/0376609.

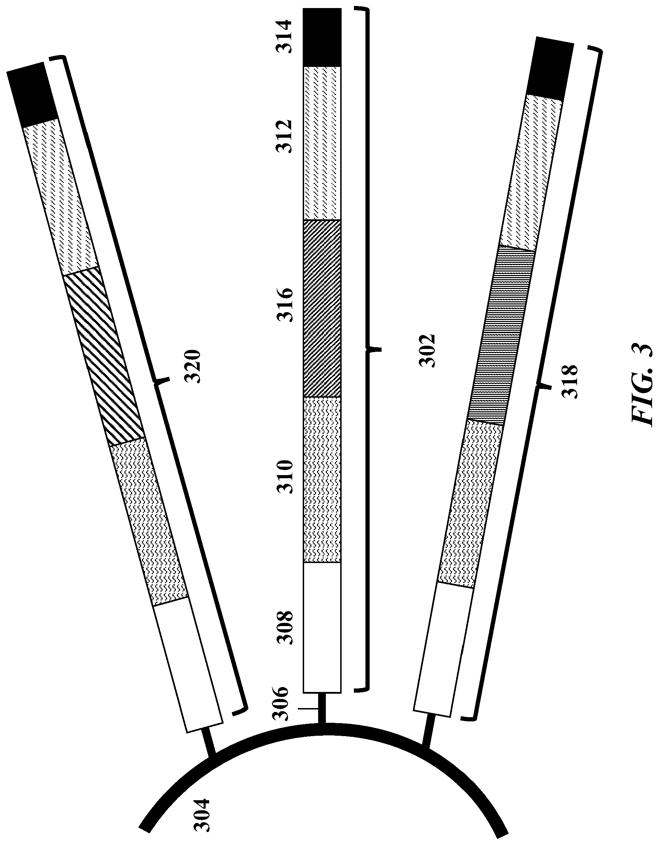

FIG. 3 illustrates an example of a barcode carrying bead. A nucleic acid molecule 302, such as an oligonucleotide, can be coupled to a bead 304 by a releasable linkage 306, such as, for example, a disulfide linker. The same bead 304 may be coupled (e.g., via releasable linkage) to one or more other nucleic acid molecules 318, 320. The nucleic acid molecule 302 may be or comprise a barcode. As noted elsewhere herein, the structure of the barcode may comprise a number of sequence elements. The nucleic acid molecule 302 may comprise a functional sequence 308 that may be used in subsequent processing. For example, the functional sequence 308 may include one or more of a sequencer specific flow cell attachment sequence (e.g., a P5 sequence for Illumina® sequencing systems) and a sequencing primer sequence (e.g., a R1 primer for Illumina® sequencing systems), or partial sequence(s) thereof. The nucleic acid molecule 302 may comprise a barcode sequence 310 for use in barcoding the sample (e.g., DNA, RNA, protein, etc.). In some cases, the barcode sequence 310 can be bead-specific such that the barcode sequence 310 is common to all nucleic acid molecules (e.g., including nucleic acid molecule 302) coupled to the same bead 304. Alternatively or in addition, the barcode sequence 310 can be partition-specific such that the barcode sequence 310 is common to all nucleic acid molecules coupled to one or more beads that are partitioned into the same partition. The nucleic acid molecule 302 may comprise a specific priming sequence 312, such as an mRNA specific priming sequence (e.g., poly-T sequence), a targeted priming sequence, and/or a random priming sequence. The nucleic acid molecule 302 may comprise an anchoring sequence 314 to ensure that the specific priming sequence 312 hybridizes at the sequence end (e.g., of the mRNA). For example, the anchoring sequence 314 can include a random short sequence of nucleotides, such as a 1-mer, 2-mer, 3-mer or longer sequence, which can ensure that a poly-T segment is more likely to hybridize at the sequence end of the poly-A tail of the mRNA.

The nucleic acid molecule 302 may comprise a unique molecular identifying sequence 316 (e.g., unique molecular identifier (UMI)). In some cases, the unique molecular identifying sequence 316 may comprise from about 5 to about 8 nucleotides. Alternatively, the unique molecular identifying sequence 316 may compress less than about 5 or more than about 8 nucleotides. The unique molecular identifying sequence 316 may be a unique sequence that varies across individual nucleic acid molecules (e.g., 302, 318, 320, etc.) coupled to a single bead (e.g., bead 304). In some cases, the unique molecular identifying sequence 316 may be a random sequence (e.g., such as a random N-mer sequence). For example, the UMI may provide a unique identifier of the starting mRNA molecule that was captured, in order to allow quantitation of the number of original expressed RNA. As will be appreciated, although FIG. 3 shows three nucleic acid molecules 302, 318, 320 coupled to the surface of the bead 304, an individual bead may be coupled to any number of individual nucleic acid molecules, for example, from one to tens to hundreds of thousands or even millions of individual nucleic acid molecules. The respective barcodes for the individual nucleic acid molecules can comprise both common sequence segments or relatively common sequence segments (e.g., 308, 310, 312, etc.) and variable or unique sequence segments (e.g., 316) between different individual nucleic acid molecules coupled to the same bead.

In operation, an analyte carrier and/or a biological particle (e.g., cell, DNA, RNA, etc.) can be co-partitioned along with a barcode bearing bead 304. The nucleic acid barcode molecules 302, 318, 320 can be released from the bead 304 in the partition. By way of example, in the context of analyzing sample RNA, the poly-T segment (e.g., 312) of one of the released nucleic acid molecules (e.g., 302) can hybridize to the poly-A tail of a mRNA molecule. Reverse transcription may result in a cDNA transcript of the mRNA, but which transcript includes each of the sequence segments 308, 310, 316 of the nucleic acid molecule 302. Because the nucleic acid molecule 302 comprises an anchoring sequence 314, it will more likely hybridize to and prime reverse transcription at the sequence end of the poly-A tail of the mRNA. Within any given partition, all of the cDNA transcripts of the individual mRNA molecules may include a common barcode sequence segment **310.** However, the transcripts made from the different mRNA molecules within a given partition may vary at the unique molecular identifying sequence **312** segment (e.g., UMI segment). Beneficially, even following any subsequent amplification of the contents of a given partition, the number of different UMIs can be indicative of the quantity of mRNA originating from a given partition, and thus from the analyte carrier and/or biological particle (e.g., cell). As noted above, the transcripts can be amplified, cleaned up and sequenced to identify the sequence of the cDNA transcript of the mRNA, as well as to sequence the barcode segment and the UMI segment. While a poly-T primer sequence is described, other targeted or random priming sequences may also be used in priming the reverse transcription reaction. Likewise, although described as releasing the barcoded oligonucleotides into the partition, in some cases, the nucleic acid molecules bound to the bead (e.g., gel bead) may be used to hybridize and capture the mRNA on the solid phase of the bead, for example, in order to facilitate the separation of the RNA from other cell contents. In such cases, further processing may be performed, in the partitions or outside the partitions (e.g., in bulk). The RNA molecules on the beads may be subjected to reverse transcription or other nucleic acid processing, additional adapter sequences may be added to the barcoded nucleic acid molecules, or other nucleic acid reactions (e.g., amplification, nucleic acid extension) may be performed. The beads or products thereof (e.g., barcoded nucleic acid molecules) may be collected from the partitions, and/or pooled together and subsequently subjected to clean up and further characterization (e.g., sequencing).

The operations described herein may be performed at any useful or convenient step. The beads comprising nucleic acid barcode molecules may be introduced into a partition (e.g., well or droplet) prior to, during, or following introduction of a sample into the partition. The nucleic acid molecules of a sample may be subjected to barcoding, which may occur on the bead (in cases where the nucleic acid molecules remain coupled to the bead) or following release of the nucleic acid barcode molecules into the partition. In cases where the nucleic acid molecules from the sample remain attached to the bead, the beads from various partitions may be collected, pooled, and subjected to further processing (e.g., reverse transcription, adapter attachment, amplification, clean up, sequencing). The processing may occur in the partition. For example, conditions sufficient for barcoding, adapter attachment, reverse transcription, or other nucleic acid processing operations may be provided in the partition and performed prior to clean up and sequencing.

A bead may comprise a capture sequence or binding sequence configured to bind to a corresponding capture sequence or binding sequence. A bead may comprise a plurality of different capture sequences or binding sequences configured to bind to different respective corresponding capture sequences or binding sequences. For example, a bead may comprise a first subset of one or more capture sequences each configured to bind to a first corresponding capture sequence, a second subset of one or more capture sequences each configured to bind to a second corresponding capture sequence, a third subset of one or more capture sequences each configured to bind to a third corresponding capture sequence, and etc. A bead may comprise any number of different capture sequences. A bead may comprise at least 2, 3, 4, 5, 6, 7, 8, 9, 10 or more different capture sequences or binding sequences configured to bind to different respective capture sequences or binding sequences, respectively. Alternatively or in addition, a bead may comprise at most about 10, 9, 8, 7, 6, 5, 4, 3, or 2 different capture sequences or binding sequences configured to bind to different respective capture sequences or binding sequences. The different capture sequences or binding sequences may be configured to facilitate analysis of a same type of analyte. The different capture sequences or binding sequences may be configured to facilitate analysis of different types of analytes (with the same bead). The capture sequence may be designed to attach to a corresponding capture sequence. Beneficially, such corresponding capture sequence may be introduced to, or otherwise induced in, an analyte carrier and/or biological particle (e.g., cell, cell bead, etc.) for performing different assays in various formats (e.g., barcoded antibodies comprising the corresponding capture sequence, barcoded MHC dextramers comprising the corresponding capture sequence, barcoded guide RNA molecules comprising the corresponding capture sequence, etc.), such that the corresponding capture sequence may later interact with the capture sequence associated with the bead. A capture sequence coupled to a bead (or other microcapsule and/or support) may be configured to attach to a linker molecule, such as a splint molecule, wherein the linker molecule is configured to couple the bead (or other microcapsule and/or support) to other molecules through the linker molecule, such as to one or more analytes or one or more other linker molecules.

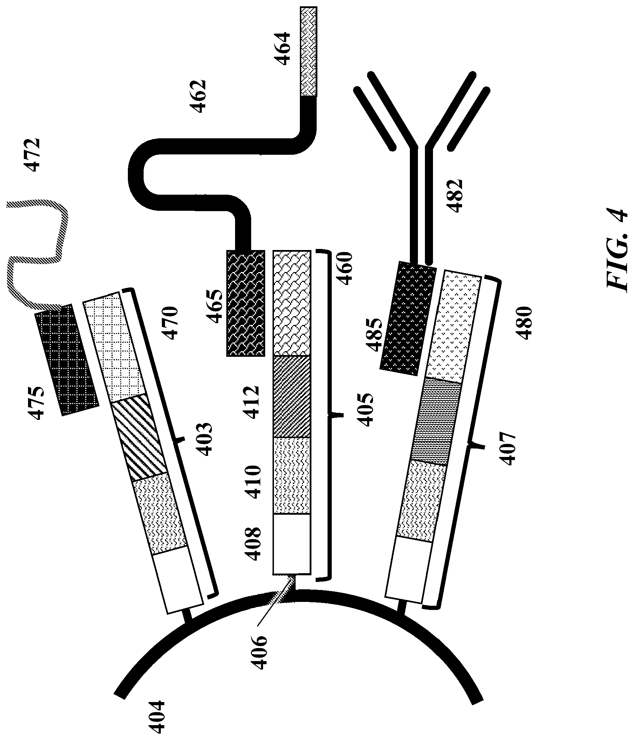

**FIG. 4** illustrates another example of a barcode carrying bead. A nucleic acid molecule **405,** such as an oligonucleotide, can be coupled to a bead **404** by a releasable linkage **406,** such as, for example, a disulfide linker. The nucleic acid molecule **405** may comprise a first capture sequence **460.** The same bead **404** may be coupled (e.g., via releasable linkage) to one or more other nucleic acid molecules **403, 407** comprising other capture sequences. The nucleic acid molecule **405** may be or comprise a barcode. As noted elsewhere herein, the structure of the barcode may comprise a number of sequence elements, such as a functional sequence **408** (e.g., flow cell attachment sequence, sequencing primer sequence, etc.), a barcode sequence **410** (e.g., bead-specific sequence common to bead, partition-specific sequence common to partition, etc.), and a unique molecular identifier **412** (e.g., unique sequence within different molecules attached to the bead), or partial sequences thereof. The capture sequence **460** may be configured to attach to a corresponding capture sequence **465.** The corresponding capture sequence **465** may be coupled to another molecule that may be an analyte or an intermediary carrier. For example, as illustrated in **FIG. 4,** the corresponding capture sequence **465** is coupled to a guide RNA molecule **462** comprising a target sequence **464,** wherein the target sequence **464** is configured to attach to the analyte. Another oligonucleotide molecule **407** attached to the bead **404** comprises a second capture sequence **480** which is configured to attach to a second corresponding capture sequence **485**. As illustrated in **FIG. 4,** the second corresponding capture sequence **485** is coupled to an antibody **482**. In some cases, the antibody **482** may have binding specificity to an analyte (e.g., surface protein). Alternatively, the antibody **482** may not have binding specificity. Another oligonucleotide molecule **403** attached to the bead **404** comprises a third capture sequence **470** which is configured to attach to a second corresponding capture sequence **475**. As illustrated in **FIG. 4,** the third corresponding capture sequence **475** is coupled to a molecule **472**. The molecule **472** may or may not be configured to target an analyte. The other oligonucleotide molecules **403, 407** may comprise the other sequences (e.g., functional sequence, barcode sequence, UMI, etc.) described with respect to oligonucleotide molecule **405**. While a single oligonucleotide molecule comprising each capture sequence is illustrated in **FIG. 4,** it will be appreciated that, for each capture sequence, the bead may comprise a set of one or more oligonucleotide molecules each comprising the capture sequence. For example, the bead may comprise any number of sets of one or more different capture sequences. Alternatively or in addition, the bead **404** may comprise other capture sequences. Alternatively or in addition, the bead **404** may comprise fewer types of capture sequences (e.g., two capture sequences). Alternatively or in addition, the bead **404** may comprise oligonucleotide molecule(s) comprising a priming sequence, such as a specific priming sequence such as an mRNA specific priming sequence (e.g., poly-T sequence), a targeted priming sequence, and/or a random priming sequence, for example, to facilitate an assay for gene expression.

In operation, the barcoded oligonucleotides may be released (e.g., in a partition), as described elsewhere herein. Alternatively, the nucleic acid molecules bound to the bead (e.g., gel bead) may be used to hybridize and capture analytes (e.g., one or more types of analytes) on the solid phase of the bead.

In some cases, precursors comprising a functional group that is reactive or capable of being activated such that it becomes reactive can be polymerized with other precursors to generate gel beads comprising the activated or activatable functional group. The functional group may then be used to attach additional species (e.g., disulfide linkers, primers, other oligonucleotides, etc.) to the gel beads. For example, some precursors comprising a carboxylic acid (COOH) group can co-polymerize with other precursors to form a gel bead that also comprises a COOH functional group. In some cases, acrylic acid (a species comprising free COOH groups), acrylamide, and bis(acryloyl)cystamine can be co-polymerized together to generate a gel bead comprising free COOH groups. The COOH groups of the gel bead can be activated (e.g., via 1-Ethyl-3-(3-dimethylaminopropyl)carbodiimide (EDC) and N-Hydroxysuccinimide (NHS) or 4-(4,6-Dimethoxy-1,3,5-triazin-2-yl)-4-methylmorpholinium chloride (DMTMM)) such that they are reactive (e.g., reactive to amine functional groups where EDC/NHS or DMTMM are used for activation). The activated COOH groups can then react with an appropriate species (e.g., a species comprising an amine functional group where the carboxylic acid groups are activated to be reactive with an amine functional group) comprising a moiety to be linked to the bead.

Beads comprising disulfide linkages in their polymeric network may be functionalized with additional species via reduction of some of the disulfide linkages to free thiols. The disulfide linkages may be reduced via, for example, the action of a reducing agent (e.g., DTT, TCEP, etc.) to generate free thiol groups, without dissolution of the bead. Free thiols of the beads can then react with free thiols of a species or a species comprising another disulfide bond (e.g., via thiol-disulfide exchange) such that the species can be linked to the beads (e.g., via a generated disulfide bond). In some cases, free thiols of the beads may react with any other suitable group. For example, free thiols of the beads may react with species comprising an acrydite moiety. The free thiol groups of the beads can react with the acrydite via Michael addition chemistry, such that the species comprising the acrydite is linked to the bead. In some cases, uncontrolled reactions can be prevented by inclusion of a thiol capping agent such as N-ethylmalieamide or iodoacetate.

Activation of disulfide linkages within a bead can be controlled such that only a small number of disulfide linkages are activated. Control may be exerted, for example, by controlling the concentration of a reducing agent used to generate free thiol groups and/or concentration of reagents used to form disulfide bonds in bead polymerization. In some cases, a low concentration (e.g., molecules of reducing agent: gel bead ratios of less than or equal to about 1:100,000,000,000, less than or equal to about 1:10,000,000,000, less than or equal to about 1:1,000,000,000, less than or equal to about 1:100,000,000, less than or equal to about 1:10,000,000, less than or equal to about 1:1,000,000, less than or equal to about 1:100,000, less than or equal to about 1:10,000) of reducing agent may be used for reduction. Controlling the number of disulfide linkages that are reduced to free thiols may be useful in ensuring bead structural integrity during functionalization. In some cases, optically-active agents, such as fluorescent dyes may be coupled to beads via free thiol groups of the beads and used to quantify the number of free thiols present in a bead and/or track a bead.

In some cases, addition of moieties to a gel bead after gel bead formation may be advantageous. For example, addition of an oligonucleotide (e.g., barcoded oligonucleotide) after gel bead formation may avoid loss of the species during chain transfer termination that can occur during polymerization. Moreover, smaller precursors (e.g., monomers or cross linkers that do not comprise side chain groups and linked moieties) may be used for polymerization and can be minimally hindered from growing chain ends due to viscous effects. In some cases, functionalization after gel bead synthesis can minimize exposure of species (e.g., oligonucleotides) to be loaded with potentially damaging agents (e.g.,

free radicals) and/or chemical environments. In some cases, the generated gel may possess an upper critical solution temperature (UCST) that can permit temperature driven swelling and collapse of a bead. Such functionality may aid in oligonucleotide (e.g., a primer) infiltration into the bead during subsequent functionalization of the bead with the oligonucleotide. Post-production functionalization may also be useful in controlling loading ratios of species in beads, such that, for example, the variability in loading ratio is minimized. Species loading may also be performed in a batch process such that a plurality of beads can be functionalized with the species in a single batch.

A bead injected or otherwise introduced into a partition may comprise releasably, cleavably, or reversibly attached barcodes. A bead injected or otherwise introduced into a partition may comprise activatable barcodes. A bead injected or otherwise introduced into a partition may be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage may be achieved through reduction of di-sulfide bonds, use of restriction enzymes, photo-activated cleavage, or cleavage via other types of stimuli (e.g., chemical, thermal, pH, enzymatic, etc.) and/or reactions, such as described elsewhere herein. Releasable barcodes may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to, or as an alternative to the cleavable linkages between the beads and the associated molecules, such as barcode containing nucleic acid molecules (e.g., barcoded oligonucleotides), the beads may be degradable, disruptable, or dissolvable spontaneously or upon exposure to one or more stimuli (e.g., temperature changes, pH changes, exposure to particular chemical species or phase, exposure to light, reducing agent, etc.). In some cases, a bead may be dissolvable, such that material components of the beads are solubilized when exposed to a particular chemical species or an environmental change, such as a change temperature or a change in pH. In some cases, a gel bead can be degraded or dissolved at elevated temperature and/or in basic conditions. In some cases, a bead may be thermally degradable such that when the bead is exposed to an appropriate change in temperature (e.g., heat), the bead degrades. Degradation or dissolution of a bead bound to a species (e.g., a nucleic acid molecule, e.g., barcoded oligonucleotide) may result in release of the species from the bead.

As will be appreciated from the above disclosure, the degradation of a bead may refer to the disassociation of a bound or entrained species from a bead, both with and without structurally degrading the physical bead itself. For example, the degradation of the bead may involve cleavage of a cleavable linkage via one or more species and/or methods described elsewhere herein. In another example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

A degradable bead may be introduced into a partition, such as a droplet of an emulsion or a well, such that the bead degrades within the partition and any associated species (e.g., oligonucleotides) are released within the droplet when the appropriate stimulus is applied. The free species (e.g., oligonucleotides, nucleic acid molecules) may interact with other reagents contained in the partition. For example, a polyacrylamide bead comprising cystamine and linked, via a disulfide bond, to a barcode sequence, may be combined with a reducing agent within a droplet of a water-in-oil emulsion. Within the droplet, the reducing agent can break the various disulfide bonds, resulting in bead degradation and release of the barcode sequence into the aqueous, inner environment of the droplet. In another example, heating of a droplet comprising a bead-bound barcode sequence in basic solution may also result in bead degradation and release of the attached barcode sequence into the aqueous, inner environment of the droplet.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In some cases, the pre-defined concentration of the primer can be limited by the process of producing nucleic acid molecule (e.g., oligonucleotide) bearing beads.

In some cases, beads can be non-covalently loaded with one or more reagents. The beads may be non-covalently loaded by subjecting the beads to conditions sufficient to swell the beads, allowing sufficient time for the reagents to diffuse into the interiors of the beads, and subjecting the beads to conditions sufficient to de-swell the beads. The swelling of the beads may be accomplished by placing the beads in a thermodynamically favorable solvent, subjecting the beads to a higher or lower temperature, subjecting the beads to a higher or lower ion concentration, and/or subjecting the beads to an electric field. The swelling of the beads may be accomplished by various swelling methods. The de-swelling of the beads may be accomplished by transferring the beads in a thermodynamically unfavorable solvent, subjecting the beads to lower or high temperatures, subjecting the beads to a lower or higher ion concentration, and/or removing an electric field. The de-swelling of the beads may be accomplished by various de-swelling methods. Transferring the beads may cause pores in the bead to shrink. The shrinking may then hinder reagents within the beads from diffusing out of the interiors of the beads. The hindrance may be due to steric interactions between the reagents and the interiors of the beads. The transfer may be accomplished microfluidically. The transfer may be achieved by moving the beads from one co-flowing solvent stream to a different co-flowing solvent stream. The swellability and/or pore size of the beads may be adjusted by changing the polymer composition of the bead.

In some cases, an acrydite moiety linked to a precursor, another species linked to a precursor, or a precursor itself can comprise a labile bond, such as chemically, thermally, or photo-sensitive bond e.g., disulfide bond, UV sensitive bond, or the like. Once acrydite moieties or other moieties comprising a labile bond are incorporated into a bead, the bead may also comprise the labile bond. The labile bond may be, for example, useful in reversibly linking (e.g., covalently linking) species (e.g., barcodes, primers, etc.) to a bead. In some cases, a thermally labile bond may include a nucleic acid hybridization based attachment, e.g., where an oligonucleotide is hybridized to a complementary sequence that is attached to the bead, such that thermal melting of the hybrid releases the oligonucleotide, e.g., a barcode containing sequence, from the microcapsule and/or support (e.g., a bead such as a gel bead).

The addition of multiple types of labile bonds to a gel bead may result in the generation of a bead capable of responding to varied stimuli. Each type of labile bond may be sensitive to an associated stimulus (e.g., chemical stimulus, light, temperature, enzymatic, etc.) such that release of species attached to a bead via each labile bond may be controlled by the application of the appropriate stimulus. Such functionality may be useful in controlled release of species from a gel bead. In some cases, another species comprising a labile bond may be linked to a gel bead after gel bead formation via, for example, an activated functional group of the gel bead as described above. As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

In some cases, a species (e.g., oligonucleotide molecules comprising barcodes) that are attached to a solid microcapsule and/or support (e.g., a bead) may comprise a U-excising element that allows the species to release from the bead. In some cases, the U-excising element may comprise a single-stranded DNA (ssDNA) sequence that contains at least one uracil. The species may be attached to a solid microcapsule and/or support via the ssDNA sequence containing the at least one uracil. The species may be released by a combination of uracil-DNA glycosylase (e.g., to remove the uracil) and an endonuclease (e.g., to induce an ssDNA break). If the endonuclease generates a 5' phosphate group from the cleavage, then additional enzyme treatment may be included in downstream processing to eliminate the phosphate group, e.g., prior to ligation of additional sequencing handle elements, e.g., Illumina full P5 sequence, partial P5 sequence, full R1 sequence, and/or partial R1 sequence.

The barcodes that are releasable as described herein may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to thermally cleavable bonds, disulfide bonds and UV sensitive bonds, other non-limiting examples of labile bonds that may be coupled to a precursor or bead include an ester linkage (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), or a phosphodiester linkage (e.g., cleavable via a nuclease (e.g., DNAase)). A bond may be cleavable via other nucleic acid molecule targeting enzymes, such as restriction enzymes (e.g., restriction endonucleases), as described further below.

Species may be encapsulated in beads during bead generation (e.g., during polymerization of precursors). Such species may or may not participate in polymerization. Such species may be entered into polymerization reaction mixtures such that generated beads comprise the species upon bead formation. In some cases, such species may be added to the gel beads after formation. Such species may include, for example, nucleic acid molecules (e.g., oligonucleotides), reagents for a nucleic acid amplification reaction (e.g., primers, polymerases, dNTPs, co-factors (e.g., ionic co-factors), buffers) including those described herein, reagents for enzymatic reactions (e.g., enzymes, co-factors, substrates, buffers), reagents for nucleic acid modification reactions such as polymerization, ligation, or digestion, and/or reagents for template preparation (e.g., tagmentation) for one or more sequencing platforms (e.g., Nextera® for Illumina®). Such species may include one or more enzymes described herein, including without limitation, polymerase, reverse transcriptase, restriction enzymes (e.g., endonuclease), transposase, ligase, proteinase K, DNAse, etc. Such species may include one or more reagents described elsewhere herein (e.g., lysis agents, inhibitors, inactivating agents, chelating agents, stimulus). Trapping of such species may be controlled by the polymer network density generated during polymerization of precursors, control of ionic charge within the gel bead (e.g., via ionic species linked to polymerized species), or by the release of other species. Encapsulated species may be released from a bead upon bead degradation and/or by application of a stimulus capable of releasing the species from the bead. Alternatively or in addition, species may be partitioned in a partition (e.g., droplet) during or subsequent to partition formation. Such species may include, without limitation, the abovementioned species that may also be encapsulated in a bead.

A degradable bead may comprise one or more species with a labile bond such that, when the bead/species is exposed to the appropriate stimuli, the bond is broken and the bead degrades. The labile bond may be a chemical bond (e.g., covalent bond, ionic bond) or may be another type of physical interaction (e.g., van der Waals interactions, dipole-dipole interactions, etc.). In some cases, a crosslinker used to generate a bead may comprise a labile bond. Upon exposure to the appropriate conditions, the labile bond can be broken and the bead degraded. For example, upon exposure of a polyacrylamide gel bead comprising cystamine crosslinkers to a reducing agent, the disulfide bonds of the cystamine can be broken and the bead degraded.

A degradable bead may be useful in more quickly releasing an attached species (e.g., a nucleic acid molecule, a barcode sequence, a primer, etc.) from the bead when the appropriate stimulus is applied to the bead as compared to a bead that does not degrade. For example, for a species bound to an inner surface of a porous bead or in the case of an encapsulated species, the species may have greater mobility and accessibility to other species in solution upon degradation of the bead. In some cases, a species may also be attached to a degradable bead via a degradable linker (e.g., disulfide linker). The degradable linker may respond to the same stimuli as the degradable bead or the two degradable species may respond to different stimuli. For example, a barcode sequence may be attached, via a disulfide bond, to a polyacrylamide bead comprising cystamine. Upon exposure of the barcoded-bead to a reducing agent, the bead degrades and the barcode sequence is released upon breakage of both the disulfide linkage between the barcode sequence and the bead and the disulfide linkages of the cystamine in the bead.

As will be appreciated from the above disclosure, while referred to as degradation of a bead, as noted above, that degradation may refer to the disassociation of a bound or entrained species from a bead, both with and without structurally degrading the physical bead itself. For example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

Where degradable beads are provided, it may be beneficial to avoid exposing such beads to the stimulus or stimuli that cause such degradation prior to a given time, in order to, for example, avoid premature bead degradation and issues that arise from such degradation, including for example poor flow characteristics and aggregation. By way of example, where beads comprise reducible cross-linking groups, such as disulfide groups, it will be desirable to avoid contacting such beads with reducing agents, e.g., DTT or other disulfide cleaving reagents. In such cases, treatment to the beads described herein will, in some cases be provided free of reducing agents, such as DTT. Because reducing agents are often provided in commercial enzyme preparations, it may be desirable to provide reducing agent free (or DTT free) enzyme preparations in treating the beads described herein. Examples of such enzymes include, e.g., polymerase enzyme preparations, reverse transcriptase enzyme preparations, ligase enzyme preparations, as well as many other enzyme preparations that may be used to treat the beads described herein. The terms "reducing agent free" or "DTT free" preparations can refer to a preparation having less than about 1/10th, less than about 1/50th, or even less than about 1/100th of the lower ranges for such materials used in degrading the beads. For example, for DTT, the reducing agent free preparation can have less than about 0.01 millimolar (mM), 0.005 mM, 0.001 mM DTT, 0.0005 mM DTT, or even less than about 0.0001 mM DTT. In many cases, the amount of DTT can be undetectable.

Numerous chemical triggers may be used to trigger the degradation of beads. Examples of these chemical changes may comprise pH-mediated changes to the integrity of a component within the bead, degradation of a component of a bead via cleavage of cross-linked bonds, and depolymerization of a component of a bead.

A bead may be formed from materials that comprise degradable chemical crosslinkers, such as BAC or cystamine. Degradation of such degradable crosslinkers may be accomplished through a number of mechanisms. In some examples, a bead may be contacted with a chemical degrading agent that may induce oxidation, reduction or other chemical changes. For example, a chemical degrading agent may be a reducing agent, such as dithiothreitol (DTT). Additional examples of reducing agents may include β-mercaptoethanol, (2S)-2-amino-1,4-dimercaptobutane (dithiobutylamine or DTBA), tris(2-carboxyethyl) phosphine (TCEP), or combinations thereof. A reducing agent may degrade the disulfide bonds formed between gel precursors forming the bead, and thus, degrade the bead. In other cases, a change in pH of a solution, such as an increase in pH, may trigger degradation of a bead. In other cases, exposure to an aqueous solution, such as water, may trigger hydrolytic degradation, and thus degradation of the bead. In some cases, any combination of stimuli may trigger degradation of a bead. For example, a change in pH may enable a chemical agent (e.g., DTT) to become an effective reducing agent.

Beads may also be induced to release their contents upon the application of a thermal stimulus. A change in temperature can cause a variety of changes to a bead. For example, heat can cause a solid bead to liquefy. A change in heat may cause melting of a bead such that a portion of the bead degrades. In other cases, heat may increase the internal pressure of the bead components such that the bead ruptures or explodes. Heat may also act upon heat-sensitive polymers used as materials to construct beads.

Any suitable agent may degrade beads. Changes in temperature or pH may be used to degrade thermo-sensitive or pH-sensitive bonds within beads. Chemical degrading agents may be used to degrade chemical bonds within beads by oxidation, reduction or other chemical changes. For example, a chemical degrading agent may be a reducing agent, such as DTT, wherein DTT may degrade the disulfide bonds formed between a crosslinker and gel precursors, thus degrading the bead. A reducing agent may be added to degrade the bead, which may or may not cause the bead to release its contents. Examples of reducing agents may include dithiothreitol (DTT), β-mercaptoethanol, (2S)-2-amino-1,4-dimercaptobutane (dithiobutylamine or DTBA), tris(2-carboxyethyl) phosphine (TCEP), or combinations thereof. The reducing agent may be present at a concentration of about 0.1mM, 0.5mM, 1mM, 5mM, 10mM. The reducing agent may be present at a concentration of at least about 0.1mM, 0.5mM, 1mM, 5mM, 10mM, or greater than 10 mM. The reducing agent may be present at concentration of at most about 10mM, 5mM, 1mM, 0.5mM, 0.1mM, or less.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In some cases, the pre-defined concentration of the primer can be limited by the process of producing oligonucleotide bearing beads.

In some examples, a partition of the plurality of partitions may comprise a single analyte carrier and/or biological particle (e.g., a single cell or a single nucleus of a cell). In some examples, a partition of the plurality of partitions may comprise multiple analyte carriers and/or biological particles. Such partitions may be referred to as multiply occupied partitions, and may comprise, for example, two, three, four or more cells and/or microcapsules and/or supports (e.g., beads) comprising barcoded nucleic acid molecules (e.g., oligonucleotides) within a single partition. Accordingly, as noted above, the flow characteristics of the analyte carrier and/or biological particle and/or bead containing fluids and partitioning fluids may be controlled to provide for such multiply occupied partitions. In particular, the flow parameters may be controlled to provide a given occupancy rate at greater than about 50% of the partitions, greater than about 75%, and in some cases greater than about 80%, 90%, 95%, or higher.

In some cases, additional microcapsules and/or supports (e.g., microcapsules, beads) can be used to deliver additional reagents to a partition. In such cases, it may be advantageous to introduce different beads into a common channel or droplet generation junction, from different bead sources (e.g., containing different associated reagents) through different channel inlets into such common channel or droplet generation junction. In such cases, the flow and frequency of the different beads into the channel or junction may be controlled to provide for a certain ratio of microcapsules and/or supports from each source, while ensuring a given pairing or combination of such beads into a partition with a given number of analyte carriers and/or biological particles (e.g., one biological particle and one bead per partition).

The partitions described herein may comprise small volumes, for example, less than about 10 microliters (μL), 5μL, 1μL, 900 picoliters (pL), 800 pL, 700 pL, 600 pL, 500 pL, 400pL, 300 pL, 200 pL, 100pL, 50 pL, 20 pL, 10 pL, 1 pL, 500 nanoliters (nL), 100 nL, 50 nL, or less.

For example, in the case of droplet based partitions, the droplets may have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. Where co-partitioned with microcapsules and/or microcapsules and/or supports, it will be appreciated that the sample fluid volume, e.g., including co-partitioned analyte carriers and/or biological particles and/or beads, within the partitions may be less than about 90% of the above described volumes, less than about 80%, less than about 70%, less than about 60%, less than about 50%, less than about 40%, less than about 30%, less than about 20%, or less than about 10% of the above described volumes.

As is described elsewhere herein, partitioning species may generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions may comprise both unoccupied partitions (e.g., empty partitions) and occupied partitions.

**Multiplexing and 5' Modules**

The present disclosures provides methods and systems for multiplexing, and otherwise increasing throughput in, analysis. For example, a single or integrated process workflow may permit the processing, identification, and/or analysis of more or multiple analytes, more or multiple types of analytes, and/or more or multiple types of analyte characterizations.

For example, in the methods and systems described herein, one or more labelling agents capable of binding to or otherwise coupling to one or more cell features may be used to characterize analyte carriers and/or biological particles and/or cell features. Cell features may include cell surface features. Cell surface features may include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. Cell features may include intracellular analytes, such as proteins, protein modifications (e.g., phosphorylation status or other post-translational modifications), nuclear proteins, nuclear membrane proteins, or any combination thereof. A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have a first reporter oligonucleotide coupled thereto, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat. Pub. 20190367969.

In a particular example, a library of potential cell feature labelling agents may be provided, where the respective cell feature labelling agents are associated with nucleic acid reporter molecules, such that a different reporter oligonucleotide sequence is associated with each labelling agent capable of binding to a specific cell feature. Different members of the library may be characterized by the presence of a different oligonucleotide sequence label. For example, an antibody capable of binding to a first protein may have associated with it a first reporter oligonucleotide sequence, while an antibody capable of binding to a second protein may have a different reporter oligonucleotide sequence associated with it. The presence of the particular oligonucleotide sequence may be indicative of the presence of a particular antibody or cell feature which may be recognized or bound by the particular antibody.

Labelling agents capable of binding to or otherwise coupling to one or more analyte carriers and/or biological particles may be used to characterize an analyte carrier and/or a biological particle as belonging to a particular set of analyte carriers and/or biological particles. For example, labeling agents may be used to label a sample of cells or a group of cells. In this way, a group of cells may be labeled as different from another group of cells. In an example, a first group of cells may originate from a first sample and a second group of cells may originate from a second sample. Labelling agents may allow the first group and second group to have a different labeling agent (or reporter oligonucleotide associated with the labeling agent). This may, for example, facilitate multiplexing, where cells of the first group and cells of the second group may be labeled separately and then pooled together for downstream analysis. The downstream detection of a label may indicate analytes as belonging to a particular group.

For example, a reporter oligonucleotide may be linked to an antibody or an epitope binding fragment thereof, and labeling an analyte carrier and/or biological particle may comprise subjecting the antibody-linked barcode molecule or the epitope binding fragment-linked barcode molecule to conditions suitable for binding the antibody to a molecule present on a surface of the analyte carrier and/or biological particle. The binding affinity between the antibody or the epitope binding fragment thereof and the molecule present on the surface may be within a desired range to ensure that the antibody or the epitope binding fragment thereof remains bound to the molecule. For example, the binding affinity may be within a desired range to ensure that the antibody or the epitope binding fragment thereof remains bound to the molecule during various sample processing steps, such as partitioning and/or nucleic acid amplification or extension. A dissociation constant (Kd) between the antibody or an epitope binding fragment thereof and the molecule to which it binds may be less than about 100 $\mu$M, 90 $\mu$M, 80 $\mu$M, 70 $\mu$M, 60 $\mu$M, 50 $\mu$M, 40 $\mu$M, 30 $\mu$M, 20 $\mu$M, 10 $\mu$M, 9 $\mu$M, 8 $\mu$M, 7 $\mu$M, 6 $\mu$M, 5 $\mu$M, 4 $\mu$M, 3 $\mu$M, 2 $\mu$M, 1 $\mu$M, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, 10 pM, 9 pM, 8 pM, 7 pM, 6 pM, 5 pM, 4 pM, 3 pM, 2 pM, or 1 pM. For example, the dissociation constant may be less than about 10 $\mu$M.

In another example, a reporter oligonucleotide may be coupled to a cell-penetrating peptide (CPP), and labeling cells may comprise delivering the CPP coupled reporter oligonucleotide into an analyte carrier and/or biological particle. Labeling analyte carriers and/or biological particles may comprise delivering the CPP conjugated oligonucleotide into a cell and/or cell bead by the cell-penetrating peptide. A cell-penetrating peptide that can be used in the methods provided herein can comprise at least one non-functional cysteine residue, which may be either free or derivatized to form a disulfide link with an oligonucleotide that has been modified for such linkage. Non-limiting examples of cell-penetrating peptides that may be used include penetratin, transportan, plsl, TAT(48-60), pVEC, MTS, and MAP. Cell-penetrating peptides useful in the methods provided herein can have the capability of inducing cell penetration for at least about 30%, 40%, 50%, 60%, 70%, 80%, 90%, 95%, 96%, 97%, 98%, 99%, or 100% of cells of a cell population. The cell-penetrating peptide may be an arginine-rich peptide transporter. The cell-penetrating peptide may be Penetratin or the Tat peptide.

In another example, a reporter oligonucleotide may be coupled to a fluorophore or dye, and labeling cells may comprise subjecting the fluorophore-linked barcode molecule to conditions suitable for binding the fluorophore to the surface of the analyte carrier and/or biological particle. Fluorophores may interact strongly with lipid bilayers and labeling analyte carriers and/or biological particles may comprise subjecting the fluorophore-linked barcode molecule to conditions such that the fluorophore binds to or is inserted into a membrane of the analyte carrier and/or biological particle. In some cases, the fluorophore is a water-soluble, organic fluorophore. The fluorophore may be Alexa 532 maleimide, tetramethylrhodamine-5-maleimide (TMR maleimide), BODIPY-TMR maleimide, Sulfo-Cy3 maleimide, Alexa 546 carboxylic acid/succinimidyl ester, Atto 550 maleimide, Cy3 carboxylic acid/succinimidyl ester, Cy3B carboxylic acid/succinimidyl ester, Atto 565 biotin, Sulforhodamine B, Alexa 594 maleimide, Texas Red maleimide, Alexa 633 maleimide, Abberior STAR 635P azide, Atto 647N maleimide, Atto 647 SE, or Sulfo-Cy5 maleimide. See, e.g., Hughes L D, et al. PLoS One. 2014 Feb. 4; 9(2):e87649, for a description of organic fluorophores.

A reporter oligonucleotide may be coupled to a lipophilic molecule, and labeling analyte carriers and/or biological particles may comprise delivering the nucleic acid barcode molecule to a membrane of the analyte carrier and/or biological particle or a nuclear membrane by the lipophilic molecule. Lipophilic molecules can associate with and/or insert into lipid membranes such as cell membranes and nuclear membranes. In some cases, the insertion can be reversible. In some cases, the association between the lipophilic molecule and analyte carrier and/or biological particle may be such that the analyte carrier and/or biological particle retains the lipophilic molecule (e.g., and associated components, such as nucleic acid barcode molecules, thereof) during subsequent processing (e.g., partitioning, cell permeabilization, amplification, pooling, etc.). The reporter nucleotide may enter into the intracellular space and/or a cell nucleus.

A reporter oligonucleotide may be part of a nucleic acid molecule comprising any number of functional sequences, as described elsewhere herein, such as a target capture sequence, a random primer sequence, and the like, and coupled to another nucleic acid molecule that is, or is derived from, the analyte.

Prior to partitioning, the cells may be incubated with the library of labelling agents, that may be labelling agents to a broad panel of different cell features, e.g., receptors, proteins, etc., and which include their associated reporter oligonucleotides. Unbound labelling agents may be washed from the cells, and the cells may then be co-partitioned (e.g., into droplets or wells ) along with partition-specific barcode oligonucleotides (e.g., attached to a microcapsule and/or support, such as a bead or gel bead) as described elsewhere herein. As a result, the partitions may include the cell or cells, as well as the bound labelling agents and their known, associated reporter oligonucleotides.

For example, to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide. For example, the first plurality of the labeling agent and second plurality of the labeling agent may interact with different cells, cell populations or samples, allowing a particular report oligonucleotide to indicate a particular cell population (or cell or sample) and cell feature. In this way, different samples or groups can be independently processed and subsequently combined together for pooled analysis (e.g., partition-based barcoding as described elsewhere herein). See, e.g., U.S. Pat. Pub. 20190323088.

As described elsewhere herein, libraries of labelling agents may be associated with a particular cell feature as well as be used to identify analytes as originating from a particular analyte carrier and/or biological particle, population, or sample. The analyte carriers and/or biological particles may be incubated with a plurality of libraries and a given analyte carrier and/or biological particle may comprise multiple labelling agents. For example, a cell may comprise coupled thereto a lipophilic labeling agent and an antibody. The lipophilic labeling agent may indicate that the cell is a member of a particular cell sample, whereas the antibody may indicate that the cell comprises a particular analyte. In this manner, the reporter oligonucleotides and labelling agents may allow multi-analyte, multiplexed analyses to be performed.

These reporter oligonucleotides may comprise nucleic acid barcode sequences that permit identification of the labelling agent which the reporter oligonucleotide is coupled to. The use of oligonucleotides as the reporter may provide advantages of being able to generate significant diversity in terms of sequence, while also being readily attachable to most biomolecules, e.g., antibodies, etc., as well as being readily detected, e.g., using sequencing or array technologies.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment) using chemical conjugation techniques (e.g., Lightning-Link® antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715. Likewise, protein and peptide biotinylation techniques have been developed and are readily available. See, e.g., U.S. Pat.

No. 6,265,552. Furthermore, click reaction chemistry such as a Methyltetrazine-PEG5-NHS Ester reaction, a TCO-PEG4-NHS Ester reaction, or the like, may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the label agent. The labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. The reporter oligonucleotides may be releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from microcapsules and/or supports elsewhere herein. The reporter oligonucleotides described herein may include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to an oligonucleotide that is complementary to a sequence of the reporter oligonucleotide, and the oligonucleotide may be allowed to hybridize to the reporter oligonucleotide.

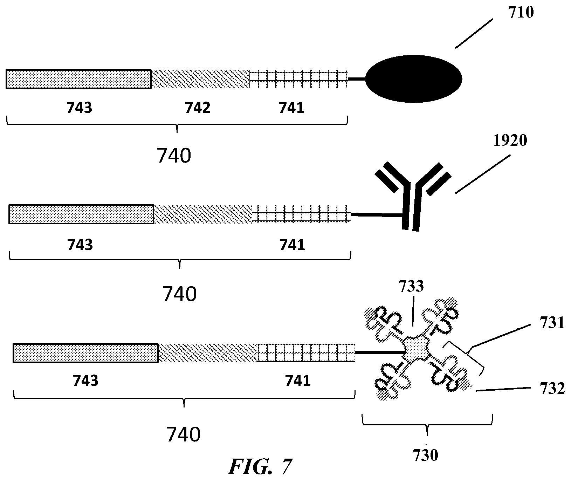

FIG. 7 describes exemplary labelling agents (710, 720, 730) comprising reporter oligonucleotides (740) attached thereto. Labelling agent 710 (e.g., any of the labelling agents described herein) is attached (either directly, e.g., covalently attached, or indirectly) to reporter oligonucleotide 740. Reporter oligonucleotide 740 may comprise barcode sequence 742 that identifies labelling agent 710. Reporter oligonucleotide 740 may also comprise one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer biding sequence (such as an R1, R2, or partial R1 or R2 sequence).

Referring to FIG. 7, reporter oligonucleotide 740 conjugated to a labelling agent (e.g., 710, 720, 730) may comprise a primer sequence 741, a barcode sequence that identifies the labelling agent (e.g., 710, 720, 730), and functional sequence 743. Functional sequence 743 may be configured to hybridize to a complementary sequence, such as a complementary sequence present on a nucleic acid barcode molecule 790 (not shown), such as those described elsewhere herein. Nucleic acid barcode molecule 790 may be attached to a microcapsule and/or support (e.g., a bead, such as a gel bead), such as those described elsewhere herein. For example, nucleic acid barcode molecule 790 may be attached to the microcapsule and/or support via a releasable linkage (e.g., comprising a labile bond), such as those described elsewhere herein. Reporter oligonucleotide 740 may comprise one or more additional functional sequences, such as those described above.

The labelling agent 710 may be a protein or polypeptide (e.g., an antigen or prospective antigen) comprising reporter oligonucleotide 740. Reporter oligonucleotide 740 may comprise barcode sequence 742 that identifies polypeptide 710 and be used to infer the presence of an analyte, e.g., a binding partner of polypeptide 710 (i.e., a molecule or compound to which polypeptide 710 can bind). The labelling agent 710 may be a lipophilic moiety (e.g., cholesterol) comprising reporter oligonucleotide 740, where the lipophilic moiety is selected such that labelling agent 710 integrates into a membrane of a cell or nucleus. Reporter oligonucleotide 740 may comprise barcode sequence 742 that identifies lipophilic moiety 710 which may be used to tag cells (e.g., groups of cells, cell samples, etc.) and may be used for multiplex analyses as described elsewhere herein. The labelling agent may be an antibody 720 (or an epitope binding fragment thereof) comprising reporter oligonucleotide 740. Reporter oligonucleotide 740 may comprise barcode sequence 742 that identifies antibody 720 and may be used to infer the presence of, e.g., a target of antibody 720 (i.e., a molecule or compound to which antibody 720 binds). Labelling agent 730 may comprise an MHC molecule 731 comprising peptide 732 and reporter oligonucleotide 740 that identifies peptide 732. The MHC molecule may be coupled to a microcapsule and/or support 733. Microcapsule and/or support 733 may be a polypeptide, such as streptavidin, or a polysaccharide, such as dextran. Reporter oligonucleotide 740 may be directly or indirectly coupled to MHC labelling agent 730 in any suitable manner. For example, reporter oligonucleotide 740 may be coupled to MHC molecule 731, microcapsule and/or support 733, or peptide 732. Labelling agent 730 may comprise a plurality of MHC molecules, (e.g. is an MHC multimer, which may be coupled to a microcapsule and/or support (e.g., 733)). There are many possible configurations of Class I and/or Class II MHC multimers that can be utilized with the compositions, methods, and systems disclosed herein, e.g., MHC tetramers, MHC pentamers (MHC assembled via a coiled-coil domain, e.g., Pro5® MHC Class I Pentamers, (ProImmune, Ltd.), MHC octamers, MHC dodecamers, MHC decorated dextran molecules (e.g., MHC Dextramer® (Immudex)), etc. For a description of exemplary labelling agents, including antibody and MHC-based labelling agents, reporter oligonucleotides, and methods of use, see, e.g., U.S. Pat. 10,550,429 and U.S. Pat. Pub. 20190367969.

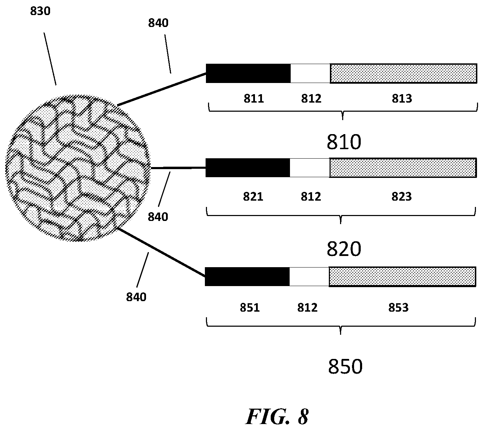

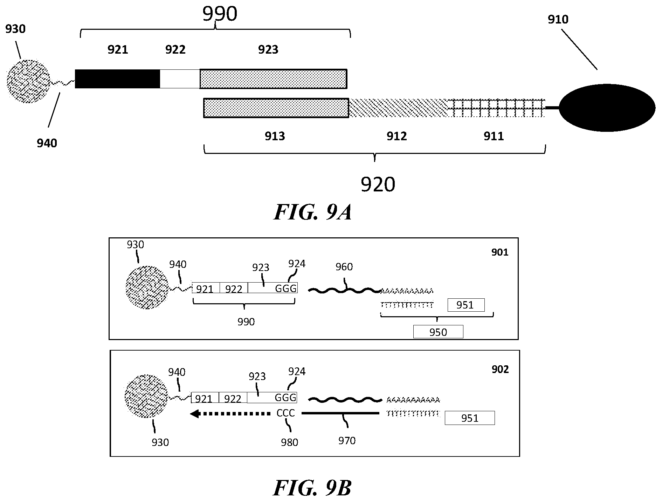

**FIG. 8** illustrates another example of a barcode carrying bead. Analysis of multiple analytes (e.g., RNA and one or more analytes using labelling agents described herein) may comprise nucleic acid barcode molecules as generally depicted in **FIG. 8**. Nucleic acid barcode molecules **810** and **820** may be attached to microcapsule and/or support **830** via a releasable linkage **840** (e.g., comprising a labile bond) as described elsewhere herein. Nucleic acid barcode molecule **810** may comprise adapter sequence **811,** barcode sequence **812** and adapter sequence **813**. Nucleic acid barcode molecule **820** may comprise adapter sequence **821,** barcode sequence **812,** and adapter sequence **823,** wherein adapter sequence **823** comprises a different sequence than adapter sequence **813**. Adapter **811** and adapter **821** may comprise the same sequence. Adapter **811** and adapter **821** may comprise different sequences. Although microcapsule and/or support **830** is shown comprising nucleic acid barcode molecules **810** and **820,** any suitable number of barcode molecules comprising common barcode sequence **812** are contemplated herein. For example, microcapsule and/or support **830** may further comprise nucleic acid barcode molecule **850**. Nucleic acid barcode molecule **850** may comprise adapter sequence **851,** barcode sequence **812** and adapter sequence **853,** wherein adapter sequence **853** comprises a different sequence than adapter sequence **813** and **823**. Nucleic acid barcode molecules (e.g., **810, 820, 850) may** comprise one or more additional functional sequences, such as a UMI or other sequences described herein. The nucleic acid barcode molecules **810, 820** or **850** may interact with analytes as described elsewhere herein, for example, as depicted in **FIGs. 9A-C.**

Referring to **FIG. 9A,** where cells are labelled with labeling agents, sequence **923** may be complementary to an adapter sequence of a reporter oligonucleotide. Cells may be contacted with one or more reporter oligonucleotide **920** conjugated labelling agents **910** (e.g., polypeptide, antibody, or others described elsewhere herein). In some cases, the cells may be further processed prior to barcoding. For example, such processing steps may include one or more washing and/or cell sorting steps. A cell that is bound to labelling agent **910** which is conjugated to oligonucleotide **920** and microcapsule and/or support **930** (e.g., a bead, such as a gel bead) comprising nucleic acid barcode molecule **990** may be partitioned into a partition amongst a plurality of partitions (e.g., a droplet of a droplet emulsion or a well of a microwell array). The partition may comprise at most a single cell bound to labelling agent **910**. Reporter oligonucleotide **920** conjugated to labelling agent **910** (e.g., polypeptide, an antibody, pMHC molecule such as an MHC multimer, etc.) may comprise a first adapter sequence **911** (e.g., a primer sequence), a barcode sequence **912** that identifies the labelling agent **910** (e.g., the polypeptide, antibody, or peptide of a pMHC molecule or complex), and an adapter sequence **913**. Adapter sequence **913** may be configured to hybridize to a complementary sequence, such as sequence **923** present on a nucleic acid barcode molecule **990**. Oligonucleotide **920** may comprise one or more additional functional sequences, such as those described elsewhere herein.

Barcoded nucleic may be generated (e.g., via a nucleic acid reaction, such as nucleic acid extension or ligation) from the constructs described in **FIGs. 9A-C.** For example, sequence **913** may then be hybridized to complementary sequence **923** to generate (e.g., via a nucleic acid reaction, such as nucleic acid extension or ligation) a barcoded nucleic acid molecule comprising cell (e.g., partition specific) barcode sequence **922** (or a reverse complement thereof) and reporter barcode sequence **912** (or a reverse complement thereof). Barcoded nucleic acid molecules can then be optionally processed as described elsewhere herein, e.g., to amplify the molecules and/or append sequencing platform specific sequences to the fragments. See, e.g., U.S. Pat. Pub. 2018/0105808. Barcoded nucleic acid molecules, or derivatives generated therefrom, can then be sequenced on a suitable sequencing platform.

Analysis of multiple analytes (e.g., nucleic acids and one or more analytes using labelling agents described herein) may be performed. For example, the workflow may comprise a workflow as generally depicted in any of **FIGs. 9A-C,** or a combination of workflows for an individual analyte, as described elsewhere herein. For example, by using a combination of the workflows as generally depicted in **FIGs. 9A-C,** multiple analytes can be analyzed.

Analysis of an analyte (e.g. a nucleic acid, a polypeptides, a carbohydrate, a lipid, etc.) may comprise a workflow as generally depicted in **FIG. 9A**. A nucleic acid barcode molecule **990** may be co-partitioned with the one or more analytes. Nucleic acid barcode molecule **990** may be attached to a microcapsule and/or support **930** (e.g., a bead, such as a gel bead), such as those described elsewhere herein. For example, nucleic acid barcode molecule **990** may be attached to a microcapsule and/or support **930** via a releasable linkage **940** (e.g., comprising a labile bond), such as those described elsewhere herein. Nucleic acid barcode molecule **990** may comprise a barcode sequence **921** and optionally comprise other additional sequences, for example, a UMI sequence **922** (or other functional sequences described elsewhere herein). The nucleic acid barcode molecule **990** may comprise a sequence **923** that may be complementary to another nucleic acid sequence, such that it may hybridize to a particular sequence.

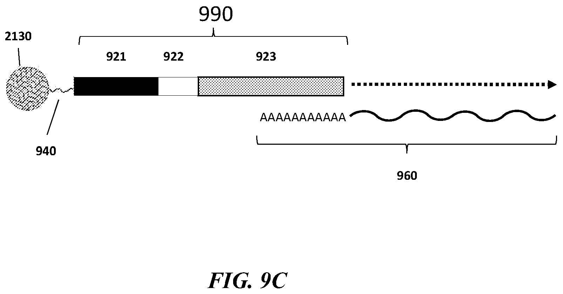

For example, sequence **923** may comprise a poly-T sequence and may be used to hybridize to mRNA. Referring to **FIG. 9C,** nucleic acid barcode molecule **990** may comprise sequence **923** complementary to a sequence of RNA molecule **960** from a cell. Sequence **923** may comprise a sequence specific for an RNA molecule. Sequence **923** may comprise a known or targeted sequence or a random sequence. A nucleic acid extension reaction may be performed, thereby generating a barcoded nucleic acid product comprising sequence **923,** the barcode sequence **921,** UMI sequence **922,** any other functional sequence, and a sequence corresponding to the RNA molecule **960**.

In another example, sequence **923** may be complementary to an overhang sequence or an adapter sequence that has been appended to an analyte. For example, referring to **FIG. 9B,** panel **901,** primer **950** may comprise a sequence complementary to a sequence of nucleic acid molecule **960** (such as an RNA encoding for a BCR sequence) from an analyte carrier. Primer **950** may comprise one or more sequences **951** that are not complementary to RNA molecule **960.** Sequence **951** may be a functional sequence as described elsewhere herein, for example, an adapter sequence, a sequencing primer sequence, or a sequence the facilitates coupling to a flow cell of a sequencer. Primer **950** may comprise a poly-T sequence. Primer **950** may comprise a sequence complementary to a target sequence in an RNA molecule. Primer **950** may comprise a sequence complementary to a region of an immune molecule, such as the constant region of a TCR or BCR sequence. Primer **950** is hybridized to nucleic acid molecule **960** and complementary molecule **970** is generated (*see* Panel **902**). For example, complementary molecule **970** may be cDNA generated in a reverse transcription reaction. An additional sequence may be appended to complementary molecule **970.** For example, the reverse transcriptase enzyme may be selected such that several non-templated bases **980** (e.g., a poly-C sequence) are appended to the cDNA. In another example, a terminal transferase may also be used to append the additional sequence. Nucleic acid barcode molecule **990** comprises a sequence **924** complementary to the non-templated bases, and the reverse transcriptase performs a template switching reaction onto nucleic acid barcode molecule **990** to generate a barcoded nucleic acid molecule comprising cell (e.g., partition specific) barcode sequence **922** (or a reverse complement thereof) and a sequence of complementary molecule **970** (or a portion thereof). Sequence **923** may comprise a sequence complementary to a region of an immune molecule, such as the constant region of a TCR or BCR sequence. Sequence **923** is hybridized to nucleic acid molecule **960** and a complementary molecule **970** is generated. For example, complementary molecule **970** may be generated in a reverse transcription reaction generating a barcoded nucleic acid molecule comprising cell (e.g., partition specific) barcode sequence **922** (or a reverse complement thereof) and a sequence of complementary molecule **970** (or a portion thereof). Additional methods and compositions suitable for barcoding cDNA generated from mRNA transcripts including those encoding V(D)J regions of an immune cell receptor and/or barcoding methods and composition including a template switch oligonucleotide are described in International Patent Application WO2018/075693A1, U.S. Patent Publication No. 2018/0105808, U.S. Patent Publication No. 2015/0376609, filed June 26, 2015, and U.S. Patent Publication No. 2019/0367969.

## Flow Sorting

A sample may derive from any useful source including any subject, such as a human subject. A sample may comprise material (e.g., one or more analyte carriers) from one or more different sources, such as one or more different subjects. Multiple samples, such as multiple samples from a single subject (e.g., multiple samples obtained in the same or different manners from the same or different bodily locations, and/or obtained at the same or different times (e.g., seconds, minutes, hours, days, weeks, months, or years)), or multiple samples from different subjects, may be obtained for analysis as described herein. For example, a first sample may be obtained from a subject at a first time and a second sample may be obtained from the subject at a second time later than the first time. The first time may be before a subject undergoes a treatment regimen or procedure (e.g., to address a disease or condition), and the second time may be during or after the subject undergoes the treatment regimen or procedure. In another example, a first sample may be obtained from a first bodily location or system of a subject (e.g., using a first collection technique) and a second sample may be obtained from a second bodily location or system of the subject (e.g., using a second collection technique), which second bodily location or system may be different than the first bodily location or system. In another example, multiple samples may be obtained from a subject at a same time from the same or different bodily locations. Different samples, such as different subjects collected from different bodily locations of a same subject, at different times, from multiple different subjects, and/or using different collection techniques, may undergo the same or different processing (e.g., as described herein). For example, a first sample may undergo a first processing protocol and a second sample may undergo a second processing protocol.

A sample may be a biological sample, such as a cell sample (e.g., as described herein). A sample may include one or more analyte carriers, such as one or more cells and/or cellular constituents, such as one or more cell nuclei. For example, a sample may comprise a plurality of analyte carriers, such as a plurality of cells and/or cellular constituents. Analyte carriers (e.g., cells or cellular constituents, such as cell nuclei) of a sample may be of a single type or a plurality of different types. For example, cells of a sample may include one or more different types or blood cells.

Cells and cellular constituents of a sample may be of any type. For example, a cell or cellular constituent may be a mammalian, fungal, plant, bacterial, or other cell type. In some cases, the cell is a mammalian cell, such as a human cell. The cell may be, for example, a stem cell, liver cell, nerve cell, bone cell, blood cell, reproductive cell, skin cell, skeletal muscle cell, cardiac muscle cell, smooth muscle cell, hair cell, hormone-secreting cell, or glandular cell. The cell may be, for example, an erythrocyte (e.g., red blood cell), a megakaryocyte (e.g., platelet precursor), a monocyte (e.g., white blood cell), a leukocyte, a B cell, a T cell (such as a helper, suppressor, cytotoxic, or natural killer T cell), an osteoclast, a dendritic cell, a connective tissue macrophage, an epidermal Langerhans cell, a microglial cell, a granulocyte, a hybridoma cell, a mast cell, a natural killer cell, a reticulocyte, a hematopoietic stem cell, a myoepithelial cell, a myeloid-derived suppressor cell, a platelet, a thymocyte, a satellite cell, an epithelial cell, an endothelial cell, an epididymal cell, a kidney cell, a liver cell, an adipocyte, a lipocyte, or a neuron cell. In some cases, the cell may be associated with a cancer, tumor, or neoplasm. In some cases, the cell may be associated with a fetus. In some cases, the cell may be a Jurkat cell.

A cell of a biological sample may have any feature or dimension. For example, a cell may have a first dimension, a second dimension, and a third dimension, where the first, second, and third dimensions are approximately the same. In other cases, the first and second dimensions may be approximately the same, and the third dimension may be different, or the first, second, and third dimensions may all be different. In some cases, a cell may comprise a dimension (e.g., a diameter) of at least about 1 $\mu$m. For example, a cell may comprise a dimension of at least about 1 micrometer ($\mu$m), 2 $\mu$m, 3 $\mu$m, 4 $\mu$m, 5 $\mu$m, 6 $\mu$m, 7 $\mu$m, 8 $\mu$m, 9 $\mu$m, 10 $\mu$m, 11 $\mu$m, 12 $\mu$m, 13 $\mu$m, 14 $\mu$m, 15 $\mu$m, 16 $\mu$m, 17 $\mu$m, 18 $\mu$m, 19 $\mu$m, 20 $\mu$m, 25 $\mu$m, 30 $\mu$m, 35 $\mu$m, 40 $\mu$m, 45 $\mu$m, 50 $\mu$m, 55 $\mu$m, 60 $\mu$m, 65 $\mu$m, 70 $\mu$m, 75 $\mu$m, 80 $\mu$m, 85 $\mu$m, 90 $\mu$m, 100 $\mu$m, 120 $\mu$m, 140 $\mu$m, 160 $\mu$m, 180 $\mu$m, 200 $\mu$m, 250 $\mu$m, 300 $\mu$m, 350 $\mu$m, 400 $\mu$m, 450 $\mu$m, 500 $\mu$m, 550 $\mu$m, 600 $\mu$m, 650 $\mu$m, 700 $\mu$m, 750 $\mu$m, 800 $\mu$m, 850 $\mu$m, 900 $\mu$m, 950 $\mu$m, 1 millimeter (mm), or greater. In some cases, the cell may comprise a dimension of between about 1 $\mu$m and 500 $\mu$m, such as between about 1 $\mu$m and 100 $\mu$m, between about 100 $\mu$m and 200 $\mu$m, between about 200 $\mu$m and 300 $\mu$m, between about 300 $\mu$m and 400 $\mu$m, or between about 400 $\mu$m and 500 $\mu$m. For example, a cell may comprise a dimension of between about 1 $\mu$m and 100 $\mu$m. Any or all dimensions of a cell may be variable. For example, the dimensions of a substantially fluid cell may vary over a rapid timescale. Dimensions of a more rigid cell may be fixed or may vary with lesser amplitude. Accordingly, the dimensions provided herein may represent averages rather than fixed values. The volume of a cell may be at least about 1 $\mu$m3. In some cases, the volume of a cell may be at least about 10 $\mu$m3. For example, the volume of the cell may be at least 1 $\mu$m3, 2 $\mu$m3, 3 $\mu$m3, 4 $\mu$m3, 5 $\mu$m3, 6 $\mu$m3, 7 $\mu$m3, 8 $\mu$m3, 9 $\mu$m3, 10 $\mu$m3, 12 $\mu$m3, 14 $\mu$m3, 16 $\mu$m3, 18 $\mu$m3, 20 $\mu$m3, 25 $\mu$m3, 30 $\mu$m3, 35 $\mu$m3, 40 $\mu$m3, 45 $\mu$m3, 50 $\mu$m3, 55 $\mu$m3, 60 $\mu$m3, 65 $\mu$m3, 70 $\mu$m3, 75 $\mu$m3, 80 $\mu$m3, 85 $\mu$m3, 90 $\mu$m3, 95 $\mu$m3, 100 $\mu$m3, 125 $\mu$m3, 150 $\mu$m3, 175 $\mu$m3, 200 $\mu$m3, 250 $\mu$m3, 300 $\mu$m3, 350 $\mu$m3, 400 $\mu$m3, 450 $\mu$m3, $\mu$m3, 500 $\mu$m3, 550 $\mu$m3, 600 $\mu$m3, 650 $\mu$m3, 700 $\mu$m3, 750 $\mu$m3, 800 $\mu$m3, 850 $\mu$m3, 900 $\mu$m3, 950 $\mu$m3, 1000 $\mu$m3, 1200 $\mu$m3, 1400 $\mu$m3, 1600 $\mu$m3, 1800 $\mu$m3, 2000 $\mu$m3, 2200 $\mu$m3, 2400 $\mu$m3, 2600 $\mu$m3, 2800 $\mu$m3, 3000 $\mu$m3, or greater. In some cases, a cell may comprise a volume of between about 1 $\mu$m3 and 100 $\mu$m3, such as between about 1 $\mu$m3 and 10 $\mu$m3, between about10 $\mu$m3 and 50 $\mu$m3, or between about 50 $\mu$m3 and 100 $\mu$m3. In some cases, a cell may comprise a volume of between about 100 $\mu$m3 and 1000 $\mu$m3, such as between about 100 $\mu$m3 and 500 $\mu$m3 or between about 500 $\mu$m3 and 1000 $\mu$m3. In some cases, a cell may comprise a volume between about 1000 $\mu$m3 and 3000 $\mu$m3, such as between about 1000 $\mu$m3 and 2000 $\mu$m3 or between about 2000 $\mu$m3 and 3000 $\mu$m3. In some cases, a cell may comprise a volume between about 1 $\mu$m3 and 3000 $\mu$m3, such as between about 1 $\mu$m3 and 2000 $\mu$m3, between about 1 $\mu$m3 and 1000 $\mu$m3, between about 1 $\mu$m3 and 500 $\mu$m3, or between about 1 $\mu$m3 and 250 $\mu$m3.

A cell of a biological sample may comprise one or more cross-sections that may be the same or different. In some cases, a cell may have a first cross-section that is different from a second cross-section. a cell may have a first cross-section that is at least about 1 $\mu$m. For example, a cell may comprise a cross-section (e.g., a first cross-section) of at least about 1 micrometer ($\mu$m), 2 $\mu$m, 3 $\mu$m, 4 $\mu$m, 5 $\mu$m, 6 $\mu$m, 7 $\mu$m, 8 $\mu$m, 9 $\mu$m, 10 $\mu$m, 11 $\mu$m, 12 $\mu$m, 13 $\mu$m, 14 $\mu$m, 15 $\mu$m, 16 $\mu$m, 17 $\mu$m, 18 $\mu$m, 19 $\mu$m, 20 $\mu$m, 25 $\mu$m, 30 $\mu$m, 35 $\mu$m, 40 $\mu$m, 45 $\mu$m, 50 $\mu$m, 55 $\mu$m, 60 $\mu$m, 65 $\mu$m, 70 $\mu$m, 75 $\mu$m, 80 $\mu$m, 85 $\mu$m, 90 $\mu$m, 100 $\mu$m, 120 $\mu$m, 140 $\mu$m, 160 $\mu$m, 180 $\mu$m, 200 $\mu$m, 250 $\mu$m, 300 $\mu$m, 350 $\mu$m, 400 $\mu$m, 450 $\mu$m, 500 $\mu$m, 550 $\mu$m, 600 $\mu$m, 650 $\mu$m, 700 $\mu$m, 750 $\mu$m, 800 $\mu$m, 850 $\mu$m, 900 $\mu$m, 950 $\mu$m, 1 millimeter (mm), or greater. In some cases, a cell may comprise a cross-section (e.g., a first cross-section) of between about 1 $\mu$m and 500 $\mu$m, such as between about 1 $\mu$m and 100 $\mu$m, between about 100 $\mu$m and 200 $\mu$m, between about 200 $\mu$m and 300 $\mu$m, between about 300 $\mu$m and 400 $\mu$m, or between about 400 $\mu$m and 500 $\mu$m. For example, a cell may comprise a cross-section (e.g., a first cross-section) of between about 1 $\mu$m and 100 $\mu$m. In some cases, the cell may have a second cross-section that is at least about 1 $\mu$m. For example, the cell may comprise a second cross-section of at least about 1 micrometer ($\mu$m), 2 $\mu$m, 3 $\mu$m, 4 $\mu$m, 5 $\mu$m, 6 $\mu$m, 7 $\mu$m, 8 $\mu$m, 9 $\mu$m, 10 $\mu$m, 11 $\mu$m, 12 $\mu$m, 13 $\mu$m, 14 $\mu$m, 15 $\mu$m, 16 $\mu$m, 17 $\mu$m, 18 $\mu$m, 19 $\mu$m, 20 $\mu$m, 25 $\mu$m, 30 $\mu$m, 35 $\mu$m, 40 $\mu$m, 45 $\mu$m, 50 $\mu$m, 55 $\mu$m, 60 $\mu$m, 65 $\mu$m, 70 $\mu$m, 75 $\mu$m, 80 $\mu$m, 85 $\mu$m, 90 $\mu$m, 100 $\mu$m, 120 $\mu$m, 140 $\mu$m, 160 $\mu$m, 180 $\mu$m, 200 $\mu$m, 250 $\mu$m, 300 $\mu$m, 350 $\mu$m, 400 $\mu$m, 450 $\mu$m, 500 $\mu$m, 550 $\mu$m, 600 $\mu$m, 650 $\mu$m, 700 $\mu$m, 750 $\mu$m, 800 $\mu$m, 850 $\mu$m, 900 $\mu$m, 950 $\mu$m, 1 millimeter (mm), or greater. In some cases, a cell may comprise a second cross-section of between about 1 $\mu$m and 500 $\mu$m, such as between about 1 $\mu$m and 100 $\mu$m, between about 100 $\mu$m and 200 $\mu$m, between about 200 $\mu$m and 300 $\mu$m, between about 300 $\mu$m and 400 $\mu$m, or between about 400 $\mu$m and 500 $\mu$m. For example, a cell may comprise a second cross-section of between about 1 $\mu$m and 100 $\mu$m.

A cross section (e.g., a first cross-section) may correspond to a diameter of a cell. In some cases, a cell may be approximately spherical. In such cases, the first cross-section may correspond to the diameter of the cell. In other cases, the cell may be approximately cylindrical. In such cases, the first cross-section may correspond to a diameter, length, or width along the approximately cylindrical cell. In some cases, the cell may comprise a surface. A cell surface may comprise one or more features. For example, a cell may comprise a dendritic receiver, flagella, roughed border, or other feature.

A characteristic or set of characteristics of a cell may be changed by one or more conditions. A condition suitable for changing a characteristic or set of characteristics of a cell may be, for example, a temperature, a pH, an ion or salt concentration, a pressure, or another condition. For example, a cell may be exposed to a chemical species that may bring about a change in one or more characteristics of the cell. In some cases, a stimulus may be used to change one or more characteristics of a cell. For example, upon application of the stimulus, one or more characteristics of a cell may be changed. The stimulus may be, for example, a thermal stimulus, a photo stimulus, a chemical stimulus, or another stimulus. In some cases, conditions sufficient to change the one or more characteristics of a cell may comprise one or more different conditions, such as a temperature and a pressure, a pH and a salt concentration, a chemical species and a temperature, or any other combination of conditions. A temperature sufficient for changing one or more characteristics of the cell may be, for example, at least about 0 degrees Celsius (°C), 1 °C, 2 °C, 3 °C, 4 °C, 5 °C, 10 °C, or higher. For example, the temperature may be about 4 °C. In other cases, a temperature sufficient for changing one or more characteristics of the cell may be, for example, at least about 25 °C, 30 °C, 35 °C, 37 °C, 40 °C, 45 °C, 50 °C, or higher. For example, the temperature may be about 37 °C. A pH sufficient for changing one or more characteristics of the cell may be, for example, between about 5 and 8, such as between about 6 and 7.

A biological sample may include a plurality of cells having different dimensions and features. In some cases, processing of the biological sample, such as cell separation and sorting (e.g., as described herein), may affect the distribution of dimensions and cellular features included in the sample by depleting cells having certain features and dimensions and/or isolating cells having certain features and dimensions.

A sample may undergo one or more processes in preparation for analysis (e.g., as described herein), including, but not limited to, filtration, selective precipitation, purification, centrifugation, permeabilization, isolation, agitation, heating, and/or other processes. For example, a sample may be filtered to remove a contaminant or other materials. In an example, a filtration process may comprise the use of microfluidics (e.g., to separate analyte carriers of different sizes, types, charges, or other features).

In an example, a sample comprising one or more cells may be processed to separate the one or more cells from other materials in the sample (e.g., using centrifugation and/or another process). In some cases, cells and/or cellular constituents of a sample may be processed to separate and/or sort groups of cells and/or cellular constituents, such as to separate and/or sort cells and/or cellular constituents of different types. Examples of cell separation include, but are not limited to, separation of white blood cells or immune cells from other blood cells and components, separation of circulating tumor cells from blood, and separation of bacteria from bodily cells and/or environmental materials. A separation process may comprise a positive selection process (e.g., targeting of a cell type of interest for retention for subsequent downstream analysis, such as by use of a monoclonal antibody that targets a surface marker of the cell type of interest), a negative selection process (e.g., removal of one or more cell types and retention of one or more other cell types of interest), and/or a depletion process (e.g., removal of a single cell type from a sample, such as removal of red blood cells from peripheral blood mononuclear cells).

Separation of one or more different types of cells may comprise, for example, centrifugation, filtration, microfluidic-based sorting, flow cytometry, fluorescence-activated cell sorting (FACS), magnetic-activated cell sorting (MACS), buoyancy-activated cell sorting (BACS), or any other useful method. For example, a flow cytometry method may be used to detect cells and/or cellular constituents based on a parameter such as a size, morphology, or protein expression. Flow cytometry-based cell sorting may comprise injecting a sample into a sheath fluid that conveys the cells and/or cellular constituents of the sample into a measurement region one at a time. In the measurement region, a light source such as a laser may interrogate the cells and/or cellular constituents and scattered light and/or fluorescence may be detected and converted into digital signals. A nozzle system (e.g., a vibrating nozzle system) may be used to generate droplets (e.g., aqueous droplets) comprising individual cells and/or cellular constituents. Droplets including cells and/or cellular constituents of interest (e.g., as determined via optical detection) may be labeled with an electric charge (e.g., using an electrical charging ring), which charge may be used to separate such droplets from droplets including other cells and/or cellular constituents. For example, FACS may comprise labeling cells and/or cellular constituents with fluorescent markers (e.g., using internal and/or external biomarkers). Cells and/or cellular constituents may then be measured and identified one by one and sorted based on the emitted fluorescence of the marker or absence thereof. MACS may use micro- or nano-scale magnetic particles to bind to cells and/or cellular constituents (e.g., via an antibody interaction with cell surface markers) to facilitate magnetic isolation of cells and/or cellular constituents of interest from other components of a sample (e.g., using a column-based analysis). BACS may use microbubbles (e.g., glass microbubbles) labeled with antibodies to target cells of interest. Cells and/or cellular components coupled to microbubbles may float to a surface of a solution, thereby separating target cells and/or cellular components from other components of a sample. Cell separation techniques may be used to enrich for populations of cells of interest (e.g., prior to partitioning, as described herein). For example, a sample comprising a plurality of cells including a plurality of cells of a given type may be subjected to a positive separation process. The plurality of cells of the given type may be labeled with a fluorescent marker (e.g., based on an expressed cell surface marker or another marker) and subjected to a FACS process to separate these cells from other cells of the plurality of cells. The selected cells may then be subjected to subsequent partition-based analysis (e.g., as described herein) or other downstream analysis. The fluorescent marker may be removed prior to such analysis or may be retained. The fluorescent marker may comprise an identifying feature, such as a nucleic acid barcode sequence and/or unique molecular identifier.

In another example, a first sample comprising a first plurality of cells including a first plurality of cells of a given type (e.g., immune cells expressing a particular marker or combination of markers) and a second sample comprising a second plurality of cells including a second plurality of cells of the given type may be subjected to a positive separation process. The first and second samples may be collected from the same or different subjects, at the same or different types, from the same or different bodily locations or systems, using the same or different collection techniques. For example, the first sample may be from a first subject and the second sample may be from a second subject different than the first subject. The first plurality of cells of the first sample may be provided a first plurality of fluorescent markers configured to label the first plurality of cells of the given type. The second plurality of cells of the second sample may be provided a second plurality of fluorescent markers configured to label the second plurality of cells of the given type. The first plurality of fluorescent markers may include a first identifying feature, such as a first barcode, while the second plurality of fluorescent markers may include a second identifying feature, such as a second barcode, that is different than the first identifying feature. The first plurality of fluorescent markers and the second plurality of fluorescent markers may fluoresce at the same intensities and over the same range of wavelengths upon excitation with a same excitation source (e.g., light source, such as a laser). The first and second samples may then be combined and subjected to a FACS process to separate cells of the given type from other cells based on the first plurality of fluorescent markers labeling the first plurality of cells of the given type and the second plurality of fluorescent markers labeling the second plurality of cells of the given type. Alternatively, the first and second samples may undergo separate FACS processes and the positively selected cells of the given type from the first sample and the positively selected cells of the given type from the second sample may then be combined for subsequent analysis. The encoded identifying features of the different fluorescent markers may be used to identify cells originating from the first sample and cells originating from the second sample. For example, the first and second identifying features may be configured to interact (e.g., in partitions, as described herein) with nucleic acid barcode molecules (e.g., as described herein) to generate barcoded nucleic acid products detectable using, e.g., nucleic acid sequencing.

## Reagents

Analyte carriers and/or biological particles may be partitioned along with lysis reagents in order to release the contents of the analyte carriers and/or biological particles within the partition. In such cases, the lysis agents can be contacted with the analyte carrier and/or biological particle suspension concurrently with, or immediately prior to, the introduction of the analyte carriers and/or biological particles into the partitioning junction/droplet generation zone (e.g., junction **210),** such as through an additional channel or channels upstream of the channel junction. Additionally or alternatively, analyte carriers and/or biological particles may be partitioned along with other reagents, as will be described further below.

The methods and systems of the present disclosure may comprise microfluidic devices and methods of use thereof, which may be used for co-partitioning analyte carriers and/or biological particles or analyte carriers and/or biological particles with reagents. Such systems and methods are described in U.S. Patent Publication No. US/20190367997.

Beneficially, when lysis reagents and analyte carriers and/or biological particles are co-partitioned, the lysis reagents can facilitate the release of the contents of the analyte carriers and/or biological particles within the partition. The contents released in a partition may remain discrete from the contents of other partitions.

As will be appreciated, the channel segments of the microfluidic devices described elsewhere herein may be coupled to any of a variety of different fluid sources or receiving components, including reservoirs, tubing, manifolds, or fluidic components of other systems. As will be appreciated, the microfluidic channel structures may have various geometries and/or configurations. For example, a microfluidic channel structure can have more than two channel junctions. For example, a microfluidic channel structure can have 2, 3, 4, 5 channel segments or more each carrying the same or different types of beads, reagents, analyte carriers and/or biological particles that meet at a channel junction. Fluid flow in each channel segment may be controlled to control the partitioning of the different elements into droplets. Fluid may be directed flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), as well as other commercially available lysis enzymes. Other lysis agents may additionally or alternatively be co-partitioned with the analyte carriers and/or biological particles to cause the release of the analyte carrier and/or biological particle's contents into the partitions. For example, in some cases, surfactant-based lysis solutions may be used to lyse cells, although these may be less desirable for emulsion based systems where the surfactants can interfere with stable emulsions. In some cases, lysis solutions may include non-ionic surfactants such as, for example, TritonX-100 and Tween 20. In some cases, lysis solutions may include ionic surfactants such as, for example, sarcosyl and sodium dodecyl sulfate (SDS). Electroporation, thermal, acoustic or mechanical cellular disruption may also be used in certain cases, e.g., non-emulsion based partitioning such as encapsulation of analyte carriers and/or biological particles that may be in addition to or in place of droplet partitioning, where any pore size of the encapsulate is sufficiently small to retain nucleic acid fragments of a given size, following cellular disruption.

Alternatively or in addition to the lysis agents co-partitioned with the analyte carriers and/or biological particles described above, other reagents can also be co-partitioned with the analyte carriers and/or biological particles, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated analyte carriers and/or biological particles (e.g., a cell or a nucleus in a polymer matrix), the analyte carriers and/or biological particles may be exposed to an appropriate stimulus to release the analyte carriers and/or biological particles or their contents from a co-partitioned microcapsule and/or support (e.g., bead). For example, in some cases, a chemical stimulus may be co-partitioned along with an encapsulated analyte carrier and/or biological particle to allow for the degradation of the microcapsule and/or support and release of the cell or its contents into the larger partition. In some cases, this stimulus may be the same as the stimulus described elsewhere herein for release of nucleic acid molecules (e.g., oligonucleotides) from their respective microcapsule and/or support (e.g., bead). In alternative examples, this may be a different and non-overlapping stimulus, in order to allow an encapsulated analyte carrier and/or biological particle to be released into a partition at a different time from the release of nucleic acid molecules into the same partition. For a description of methods, compositions, and systems for encapsulating cells (also referred to as a "cell bead"), see, e.g., U.S. Pat. 10,428,326 and U.S. Pat. Pub. 20190100632.

Additional reagents may also be co-partitioned with the analyte carriers and/or biological particles, such as endonucleases to fragment a analyte carrier and/or biological particle's DNA, DNA polymerase enzymes and dNTPs used to amplify the analyte carrier and/or biological particle's nucleic acid fragments and to attach the barcode molecular tags to the amplified fragments. Other enzymes may be co-partitioned, including without limitation, polymerase, transposase, ligase, proteinase K, DNAse, etc. Additional reagents may also include reverse transcriptase enzymes, including enzymes with terminal transferase activity, primers and oligonucleotides, and switch oligonucleotides (also referred to herein as "switch oligos" or "template switching oligonucleotides") which can be used for template switching. In some cases, template switching can be used to increase the length of a cDNA. In some cases, template switching can be used to append a predefined nucleic acid sequence to the cDNA. In an example of template switching, cDNA can be generated from reverse transcription of a template, e.g., cellular mRNA, where a reverse transcriptase with terminal transferase activity can add additional nucleotides, e.g., polyC, to the cDNA in a template independent manner. Switch oligos can include sequences complementary to the additional nucleotides, e.g., polyG. The additional nucleotides (e.g., polyC) on the cDNA can hybridize to the additional nucleotides (e.g., polyG) on the switch oligo, whereby the switch oligo can be used by the reverse transcriptase as template to further extend the cDNA. Template switching oligonucleotides may comprise a hybridization region and a template region. The hybridization region can comprise any sequence capable of hybridizing to the target. In some cases, as previously described, the hybridization region comprises a series of G bases to complement the overhanging C bases at the 3' end of a cDNA molecule. The series of G bases may comprise 1 G base, 2 G bases, 3 G bases, 4 G bases, 5 G bases or more than 5 G bases. The template sequence can comprise any sequence to be incorporated into the cDNA. In some cases, the template region comprises at least 1 (e.g., at least 2, 3, 4, 5 or more) tag sequences and/or functional sequences. Switch oligos may comprise deoxyribonucleic acids; ribonucleic acids; modified nucleic acids including 2-Aminopurine, 2,6-Diaminopurine (2-Amino-dA), inverted dT, 5-Methyl dC, 2'-deoxyInosine, Super T (5-hydroxybutynl-2'-deoxyuridine), Super G (8-aza-7-deazaguanosine), locked nucleic acids (LNAs), unlocked nucleic acids (UNAs, e.g., UNA-A, UNA-U, UNA-C, UNA-G), Iso-dG, Iso-dC, 2' Fluoro bases (e.g., Fluoro C, Fluoro U, Fluoro A, and Fluoro G), or any combination.

In some cases, the length of a switch oligo may be at least about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 , 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides or longer.

In some cases, the length of a switch oligo may be at most about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 , 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides.

Once the contents of the cells are released into their respective partitions, the macromolecular components (e.g., macromolecular constituents of biological particles, such as RNA, DNA, or proteins) contained therein may be further processed within the partitions. In accordance with the methods and systems described herein, the macromolecular component contents of individual analyte carriers and/or biological particles can be provided with unique identifiers such that, upon characterization of those macromolecular components they may be attributed as having been derived from the same analyte carrier(s) and/or biological particle or particles. The ability to attribute characteristics to individual analyte carriers and/or biological particles or groups of analyte carriers and/or biological particles is provided by the assignment of unique identifiers specifically to an individual analyte carrier and/or biological particle or groups of analyte carriers and/or biological particles. Unique identifiers, e.g., in the form of nucleic acid barcodes can be assigned or associated with individual analyte carriers and/or biological particles or populations of analyte carriers and/or biological particles, in order to tag or label the analyte carrier and/or biological particle's macromolecular components (and as a result, its characteristics) with the unique identifiers. These unique identifiers can then be used to attribute the analyte carrier and/or biological particle's components and characteristics to an individual analyte carrier and/or biological particle or group of analyte carriers and/or biological particles.

This can be performed by co-partitioning the individual analyte carrier and/or biological particle or groups of analyte carriers and/or biological particles with the unique identifiers, such as described above (with reference to **FIG. 2).** The unique identifiers can be provided in the form of nucleic acid molecules (e.g., oligonucleotides) that comprise nucleic acid barcode sequences that may be attached to or otherwise associated with the nucleic acid contents of individual analyte carrier and/or biological particle, or to other components of the analyte carrier and/or biological particle, and particularly to fragments of those nucleic acids. The nucleic acid molecules are partitioned such that as between nucleic acid molecules in a given partition, the nucleic acid barcode sequences contained therein are the same, but as between different partitions, the nucleic acid molecule can, and do have differing barcode sequences, or at least represent a large number of different barcode sequences across all of the partitions in a given analysis. Only one nucleic acid barcode sequence may be associated with a given partition, although in some cases, two or more different barcode sequences may be present.

The nucleic acid barcode sequences can include from about 6 to about 20 or more nucleotides within the sequence of the nucleic acid molecules (e.g., oligonucleotides). The nucleic acid barcode sequences can include from about 6 to about 20, 30, 40, 50, 60, 70, 80, 90, 100 or more nucleotides. In some cases, the length of a barcode sequence may be about 6, 7, 8, 9, 10, 11, 12, 13, 14, 15, 16, 17, 18, 19, 20 nucleotides or longer. In some cases, the length of a barcode sequence may be at least about 6, 7, 8, 9, 10, 11, 12, 13, 14, 15, 16, 17, 18, 19, 20 nucleotides or longer. In some cases, the length of a barcode sequence may be at most about 6, 7, 8, 9, 10, 11, 12, 13, 14, 15, 16, 17, 18, 19, 20 nucleotides or shorter. These nucleotides may be completely contiguous, i.e., in a single stretch of adjacent nucleotides, or they may be separated into two or more separate subsequences that are separated by 1 or more nucleotides. In some cases, separated barcode subsequences can be from about 4 to about 16 nucleotides in length. In some cases, the barcode subsequence may be about 4, 5, 6, 7, 8, 9, 10, 11, 12, 13, 14, 15, 16 nucleotides or longer. In some cases, the barcode subsequence may be at least about 4, 5, 6, 7, 8, 9, 10, 11, 12, 13, 14, 15, 16 nucleotides or longer. In some cases, the barcode subsequence may be at most about 4, 5, 6, 7, 8, 9, 10, 11, 12, 13, 14, 15, 16 nucleotides or shorter.

The co-partitioned nucleic acid molecules can also comprise other functional sequences useful in the processing of the nucleic acids from the co-partitioned analyte carrier and/or biological particles. These sequences include, e.g., targeted or random/universal amplification primer sequences for amplifying nucleic acids (e.g., mRNA, the genomic DNA) from the individual analyte carriers and/or biological particles within the partitions while attaching the associated barcode sequences, sequencing primers or primer recognition sites, hybridization or probing sequences, e.g., for identification of presence of the sequences or for pulling down barcoded nucleic acids, or any of a number of other potential functional sequences. Other mechanisms of co-partitioning oligonucleotides may also be employed, including, e.g., coalescence of two or more droplets, where one droplet contains oligonucleotides, or microdispensing of oligonucleotides (e.g., attached to a bead) into partitions, e.g., droplets within microfluidic systems.

In an example, microcapsules and/or supports, such as beads, are provided that each include large numbers of the above described barcoded nucleic acid molecules (e.g., barcoded oligonucleotides) releasably attached to the beads, where all of the nucleic acid molecules attached to a particular bead will include the same nucleic acid barcode sequence, but where a large number of diverse barcode sequences are represented across the population of beads used. Hydrogel beads, e.g., comprising polyacrylamide polymer matrices, may be used as a solid microcapsule and/or support and delivery vehicle for the nucleic acid molecules into the partitions, as they are capable of carrying large numbers of nucleic acid molecules, and may be configured to release those nucleic acid molecules upon exposure to a particular stimulus, as described elsewhere herein. In some cases, the population of beads provides a diverse barcode sequence library that includes at least about 1,000 different barcode sequences, at least about 5,000 different barcode sequences, at least about 10,000 different barcode sequences, at least about 50,000 different barcode sequences, at least about 100,000 different barcode sequences, at least about 1,000,000 different barcode sequences, at least about 5,000,000 different barcode sequences, or at least about 10,000,000 different barcode sequences, or more. Additionally, each bead can be provided with large numbers of nucleic acid (e.g., oligonucleotide) molecules attached. In particular, the number of molecules of nucleic acid molecules including the barcode sequence on an individual bead can be at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules, or more. Nucleic acid molecules of a given bead can include identical (or common) barcode sequences, different barcode sequences, or a combination of both. Nucleic acid molecules of a given bead can include multiple sets of nucleic acid molecules. Nucleic acid molecules of a given set can include identical barcode sequences. The identical barcode sequences can be different from barcode sequences of nucleic acid molecules of another set.

Moreover, when the population of beads is partitioned, the resulting population of partitions can also include a diverse barcode library that includes at least about 1,000 different barcode sequences, at least about 5,000 different barcode sequences, at least about 10,000 different barcode sequences, at least at least about 50,000 different barcode sequences, at least about 100,000 different barcode sequences, at least about 1,000,000 different barcode sequences, at least about 5,000,000 different barcode sequences, or at least about 10,000,000 different barcode sequences. Additionally, each partition of the population can include at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules.

In some cases, it may be desirable to incorporate multiple different barcodes within a given partition, either attached to a single or multiple beads within the partition. For example, in some cases, a mixed, but known set of barcode sequences may provide greater assurance of identification in the subsequent processing, e.g., by providing a stronger address or attribution of the barcodes to a given partition, as a duplicate or independent confirmation of the output from a given partition.

The nucleic acid molecules (e.g., oligonucleotides) are releasable from the beads upon the application of a particular stimulus to the beads. In some cases, the stimulus may be a photo-stimulus, e.g., through cleavage of a photo-labile linkage that releases the nucleic acid molecules. In other cases, a thermal stimulus may be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of analyte carriers and/or biological particles, and may be degraded for release of the attached nucleic acid molecules through exposure to a reducing agent, such as DTT.

Also provided are systems and methods for controlled partitioning. Droplet size may be controlled by adjusting certain geometric features in channel architecture (e.g., microfluidics channel architecture). For example, an expansion angle, width, and/or length of a channel may be adjusted to control droplet size.

FIG. 2 shows an example of a microfluidic channel structure for the controlled partitioning of beads into discrete droplets. A channel structure 200 can include a channel segment 202 communicating at a channel junction 206 (or intersection) with a reservoir 204. The reservoir 204 can be a chamber. Any reference to "reservoir," as used herein, can also refer to a "chamber." In operation, an aqueous fluid 208 that includes suspended beads 212 may be transported along the channel segment 202 into the junction 206 to meet a second fluid 210 that is immiscible with the aqueous fluid 208 in the reservoir 204 to create droplets 216, 218 of the aqueous fluid 208 flowing into the reservoir 204. At the junction 206 where the aqueous fluid 208 and the second fluid 210 meet, droplets can form based on factors such as the hydrodynamic forces at the junction 206, flow rates of the two fluids 208, 210, fluid properties, and certain geometric parameters (e.g., $w$, $h_0$, $\alpha$, etc.) of the channel structure 200. A plurality of droplets can be collected in the reservoir 204 by continuously injecting the aqueous fluid 208 from the channel segment 202 through the junction 206.

A discrete droplet generated may include a bead (e.g., as in occupied droplets 216). Alternatively, a discrete droplet generated may include more than one bead. Alternatively, a discrete droplet generated may not include any beads (e.g., as in unoccupied droplet 218). A discrete droplet generated may contain one or more analyte carriers and/or biological particles, as described elsewhere herein. A discrete droplet generated may comprise one or more reagents, as described elsewhere herein.

The aqueous fluid **208** may have a substantially uniform concentration or frequency of beads **212.** The beads **212** can be introduced into the channel segment **202** from a separate channel (not shown in **FIG. 2).** The frequency of beads **212** in the channel segment **202** may be controlled by controlling the frequency in which the beads **212** are introduced into the channel segment **202** and/or the relative flow rates of the fluids in the channel segment **202** and the separate channel. The beads may be introduced into the channel segment **202** from a plurality of different channels, and the frequency controlled accordingly.

The aqueous fluid **208** in the channel segment **202** may comprise analyte carriers and/or biological particles. The aqueous fluid **208** may have a substantially uniform concentration or frequency of analyte carriers and/or biological particles. As with the beads, the analyte carriers and/or biological particles can be introduced into the channel segment **202** from a separate channel. The frequency or concentration of the analyte carriers and/or biological particles in the aqueous fluid **208** in the channel segment **202** may be controlled by controlling the frequency in which the analyte carriers and/or biological particles are introduced into the channel segment **202** and/or the relative flow rates of the fluids in the channel segment **202** and the separate channel. The analyte carriers and/or biological particles may be introduced into the channel segment **202** from a plurality of different channels, and the frequency controlled accordingly. A first separate channel may introduce beads and a second separate channel can introduce analyte carriers and/or biological particles into the channel segment **202.** The first separate channel introducing the beads may be upstream or downstream of the second separate channel introducing the analyte carriers and/or biological particles.

The second fluid **210** can comprise an oil, such as a fluorinated oil, that includes a fluorosurfactant for stabilizing the resulting droplets, for example, inhibiting subsequent coalescence of the resulting droplets.

The second fluid **210** may not be subjected to and/or directed to any flow in or out of the reservoir **204.** For example, the second fluid **210** may be substantially stationary in the reservoir **204.** The second fluid **210** may be subjected to flow within the reservoir **204,** but not in or out of the reservoir **204,** such as via application of pressure to the reservoir **204** and/or as affected by the incoming flow of the aqueous fluid **208** at the junction **206.** Alternatively, the second fluid **210** may be subjected and/or directed to flow in or out of the reservoir **204.** For example, the reservoir **204** can be a channel directing the second fluid **210** from upstream to downstream, transporting the generated droplets.

The channel structure **200** at or near the junction **206** may have certain geometric features that at least partly determine the sizes of the droplets formed by the channel structure **200.** The channel segment **202** can have a height, $h_0$ and width, w, at or near the junction **206.** By way of example, the channel segment **202** can comprise a rectangular cross-section that leads to a reservoir **204** having a wider cross-section (such as in width or diameter). Alternatively, the cross-section of the channel segment **202** can be other shapes, such as a circular shape, trapezoidal shape, polygonal shape, or any other shapes. The top and bottom walls of the reservoir **204** at or near the junction **206** can be inclined at an expansion angle, $\alpha$. The expansion angle, $\alpha$, allows the tongue (portion of the aqueous fluid **208** leaving channel segment **202** at junction **206** and entering the reservoir **204** before droplet formation) to increase in depth and facilitate decrease in curvature of the intermediately formed droplet. Droplet size may decrease with increasing expansion angle. The resulting droplet radius, $R_d$, may be predicted by the following equation for the aforementioned geometric parameters of $h_0$, w, and $\alpha$:

$$ R_d \approx 0.44 \left( 1 + 2.2\sqrt{\tan\alpha}\,\frac{w}{h_0} \right) \frac{h_0}{\sqrt{\tan\alpha}} $$

By way of example, for a channel structure with w = 21 μm, h = 21 μm, and $\alpha$ = 3°, the predicted droplet size is 121 μm. In another example, for a channel structure with w = 25 μm, h = 25 μm, and $\alpha$ = 5°, the predicted droplet size is 123 μm. In another example, for a channel structure with w = 28 μm, h = 28 μm, and $\alpha$ = 7°, the predicted droplet size is 124 μm.

The expansion angle, $\alpha$, may be between a range of from about 0.5° to about 4°, from about 0.1° to about 10°, or from about 0° to about 90°. For example, the expansion angle can be at least about 0.01°, 0.1°, 0.2°, 0.3°, 0.4°, 0.5°, 0.6°, 0.7°, 0.8°, 0.9°, 1°, 2°, 3°, 4°, 5°, 6°, 7°, 8°, 9°, 10°, 15°, 20°, 25°, 30°, 35°, 40°, 45°, 50°, 55°, 60°, 65°, 70°, 75°, 80°, 85°, or higher. The expansion angle can be at most about 89°, 88°, 87°, 86°, 85°, 84°, 83°, 82°, 81°, 80°, 75°, 70°, 65°, 60°, 55°, 50°, 45°, 40°, 35°, 30°, 25°, 20°, 15°, 10°, 9°, 8°, 7°, 6°, 5°, 4°, 3°, 2°, 1°, 0.1°, 0.01°, or less. The width, w, may be between a range of from about 100 micrometers (μm) to about 500 μm. The width, w, may be between a range of from about 10 μm to about 200 μm. Alternatively, the width can be less than about 10 μm. Alternatively, the width can be greater than about 500 μm. The flow rate of the aqueous fluid **208** entering the junction **206** may be between about 0.04 microliters (μL)/minute (min) and about 40 μL/min. The flow rate of the aqueous fluid **208** entering the junction **206** may be between about 0.01 microliters (μL)/minute (min) and about 100 μL/min. Alternatively, the flow rate of the aqueous fluid **208** entering the junction **206** can be less than about 0.01 μL/min. Alternatively, the flow rate of the aqueous fluid **208** entering the junction **206** can be greater than about 40 μL/min, such as 45 μL/min, 50 μL/min, 55 μL/min, 60 μL/min, 65 μL/min, 70 μL/min, 75 μL/min, 80 μL/min, 85 μL/min, 90 μL/min, 95 μL/min, 100 μL/min, 110 μL/min, 120 μL/min, 130 μL/min, 140 μL/min, 150

µL/min, or greater. At lower flow rates, such as flow rates of about less than or equal to 10 microliters/minute, the droplet radius may not be dependent on the flow rate of the aqueous fluid **208** entering the junction **206.**

At least about 50% of the droplets generated may have uniform size. At least about 55%, 60%, 65%, 70%, 75%, 80%, 85%, 90%, 95%, 96%, 97%, 98%, 99%, or greater of the droplets generated may have uniform size. Alternatively, less than about 50% of the droplets generated can have uniform size.

The throughput of droplet generation can be increased by increasing the points of generation, such as increasing the number of junctions (e.g., junction **206**) between aqueous fluid **208** channel segments (e.g., channel segment **202**) and the reservoir **204.** Alternatively, or in addition, the throughput of droplet generation can be increased by increasing the flow rate of the aqueous fluid **208** in the channel segment **202.**

The methods and systems described herein may be used to greatly increase the efficiency of single cell applications and/or other applications receiving droplet-based input. For example, following the sorting of occupied cells and/or appropriately-sized cells, subsequent operations that can be performed can include generation of amplification products, purification (e.g., via solid phase reversible immobilization (SPRI)), further processing (e.g., shearing, ligation of functional sequences, and subsequent amplification (e.g., via PCR)). These operations may occur in bulk (e.g., outside the partition). In the case where a partition is a droplet in an emulsion, the emulsion can be broken and the contents of the droplet pooled for additional operations. Additional reagents that may be co-partitioned along with the barcode bearing bead may include oligonucleotides to block ribosomal RNA (rRNA) and nucleases to digest genomic DNA from cells. Alternatively, rRNA removal agents may be applied during additional processing operations. The configuration of the constructs generated by such a method can help minimize (or avoid) sequencing of the poly-T sequence during sequencing and/or sequence the 5' end of a polynucleotide sequence. The amplification products, for example, first amplification products and/or second amplification products, may be subject to sequencing for sequence analysis. In some cases, amplification may be performed using the Partial Hairpin Amplification for Sequencing (PHASE) method.

A variety of applications require the evaluation of the presence and quantification of different analyte carrier and/or biological particle or organism types within a population of analyte carriers and/or biological particles, including, for example, microbiome analysis and characterization, environmental testing, food safety testing, epidemiological analysis, e.g., in tracing contamination or the like.

### High Throughput Analysis of Samples

The systems and methods described herein allow for partition-based high throughput analysis of analyte carriers and/or biological particles, e.g., cells or nuclei, in a single experiment. The high throughput analysis of analyte carriers and/or biological particles may be performed in a plurality of droplets in an emulsion. A plurality of samples which comprise a plurality of analyte carriers and/or biological particles, e.g., cells or nuclei, may be processed in droplets on a large scale, e.g., hundreds of thousands of analyte carriers and/or biological particles, in a single experiment. For example, the microfluidic chip depicted in **FIG. 16** allows a user to load thousands of analyte carriers and/or biological particles, e.g., cells or nuclei, into each of the 16 Sample wells shown, i.e., 2A Sample row and 2B Sample row, for processing. Each of the 16 wells may be loaded with between about 2,000 to about 20,000 analyte carriers and/or biological particles to allow between about 32,000 and about 320,000 analyte carriers and/or biological particles per chip. Each of the 16 wells may be loaded with between about 30,000 and about 60,000 analyte carriers and/or biological particles to allow between about 480,000 and about 960,000 analyte carriers and/or biological particles per chip. Each of the 16 wells may be loaded with about 60,000 analyte carriers and/or biological particles to allow for up about 960,000 analyte carriers and/or biological particles per chip.

High throughput single cell experiments allow more effective analysis and characterization of complex tissues with heterogeneous cell populations. Such experiments allow a user to gain greater insight into molecular diversity and tissue composition, resolve complexities of disease phenotypes, and link subtle or rare cellular states to specific transcriptomic profiles. In addition, higher throughput scaling allows for the profiling of hundreds of thousands to millions of cells with multiomic outputs. The present disclosure provides high throughput single cell or single nucleus analysis methods that allow for scaled-up screening of cells or nuclei following perturbation, e.g., drug and/or gene editing, such as CRISPR-based editing. An increased cell or cell nucleus throughput allows for a greater number of genes to be perturbed enabling investigation of gene function across entire pathways in response to some stimulus, e.g., a drug. A multiplexed drug screen with perturbations across an entire key pathway could provide insight on a drug's mechanism of action and any key gene modules that mediate drug effects. Increased scale can also provide greater understanding of disease stratification across complex diseases. Greater sample numbers can prove useful in (i) developing new biomarkers and improved stratification of clinical cohorts, (ii) understanding why disease types are responsive vs. resistant, e.g., in oncological or neurodegenerative disease, which can help define more precise subpopulations. The present disclosure provides methods for multiplexing samples per input well of a microfluidic chip, e.g., **FIG. 16** which depicts 16 different sample input wells. A first sample of analyte carriers and/or biological particles, e.g., cells or nuclei, may be contacted with a first labeling agent, e.g., a labeling agent comprising or associated with a first reporter oligonucleotide, a second sample of analyte carriers and/or biological particles, e.g., cells or nuclei, may be contacted with a second labeling agent, e.g., a labeling agent comprising or associated with a second reporter oligonucleotide, etc. to provide multiple different labeled samples of analyte carriers and/or biological particles, e.g., cells or nuclei, (each comprising their respective labeling agent with a respective reporter oligonucleotide) that can be pooled for addition to an input well of a microfluidic chip, e.g., **FIG. 16.** The first sample of analyte carriers and/or biological particles, e.g., cells or nuclei, may be (or may be derived from) a plurality of analyte carriers and/or biological particles that have been perturbed by a first condition. The first condition may be a first treatment with a first drug and/or a first edited gene, such as by CRISPR-based gene editing including, without limitation, CRISPR KO, CRISPRi, CRISPRa, CRISPRoff, and CRISPRon editing. The first treatment may be a drug treatment for a first period of time or a drug treatment ending at a first time point, the second treatment may be a drug treatment for a second period of time or a drug treatment ending at a second time point, etc. to provide multiple different samples subjected to drug treatments for different periods of time or ending at different time point. The drug treatments may be with the same drug, with different drugs individually, or with different drugs in combination. The labeling agent(s) may be provided as part of a feature binding group, as further described herein. A labeling agent may be an agent coupled to a reporter oligonucleotide as further described herein. The labeling agent may be selected from a number of labeling agents including, without limitation, a lipophilic moiety (such as cholesterol), an antibody or antibody fragment, an epitope binding moiety, a protein, a peptide, a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof.

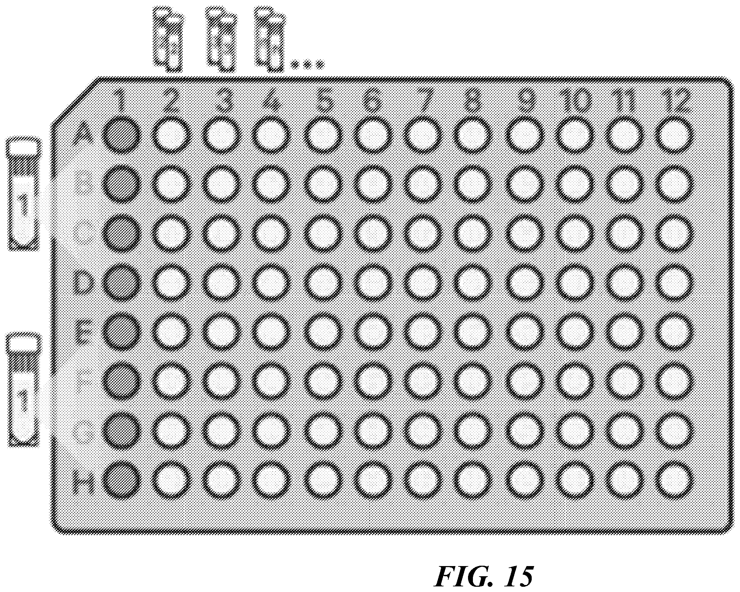

**FIG. 15** depicts a sample preparation approach based on a 96-well plate for biological particles, e.g., cells or nuclei. A sample of analyte carriers and/or biological particles can be added to each well in a row wherein each row represents a different experimental condition, e.g., drug treatment and/or incubation timeTable 1 shows the conditions for each well in row B from **FIG. 15** where Cell Line #1 is treated with drug A for 4, 16, or 24 hours in duplicate. In each well from row B, the Cell Line #1 sample is labeled with a different Oligo Label - Oligo Label #1 through Oligo Label #12. In another example, Table 2 shows the conditions for column 1 of the same plate depicted in **FIG. 15.** In this case, the incubation time of 4 hours and Cell line (#1) is the same for each well in column 1 (wells A1 to H1) but the drug being tested varies, e.g., A, B, C, D, and different combinations thereof.

Table 1

| Position | Drug | Incubation time | Cell Line | Oligo label |
|---|---|---|---|---|
| B1 | A | 4 hours | #1 | #1 |
| B2 | A | 4 hours | #1 | #2 |
| B3 | A | 16 hours | #1 | #3 |
| B4 | A | 16 hours | #1 | #4 |
| B5 | A | 24 hours | #1 | #5 |
| B6 | A | 24 hours | #1 | #6 |
| B7 | A | 4 hours | #2 | #7 |
| B8 | A | 4 hours | #2 | #8 |
| B9 | A | 16 hours | #2 | #9 |
| B10 | A | 16 hours | #2 | #10 |
| B11 | A | 24 hours | #2 | #11 |
| B12 | A | 24 hours | #2 | #12 |

Table 2

| Position | Drug | Incubation time | Cell line | Oligo label |
|---|---|---|---|---|
| A1 | None | 4 hours | #1 | #1 |
| B1 | A | 4 hours | #1 | #1 |
| C1 | B | 4 hours | #1 | #1 |
| D1 | A+B | 4 hours | #1 | #1 |
| E1 | C | 4 hours | #1 | #1 |

(continued)

| Position | Drug | Incubation time | Cell line | Oligo label |
|----------|------|-----------------|-----------|-------------|
| F1 | B+C | 4 hours | #1 | #1 |
| G1 | D | 4 hours | #1 | #1 |
| H1 | C+D | 4 hours | #1 | #1 |

Referring again to Table 1, the use of different labels for each well in row B allows each sample in wells B1 to B12 to be pooled into one suspension of labelled analyte carriers and/or biological particles, e.g., single cells or single nuclei, (from row B), which can then be processed in a single analyte carrier and/or biological particle, e.g., single cell/nucleus, barcoding workflow. **FIG. 16** depicts an example of a microfluidic chip for single cell/nucleus barcoding. The suspension of labelled single cells or nuclei, e.g., from wells B1 to B12, can be loaded into well #1 of the row labelled "2A Sample" in **FIG. 16.** The presence of a different oligonucleotide label with each sample from wells B1 to B12 allows for the partitioning (e.g., in a droplet in an emulsion) of a single analyte carrier and/or biological particle, e.g., a single cell or nucleus, with a plurality of nucleic acid barcode molecules that have a common barcode sequence unique to the partition. Within the partition, the oligonucleotide labels and analytes from the single cell/nucleus are coupled to the plurality of nucleic acid barcode molecules. Subsequently, barcoded nucleic acid molecules are generated using the oligonucleotide labels and analytes coupled to the plurality of nucleic acid barcode molecules. The generation step may occur in the partition, e.g., through a nucleic acid extension reaction such as reverse transcription. The further processing may occur outside of the partition. In the case of droplets in an emulsion, the oligonucleotide labels and analytes may be coupled to the plurality of nucleic acid barcode molecules without further processing within the droplet, e.g., without a nucleic acid extension reaction, and then the droplets are broken. The contents of the droplets are pooled for further processing, e.g., a nucleic acid extension reaction such as a reverse transcription reaction, which can be performed in bulk to generate barcoded nucleic acid molecules.

Such barcoded nucleic acid molecules (generated within the first partition or outside of the first partition) may comprise a first plurality of barcoded molecules that may be identified as originating from a first partition via a common barcode sequence (e.g., provided by a nucleic acid barcode molecule from a bead as further described herein). This first plurality of barcoded molecules from the first partition may comprise (a) a first barcoded nucleic acid molecule comprising the common barcode sequence unique to the first partition and a sequence corresponding to oligonucleotide label #1 (see row 2 of Table 1 - position B1), and (b) a second barcoded nucleic acid molecule comprising the common barcode sequence unique to the first partition and a sequence corresponding to an analyte from a single cell or single nucleus (e.g., an RNA analyte such as an mRNA). Upon sequencing of these barcoded nucleic acid molecules, it can be determined via the common barcode sequence that the first partition contained only a single analyte carrier and/or biological particle (e.g., a cell or nucleus) comprising oligonucleotide label, i.e., Oligo label #1, and any additional analyte carriers and/or biological particles comprising a different oligonucleotide label (e.g., Oligo label 2, 3, etc) were absent from the first partition. In such a case, the sequence data accurately reflects analytes from a single analyte carrier and/or biological particle, e.g., a single cell or nucleus, processed from the first partition.

However, the first plurality of barcoded molecules from the first partition may further comprise (c) a third barcoded nucleic acid molecule comprising the common barcode sequence unique to the first partition and a sequence corresponding to oligonucleotide label #2 (see row 3 of Table 1 - position B2) and (d) a fourth barcoded nucleic acid molecule comprising the common barcode sequence unique to the first partition and a sequence corresponding to an analyte from a single cell or single nucleus (e.g., an RNA analyte such as an mRNA). Upon sequencing of the barcoded nucleic acid molecules, it can be determined via the common barcode sequence that the first partition comprised a first analyte carrier and/or biological particle (e.g., a cell or nucleus) comprising Oligo label #1 and a second analyte carrier and/or biological particle (e.g., a cell or nucleus) comprising Oligo label #2. As a result, the barcoded molecules corresponding to analytes (e.g., the second and fourth barcoded nucleic acid molecules) cannot be attributed to a single analyte carrier and/or biological particle, e.g., a single cell or nucleus. The sequencing data for this set of barcoded molecules can be excluded computationally to improve the overall quality of the sequencing data for analytes from single analyte carriers and/or biological particles in the experiment.

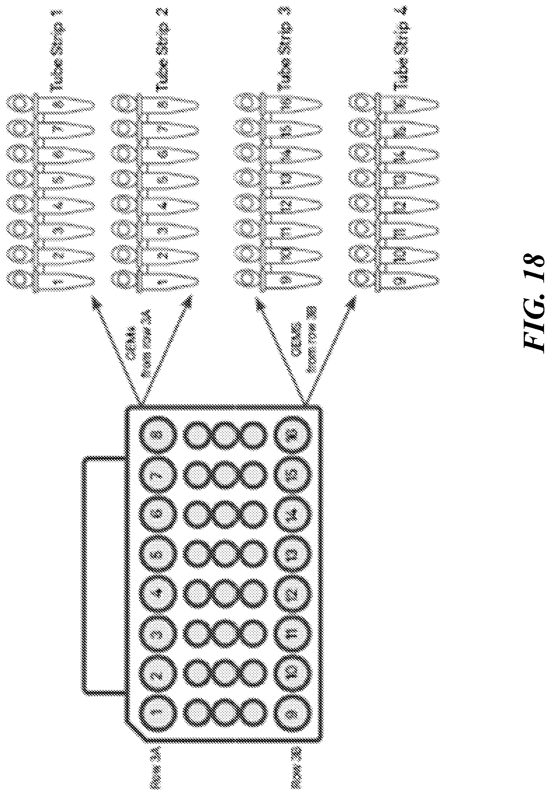

**FIG. 18** depicts retrieval of gel bead in emulsions (GEMs) comprising barcoded nucleic acid molecules. For a sample loaded in row 2A or 2B of the microfluidic chip depicted in **FIG. 16,** the GEMs are retrieved from the corresponding well in row 3A or 3B as shown in **FIG. 16 and 18.** Once retrieved, the GEMs may be transferred to a tube or a strip of tubes or strips of tubes.

The samples of analyte carriers and/or biological particles, e.g., cells or nuclei, may be derived from one common sample, e.g., a single donor, cell line, etc. or are derived from more than one sample, e.g., more than a single donor, cell line, etc. The methods for multiplexing samples per input well of a microfluidic chip, e.g., **FIG. 16,** may comprise labeling different samples with a labeling agent. For example, a first sample of cells or nuclei from a first sample (e.g., a first donor or first cell line) may be contacted with a first labeling agent, e.g., a labeling agent comprising or associated with a first reporter oligonucleotide, a second sample of cells or nuclei (e.g., a first donor or first cell line) may be contacted with a second labeling agent, e.g., a labeling agent comprising or associated with a second reporter oligonucleotide, etc. to provide multiple different labeled samples of cells or nuclei, e.g., different donors or different cell lines (each comprising their respective labeling agent with a respective reporter oligonucleotide) that can be pooled for addition to an input well of a microfluidic chip, e.g., **FIG. 16.**

**Drugs**

The present disclosure provides for drug discovery processes that allows for high throughput screening of compounds at different time points. The screening process can allowing for a deeper understanding of cell pathways that can be disrupted and/or affected by a treatment.

The one or more compounds may be screened for treatment of, for example, an infectious disease, a proliferative disease, a cancer, a solid tumor, or a liquid tumor. Treatment can comprise, for example a reduction in tumor size, a reduction in tumor volume, a decrease in the number of tumors, a decrease in the number of metastatic lesions, an increase in average survival of a subject, a decrease in mortality rate, a decrease in tumor growth rate, a decrease of tumor regrowth, a reduction in the rate of cellular proliferate, a reduction in the proportion of proliferating cells, a decrease in the size of a zone of cellular proliferation, or a decrease in the number or proportion of cells having an abnormal appearance or morphology.

The cancer may comprise acute lymphoblastic leukemia, acute myeloid leukemia, adrenocortical carcinoma, Kaposi sarcoma, AIDS-related lymphoma, primary CNS lymphoma, anal cancer, appendix cancer, childhood astrocytoma, central nervous system cancer, atypical teratoid/rhabdoid tumors, basal cell carcinoma, bile duct cancer, bladder cancer, bone cancer, Ewing sarcoma, osteosarcoma, malignant fibrous histiocytoma, brain tumors, brain cancer, breast cancer, bronchial tumors, lung cancer, Burkitt lymphoma, non-Hodgkin lymphoma, gastrointestinal cancer, carcinoid tumors, carcinoma, cardiac tumors, medulloblastoma tumors, germ cell tumors, cervical cancer, cholangiocarcinoma, chordoma, chronic lymphocytic leukemia, chronic myelogenous leukemia, chronic myeloproliferative neoplasms, colorectal cancer, craniopharyngioma, cutaneous T-cell lymphoma, ductal carcinoma, embryonal tumors, endometrial cancer, uterine cancer, ependymoma, esophageal cancer, esthesioneuroblastoma, head and neck cancer, extracranial germ cell tumors, extragonadal germ cell tumors, eye cancer, intraocular melanoma, retinoblastoma, fallopian tube cancer, gallbladder cancer, gastric (stomach) cancer, gastrointestinal carcinoid tumor, soft tissue sarcoma, gastrointestinal stromal tumors, ovarian germ cell tumors, testicular cancer, gestational trophoblastic disease, hairy cell leukemia, hepatocellular (liver) cancer, Langerhans cell histiocytosis, Hodgkin lymphoma, hypopharyngeal cancer, islet cell tumors, pancreatic neuroendocrine tumors, kidney (renal cell) cancer, lip and oral cavity cancer, lung cancer, non-small cell lung cancer, small cell lung cancer, pleuropulmonary blastoma, tracheobronchial tumors, leukemia, lymphoma, male breast cancer, melanoma, Merkel cell carcinoma, malignant mesothelioma, metastatic cancer, metastatic squamous neck cancer, midline tract carcinoma, mouth cancer, multiple endocrine neoplasia syndromes, multiple myeloma/plasma cell neoplasms, mycosis fungoides, myelodysplastic syndromes, myelodysplastic/myeloproliferative neoplasms, chronic myelogenous leukemia, nasal cavity and paranasal sinus cancer, nasopharyngeal cancer, neuroblastoma, oral cancer, oropharyngeal cancer, osteosarcoma, pancreatic cancer, pancreatic neuroendocrine tumors, islet cell tumors, papillomatosis, paraganglioma, parathyroid cancer, penile cancer, pharyngeal cancer, pheochromocytoma, pituitary tumors, plasma cell neoplasm, pregnancy and breast cancer, primary peritoneal cancer, prostate cancer, rectal cancer, retinoblastoma, rhabdomyosarcoma, salivary gland cancer, sarcoma, vascular tumors, uterine sarcoma, Sezary syndrome, skin cancer, small intestine cancer, stomach (gastric) cancer, T-cell lymphoma, testicular cancer, throat cancer, nasopharyngeal cancer, oropharyngeal cancer, hypopharyngeal cancer, thymoma and thymic carcinoma, thyroid cancer, tracheobronchial tumors, transitional cell cancer of the renal pelvis and ureter, urethral cancer, vaginal cancer, vascular tumors, vulvar cancer, or Wilms tumors.

The cancer may be lung cancer. The lung cancer may be an epithelial lung cancer. The epithelial lung cancer may be non-small cell lung cancer (NSCLC).

The one or more compounds, e.g., one or more cancer drugs used in screening may be one or more chemotherapy drugs. The one or more chemotherapy drugs can be an alkylating agent, an antimetabolite, an anti-tumor antibiotic, a topoisomerase inhibitor, a mitotic inhibitors, a corticosteroid, a steroid, or another drug.

The one or more chemotherapy drugs may be one or more alkylating agents. An alkylating agent can keep cells from reproducing by damaging its DNA. These drugs can work in all phases of the cell cycle and can be used to treat many different cancers. The one or more alkylating agents can be altretamine, bendamustine, busulfan, carboplatin, carmustine, chlorambucil, cisplatin, cyclophosphamide, dacarbazine, ifosfamide, lomustine, mechlorethamine, melphalan, oxaliplatin, temozolomide, thiotepa, or trabectedin.

The one or more chemotherapy drugs can be one or more nitrosoureas. A nitrosourea can be a group of alkylating agents that can cross the blood-brain barrier. The one or more nitrosoureas can be carmustine, lomustine, or streptozocin.

**EP 4 298 239 B1**

The one or more chemotherapy drugs can be one or more antimetabolites. An antimetabolites can interfere with DNA and RNA by acting as a substitute for the normal building blocks of RNA and DNA; when this happens, the DNA cannot make copies of itself and a cell cannot reproduce. The one or more antimetabolites can be azacitidine, 5-fluorouracil, 6-mercaptopurine, capecitabine, cladribine, clofarabine, cytarabine, decitabine, floxuridine, fludarabine, hydroxyurea, methotrexate, nelarabine,pemetrexed, pentostatin, pralatrexate, thioguanine, or trifluridine/tipiracil combination.

The one or more chemotherapy drugs can be one or more anti-tumor antibiotics. An anti-tumor antibiotic can work by changing the DNA inside cancer cells to keep them from growing and multiplying. The one or more anti-tumor antibiotics can be an anthracycline, bleomycin, dactinomycin, mitomycin-C, or mitoxantrone. An anthracycline can be an anti-tumor antibiotic that interferes with enzymes involved in copying DNA during the cell cycle; they can bind with DNA so it cannot make copies of itself, and a cell cannot reproduce. An anthracycline can be daunorubicin, doxorubicin (Adriamycin), doxorubicin liposomal, epirubicin, idarubicin, or valrubicin.

The one or more chemotherapy drugs can be one or more topoisomerase inhibitors. A topoisomerase inhibitor can interfere with topoisomerase enzymes, which can help separate the strands of DNA so they can be copied. The topoisomerase inhibitor can be a Topoisomerase I inhibitor (a campthothecin). The topoisomerase I inhibitor can be irinotecan, irinotecan liposomal, or topotecan. The topoisomerase inhibitor can be a topoisomerase II inhibitor (a epipodophyllotixin). The topoisomerase II inhibitor can be etoposide (VP-16), mitoxantrone, or teniposide.

The one or more chemotherapy drugs can be one or more mitotic inhibitors. A mitotic inhibitor can be a compound derived from a natural product such as a plant; they can work by stopping cells from dividing to form new cells. The one or more mitotic inhibitors can be a taxane (e.g. cabazitaxel, docetaxel, nab-paclitaxel, or paclitaxel) or a vinca alkaloid (e.g., vinblastine, vincristine, vincristine liposomal, or vinorelbine).

The one or more chemotherapy drugs can be one or more corticosteroids. A corticosteroid can be a natural hormone and hormone-like drug. The one or more corticosteroids can be prednisone, methylprednisolone, and dexamethasone.

The one or more chemotherapy drugs can be all-trans-retinoic acid, arsenic trioxide, asparaginase, eribulin, hydroxyurea, ixabepilone, mitotane, omacetaxine, pegaspargase, procarbazine, romidepsin, or vorinostat.

The one or more chemotherapy drugs can be one or more checkpoint inhibitor drugs. Checkpoint inhibitor drugs can be inhibitors of PD-1 (programmed cell death protein 1), PD-L1 (programmed death ligand 1), or CTLA4 (cytotoxic t-lymphocyte-associated protein 4). Checkpoint inhibitors can target key regulators of the immune system that, when stimulated, can dampen the immune response to an immunologic stimulus; some cancers protect themselves from attack by stimulating immune target checkpoints. The one or more checkpoint inhibitor drugs can be monoclonal antibodies. The one or more PD-1 inhibitors can be pembrolizumab, nivolumab, and cemiplimab. The one or more PD-L1 inhibitors can be atezolizumab, avelumab, and durvalumab. The one or more CTLA4 inhibitors can be ipilimumab.

The one or more chemotherapy drugs can be one or more tissue factor (TF) inhibitors, such as tissue factor pathway inhibitor (TFPI), a natural inhibitor of tissue factor coagulant and signaling activities. The one or more drugs can be one or more heparins and heparin derivatives, which can be used to induce the release of TFPI from the vascular endothelium.

The cancer drug can be a targeted therapy. The one or more drugs can be a hormone therapy. The one or more drugs can be an immunotherapy. The one or more drugs can be one or more monoclonal antibodies.

The one or more monoclonal antibodies can be atezolizumab, avelumab, bevacizumab, cemiplimab, cetuximab, daratumumab, dinutuximab, durvalumab, elotuzumab, ipilimumab, isatuximab, mogamulizumab, necitumumab, nivolumab, obinutuzumab, ofatumumab, olaratumab, panitumumab, pembrolizumab, pertuzumab, ramucirumab, rituximab, and trastuzumab. The one or more monoclonal antibodies can be antibody-drug conjugates. The one or more antibody-drug conjugates can be gemtuzumab ozogamicin, brentuximab vedotin, trastuzumab emtansine, inotuzumab ozogamicin, polatuzumab vedotin, enforfumab vedotin, trastuzumab deruxtecan, Sacituzumab govitecan, moxetumomab pasudotox, ibritumomab tiuxetan, iodine tositumomab, and blinatumomab.

The drug, e.g., cancer drugs may be used singly. The cancer drugs used in screening may be used in combination. For example, a cancer drug can be combined in a treatment with 1, 2, 3, 4, 5, 6, 7, 8, 9, 10, or more additional cancer drugs. A cancer drug can be combined in a treatment with 1, 2, 3, 4, 5, 6, 7, 8, 9, 10, or more additional non-cancer drugs. A cancer drug can be, for example, at least one CTLA-4 inhibitor and at least one PD-1 inhibitor. A cancer drug be, for example, one or more drugs selected from the group consisting of chemotherapy drugs, CTLA-4 inhibitors, PD-1 inhibitors, PD-L1 inhibitors, and TF inhibitors.

A single cell can be contacted with a combination of one or more drugs from one or more groups (e.g., 2, 3, 4, 5, 6, 7, 8, 9, 10, 11, 12, 13, 14, 15, or 16 groups) of alkylating agents, antimetabolites, anti-tumor antibiotics, topoisomerase inhibitors, mitotic inhibitors, corticosteroids, steroids, CTLA-4 inhibitors, PD-1 inhibitors, PD-L1 inhibitors, TF inhibitors, monoclonal antibodies, antibody-drug conjugates, and other drugs. A single cell can be contacted with at least one alkylating agent and at least one antimetabolite. A single cell can be contacted with at least one alkylating agent and at least one anti-tumor antibiotic. A single cell can be contacted with at least one alkylating agent and at least one topoisomerase inhibitor. A single cell can be contacted with at least one alkylating agent and at least one mitotic inhibitor. A single cell can be contacted with at least one alkylating agent and at least one corticosteroid. A single cell can be contacted with at least one alkylating agent and at least one steroid. A single cell can be contacted with at least one alkylating agent and at least one CTLA-4 inhibitor. A single cell can be contacted with at least one alkylating agent and at least one PD-1 inhibitor. A single cell can be contacted with at least one alkylating agent and at least one PD-L1 inhibitor. A single cell can be contacted with at least one alkylating agent and at least one TF inhibitor. A single cell can be contacted with at least one alkylating agent and at least one monoclonal antibody. A single cell can be contacted with at least one alkylating agent and at least one antibody-drug conjugate. A single cell can be contacted with at least one alkylating agent and at least one other drug. A single cell can be contacted with at least one antimetabolite and at least one anti-tumor antibiotic. A single cell can be contacted with at least one antimetabolite and at least one topoisomerase inhibitor. A single cell can be contacted with at least one antimetabolite and at least one mitotic inhibitor. A single cell can be contacted with at least one antimetabolite and at least one corticosteroid. A single cell can be contacted with at least one antimetabolite and at least one steroid. A single cell can be contacted with at least one antimetabolite and at least one CTLA-4 inhibitor. A single cell can be contacted with at least one antimetabolite and at least one PD-1 inhibitor. A single cell can be contacted with at least one antimetabolite and at least one PD-L1 inhibitor. A single cell can be contacted with at least one antimetabolite and at least one TF inhibitor. A single cell can be contacted with at least one antimetabolite and at least one monoclonal antibody. A single cell can be contacted with at least one antimetabolite and at least one antibody-drug conjugate. A single cell can be contacted with at least one antimetabolite and at least one other drug. A single cell can be contacted with at least one anti-tumor antibiotic and at least one topoisomerase inhibitor. A single cell can be contacted with at least one anti-tumor antibiotic and at least one mitotic inhibitor, A single cell can be contacted with at least one anti-tumor antibiotic and at least one corticosteroid. A single cell can be contacted with at least one anti-tumor antibiotic and at least one steroid. A single cell can be contacted with at least one anti-tumor antibiotic and at least one CTLA-4 inhibitor. A single cell can be contacted with at least one anti-tumor antibiotic and at least one PD-1 inhibitor. A single cell can be contacted with at least one anti-tumor antibiotic and at least one PD-L1 inhibitor. A single cell can be contacted with at least one anti-tumor antibiotic and at least one TF inhibitor. A single cell can be contacted with at least one anti-tumor antibiotic and at least one monoclonal antibody. A single cell can be contacted with at least one anti-tumor antibiotic and at least one antibody-drug conjugate. A single cell can be contacted with at least one anti-tumor antibiotic and at least one other drug. A single cell can be contacted with at least one topoisomerase inhibitor and at least one mitotic inhibitor. A single cell can be contacted with at least one topoisomerase inhibitor and at least one corticosteroid. A single cell can be contacted with at least one topoisomerase inhibitor and at least one steroid. A single cell can be contacted with at least one topoisomerase inhibitor and at least one CTLA-4 inhibitor. A single cell can be contacted with at least one topoisomerase inhibitor and at least one PD-1 inhibitor. A single cell can be contacted with at least one topoisomerase inhibitor and at least one PD-L1 inhibitor. A single cell can be contacted with at least one topoisomerase inhibitor and at least one TF inhibitor. A single cell can be contacted with at least one topoisomerase inhibitor and at least one monoclonal antibody. A single cell can be contacted with at least one topoisomerase inhibitor and at least one antibody-drug conjugate. A single cell can be contacted with at least topoisomerase inhibitor and at least one other drug. A single cell can be contacted with at least one mitotic inhibitor and at least one corticosteroid. A single cell can be contacted with at least one mitotic inhibitor and at least one steroid. A single cell can be contacted with at least one mitotic inhibitor and at least one CTLA-4 inhibitor. A single cell can be contacted with at least one mitotic inhibitor and at least one PD-1 inhibitor. A single cell can be contacted with at least one mitotic inhibitor and at least one PD-L1 inhibitor. A single cell can be contacted with at least one mitotic inhibitor and at least one TF inhibitor. A single cell can be contacted with at least one mitotic inhibitor and at least one monoclonal antibody. A single cell can be contacted with at least one mitotic inhibitor and at least one antibody-drug conjugate. A single cell can be contacted with at least one mitotic inhibitor and at least one other drug. A single cell can be contacted with at least one corticosteroid at least one steroid. A single cell can be contacted with at least one corticosteroid at least one CTLA-4 inhibitor. A single cell can be contacted with at least one corticosteroid at least one PD-1 inhibitor. A single cell can be contacted with at least one corticosteroid at least one PD-L1 inhibitor. A single cell can be contacted with at least one corticosteroid at least one TF inhibitor. A single cell can be contacted with at least one corticosteroid at least one monoclonal antibody. A single cell can be contacted with at least one corticosteroid at least one antibody-drug conjugate. A single cell can be contacted with at least one corticosteroid at least one other drug. A single cell can be contacted with at least one steroid at least one CTLA-4 inhibitor. A single cell can be contacted with at least one steroid at least one PD-1 inhibitor. A single cell can be contacted with at least one steroid at least one PD-L1 inhibitor. A single cell can be contacted with at least one steroid at least one TF inhibitor. A single cell can be contacted with at least one steroid at least one monoclonal antibody. A single cell can be contacted with at least one steroid at least one antibody-drug conjugate. A single cell can be contacted with at least one steroid at least one other drug. A single cell can be contacted with at least one CTLA-4 inhibitor at least one PD-1 inhibitor. A single cell can be contacted with at least one CTLA-4 inhibitor at least one PD-L1 inhibitor. A single cell can be contacted with at least one CTLA-4 inhibitor at least one TF inhibitor. A single cell can be contacted with at least one CTLA-4 inhibitor at least one monoclonal antibody. A single cell can be contacted with at least one CTLA-4 inhibitor at least one antibody-drug conjugate. A single cell can be contacted with at least one CTLA-4 inhibitor at least one other drug. A single cell can be contacted with at least one PD-1 inhibitor at least one PD-L1 inhibitor. A single cell can be contacted with at least one PD-1 inhibitor at least one TF inhibitor. A single cell can be contacted with at least one PD-1 inhibitor at least one monoclonal antibody. A single cell can be contacted with at least one PD-1 inhibitor at least one antibody-drug conjugate. A single cell can be contacted with at least one PD-1 inhibitor at least one other drug. A single cell can be contacted with at least one PD-L1 inhibitor at least one TF inhibitor. A single cell can be contacted with at least one PD-L1 inhibitor at least one monoclonal antibody. A single cell can be contacted with at least one PD-L1 inhibitor at least one antibody-drug conjugate. A single cell can be contacted with at least one PD-L1 inhibitor at least one other drug. A single cell can be contacted with at least one TF inhibitor at least one monoclonal antibody. A single cell can be contacted with at least one TF inhibitor at least one antibody-drug conjugate. A single cell can be contacted with at least one TF inhibitor at least one other drug. A single cell can be contacted with at least one monoclonal antibody at least one antibody-drug conjugate. A single cell can be contacted with at least one monoclonal antibody at least one other drug. A single cell can be contacted with at least one antibody-drug conjugate at least one other drug.

## Computer systems

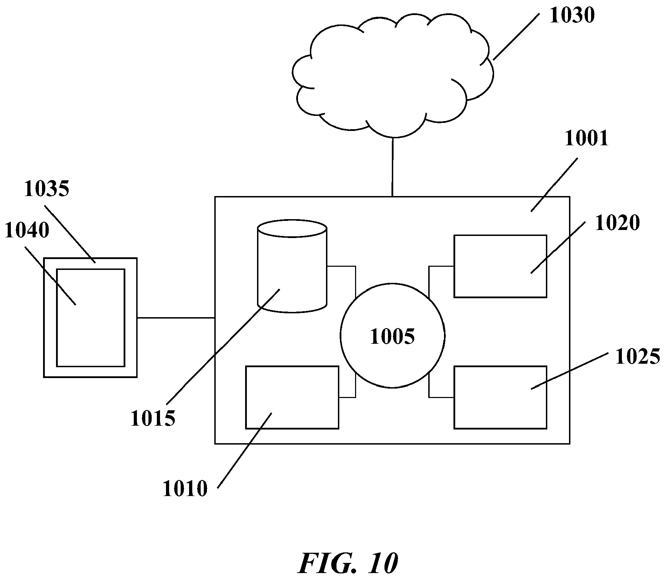

The present disclosure provides computer systems that are programmed to implement methods of the disclosure. **FIG. 10** shows a computer system **1001** that is programmed or otherwise configured to process or analyze sequencing reads. The computer system **1001** can regulate various features of the present disclosure, such as, for example, aligning sequencing reads, indexing sequencing reads to a cell, partition, etc. The computer system **1001** can be an electronic device of a user or a computer system that is remotely located with respect to the electronic device. The electronic device can be a mobile electronic device.

The computer system **1001** includes a central processing unit (CPU, also "processor" and "computer processor" herein) **1005,** which can be a single core or multi core processor, or a plurality of processors for parallel processing. The computer system **1001** also includes memory or memory location **1010** (e.g., random-access memory, read-only memory, flash memory), electronic storage unit **1015** (e.g., hard disk), communication interface **1020** (e.g., network adapter) for communicating with one or more other systems, and peripheral devices **1025,** such as cache, other memory, data storage and/or electronic display adapters. The memory **1010,** storage unit **1015,** interface **1020** and peripheral devices **1025** are in communication with the CPU **1005** through a communication bus (solid lines), such as a motherboard. The storage unit **1015** can be a data storage unit (or data repository) for storing data. The computer system **1001** can be operatively coupled to a computer network ("network") **1030** with the aid of the communication interface **1020.** The network **1030** can be the Internet, an internet and/or extranet, or an intranet and/or extranet that is in communication with the Internet. The network **1030** in some cases is a telecommunication and/or data network. The network **1030** can include one or more computer servers, which can enable distributed computing, such as cloud computing. The network **1030,** in some cases with the aid of the computer system **1001,** can implement a peer-to-peer network, which may enable devices coupled to the computer system **1001** to behave as a client or a server.

The CPU **1005** can execute a sequence of machine-readable instructions, which can be embodied in a program or software. The instructions may be stored in a memory location, such as the memory **1010.** The instructions can be directed to the CPU **1005,** which can subsequently program or otherwise configure the CPU **1005** to implement methods of the present disclosure. Examples of operations performed by the CPU **1005** can include fetch, decode, execute, and writeback.

The CPU **1005** can be part of a circuit, such as an integrated circuit. One or more other components of the system **1001** can be included in the circuit. In some cases, the circuit is an application specific integrated circuit (ASIC).

The storage unit **1015** can store files, such as drivers, libraries and saved programs. The storage unit **1015** can store user data, e.g., user preferences and user programs. The computer system **1001** in some cases can include one or more additional data storage units that are external to the computer system **1001,** such as located on a remote server that is in communication with the computer system **1001** through an intranet or the Internet.

The computer system **1001** can communicate with one or more remote computer systems through the network **1030.** The computer system **1001** may communicate with a remote computer system of a user (e.g., operator). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system **1001** via the network **1030.**

Methods as described herein can be implemented by way of machine (e.g., computer processor) executable code stored on an electronic storage location of the computer system **1001,** such as, for example, on the memory **1010** or electronic storage unit **1015.** The machine executable or machine readable code can be provided in the form of software. During use, the code can be executed by the processor **1005.** In some cases, the code can be retrieved from the storage unit **1015** and stored on the memory **1010** for ready access by the processor **1005.** In some situations, the electronic storage unit **1015** can be precluded, and machine-executable instructions are stored on memory **1010.**

The code can be pre-compiled and configured for use with a machine having a processor adapted to execute the code, or can be compiled during runtime. The code can be supplied in a programming language that can be selected to enable the code to execute in a pre-compiled or as-compiled fashion.

Features of the systems and methods described herein, such as the computer system **1001,** can be embodied in programming. Various features of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system **1001** can include or be in communication with an electronic display **1035** that comprises a user interface (UI) **1040** for providing, for example, results of sequencing analysis, etc.. Examples of UIs include, without limitation, a graphical user interface (GUI) and web-based user interface.

Methods and systems of the present disclosure can be implemented by way of one or more algorithms. An algorithm can be implemented by way of software upon execution by the central processing unit 1005. The algorithm can, for example, perform sequencing.

Devices, systems, compositions and methods of the present disclosure may be used for various applications, such as, for example, processing a single analyte (e.g., RNA, DNA, or protein) or multiple analytes (e.g., DNA and RNA, DNA and protein, RNA and protein, or RNA, DNA and protein) from a single cell. For example, a analyte carrier and/or biological particle (e.g., a cell or cell bead) is partitioned in a partition (e.g., droplet), and multiple analytes from the analyte carrier and/or biological particle are processed for subsequent processing. The multiple analytes may be from the single cell. This may enable, for example, simultaneous proteomic, transcriptomic and genomic analysis of the cell.

## Examples

### Example 1 - High Throughput Sample Preparation for single cell/nucleus RNA sequencing

Single cell gene expression solutions (10x Genomics) enable the generation of libraries to study gene expression profiles, cell surface protein expression, and/or CRISPR screening in hundreds, thousands, and even a million cells (10x Genomics Chromium X instrument). Single cell samples may be multiplexed with labeling agents (3' CellPlex Kit - 10x Genomics or TotalSeq antibodies - BioLegend). Single cell samples may be prepared for multiplex labeling (lipophilic agent or protein labeling agent) using a high-throughput, microwell plate-based approach, which can enable sample labeling at a scale suitable for application such as drug and/or CRISPR screening.

Reagents: 3' CellPlex Kit Set A (10x Genomics), Microplate, 96 well, pp, v-bottom, (chimney well), natural (Griener Bio-One), UltraPure Bovine Serum Albumin (Thermo Fisher Scientific), Phosphate Buffered Saline (PBS), 1X without Calcium and Magnesium (Corning), TotalSeq™ Antibody-Oligonucleotide Conjugates (BioLegend) including TotalSeq™-B and TotalSeq™-C, and Fetal Bovine Serum (FBS) (VWR).

High Throughput Cell Multiplexing Oligonucleotide Plate: Thaw oligonucleotides from the 3' CellPlex Kit Set A (10x Genomics), which contains 12 different multiplexing oligonucleotides conjugated to a lipid, at room temperature, then vortex 5 sec at maximum speed and centrifuge briefly for 5 sec. In a 96-well plate, distribute 105 ul of a first Cell Multiplexing oligo into each well of the first column of the plate, i.e., rows A-H in column 1 (see **FIG. 15**). Other Cell Multiplexing oligos (from the set of 12) can be distributed to each well in other columns of the same plate, e.g., a second Cell Multiplexing oligo into rows A-H in column 2. The plate may be sealed with a plate sealer and left at room temperature until used. The Cell Multiplexing oligos from this first plate may be subsequently transferred to a second plate which contains cellular samples, e.g., single cells or nuclei, in each well for staining of the cellular sample with a different Cell Multiplexing oligo in each row of a particular column.

Cells per well: The cells per well may be 0.1 - 2 x $10^5$ cells per well in an additional 96-well cell sample plate for staining. For cells in suspension, 0.1 - 2 x $10^5$ cells per well may be used. For adherent cells, 0.1 - 1 x $10^5$ cells per well may be used. If transferring adherent cells, was cells with PBS (PBS + 1% BSA chilled at 4°C), trypsinize, and quench with cell culture media prior to transfer. Centrifuge cells at room temperature. Use of a swinging bucket rotor motor is recommended for higher cell recovery. Centrifugation speed and time depends on sample type - cell lines (speed: 300 rcf for 5 minutes), PBMCs/dissociated brain tissue (speed 400 rcf for 5 minutes) and dissociated tumor cells (speed 150 rcf for 10 minutes). Following centrifugation, supernatant is removed using a multichannel vacuum pump without disturbing the pellet. Using a multichannel pipette, add 100 ul of the Cell Multiplexing oligo (room temperature) from the Cell Multiplexing oligo plate to the cell sample plate for staining. Gently pipette mix 10-15 times to resuspend. Incubate the cell sample plate with CellPlex oligos for 5 minutes at room temperature. Wash by adding 200 ul of PBS (PBS + 1% BSA chilled at 4°C) to the samples and gently pipette mix. Remove the supernatant with a multichannel vacuum pump.

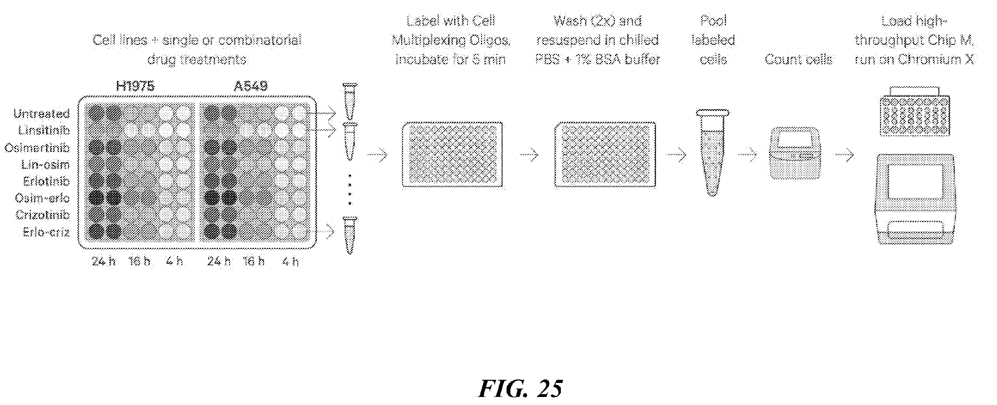

Add 300 ul PBS + 1% BSA to the samples and gently pipette mix. Remove the supernatant with a multichannel vacuum pump. These two steps are repeated for a total of two washes. Resuspend in 20-50 ul PBS + 1% BSA. Each row of the staining plate can be combined into a separate 2-ml microcentrifuge tube, which results in a 12-plexed sample where all 12 rows contained a different Cell Multiplexing oligo, an 11-plexed samples where 11 of 12 rows contained a different Cell Multiplexing oligo, etc. Determine cell concentrations and viability of the pooled sample, e.g., using a Countess II Automated Cell counter or a hemocytometer. The pooled samples may be further processed for single cell or nucleus analysis, e.g., Chromium Single Cell RNA Sequencing protocols with Feature Barcode technology for Cell Multiplexing (10x Genomics) **(FIG. 25).**

## Example 2 - Time course experiment using a drug-treated cell line

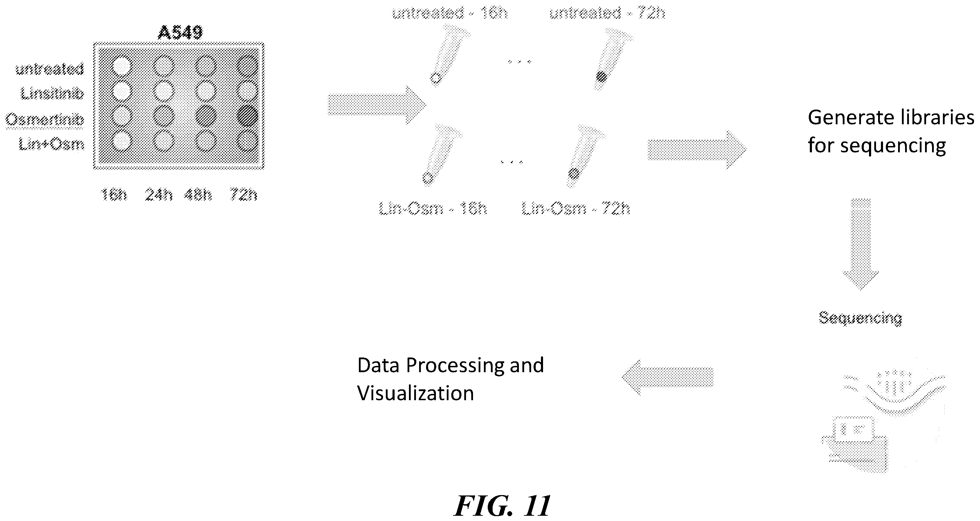

An experiment was performed to measure gene expression levels in a drug-treated A549 human lung (carcinoma) cell line (ATCC). 500-1000 cells were used per sample. Samples were treated with a combination of two lung cancer drugs (Osimertinib and Linsitinib) using the Single Cell 3' Gene Expression Low Throughput Kit and Chromium Controller (10x Genomics) and the impact of drug treatment compared with untreated samples at over 72 hours. Uniform Manifold Approximation and Projection (UMAP) plots were generated from the resulting sequencing libraries. One UMAP plot overlayed each time point and the two drug conditions, and the untreated control. As expected, drug treatments results in changes in the UMAP projections at all timepoints. The experimental workflow is generally depicted in **FIG. 11.**

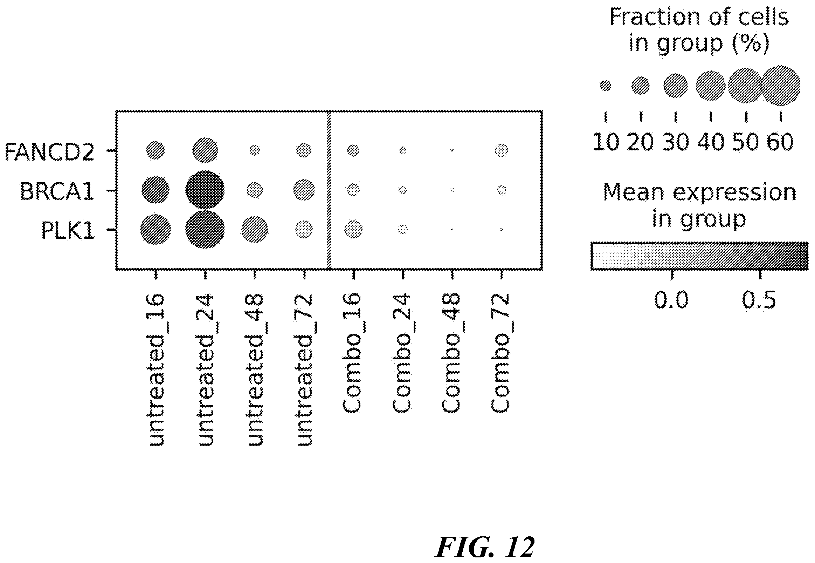

The expression levels of genes associated with pathways known to be affected by these drugs was examined. In **FIG. 12,** the expression of 3 genes in the DNA repair pathway (FANCD2, BRCA1 and PLK1) in one of the treated samples is shown. The dot plot shows that the drugs have reached the maximum effect on these genes at around the 24-hour timepoint. Additional experiments were designed to focus on sub-24-hour timepoints in order to observe the effects of the drug when it is most active.

## Example 3 - High-throughput drug screening experiment

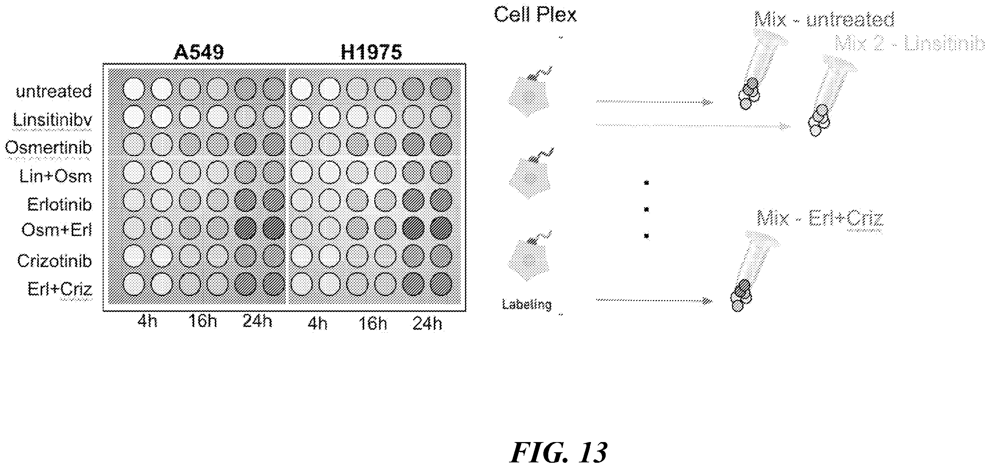

An experiment was performed to measure gene expression levels in two drug-treated cell lines: A549 human non-small cell lung cancer or NSCLC (carcinoma) cell line (ATCC) and H1975 human lung non-small cell carcinoma cell line (ATCC). Eight different conditions were used (1: Untreated; 2: Linsitinib (Lin); 3: Osimertinib (Osm); 4: Linsitinib + Osimertinib; 5: Erlotinib (Erl); 6: Osimertinib + Erlotinib; 7: Crizotinib (Criz); 8: Erlotinib + Crizotinib) on cell samples over 3 different timepoints from (4 hours, 16 hours, and 24 hours) (FIG. 25). Cells were seeded in wells (~40,000 cells per well) on a 96-well plate, treated, and then harvested. Following treatment but prior to harvesting, the cells were tagged using the 3' CellPlex technology (10x Genomics), which allows cells to be labeled with an oligonucleotide-lipophilic conjugated reagent. For this experiment, 12 different tags were used to label various experimental conditions, e.g., two cell lines, 3 different time points, 8 different experimental treatment conditions, etc. As shown in **FIG. 13,** row one of the 96-well plate corresponds to the untreated sample and a different tag was applied to each well in row one. Each well in row one was labeled with a different tag that corresponds to a specific experimental condition (or sample) - 12 rows labeled with 12 different tags. Each tag includes a different oligonucleotide having a specific barcode sequence that allows, upon sequencing, a determination of the sample origin of each single cell. At the harvesting stage, cells from each well in each row were removed (i.e., trypsin treatment), pooled together as depicted in **FIG. 13.** e.g., row 1: Mix - untreated; row 2: Mix 2- Linsitinib; and row 8: Mix - Erl+Criz. Each of the eight pooled samples were loaded onto a high-throughput (HTP) microfluidic chip (10x Genomics) for 3' gene expression single cell analysis. The HTP chip allowed each sample to be loaded into a sample well for processing on the Chromium X instrument (10x Genomics). Following barcoding in Gel beads-in-Emulsions (GEMs), sequencing libraries were prepared and enriched using a pan-cancer and gene signature panel (single cell Targeted Gene Expression solution - 10x Genomics). Final libraries were sequenced to 10,000 reads per cell.

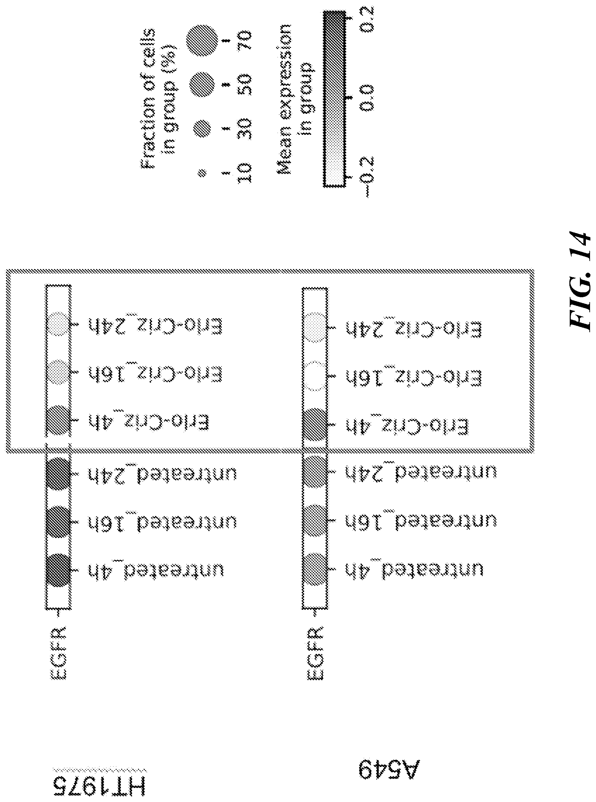

Using UMAP plots for nearly 400,000 total cells profiled, pooled samples were demultiplexed with Cell Ranger 6.0 (10x Genomics) and distinct transcriptional profiles for each cell line were observed, independent of treatment conditions. The effect of drug treatments on each cell line over time was investigated and clustering of each cell line cluster was found to be driven by the treatment condition. For each cell line, it was observed that a specific treatment condition leads to changes in the transcriptional profiles that are common to both cell lines. The Erlotinib treatment led to notable changes in the transcriptional profiles over the short time course that was examined, with the biggest change occurring between 4 and 16 hours (see **FIG. 14**). Upon examination of the specific genes driving these global changes, notable downregulation in the expression of EGFR was observed. Erlotinib inhibits EGFR at the protein level but the rapid post-treatment downregulation at the mRNA level was striking. This experiment (conducted in a matter of days) was able to screen 96 conditions (or 192 individual samples) simultaneously on almost half a million cells from a single HT Kit on a single microfluidic chip for subsequent processing on the Chromium X (10x Genomics).

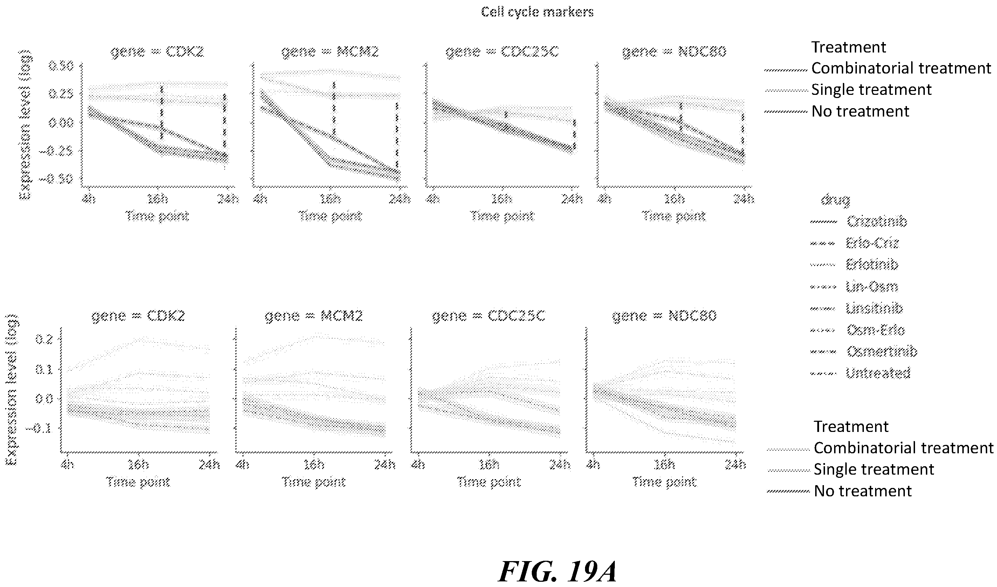

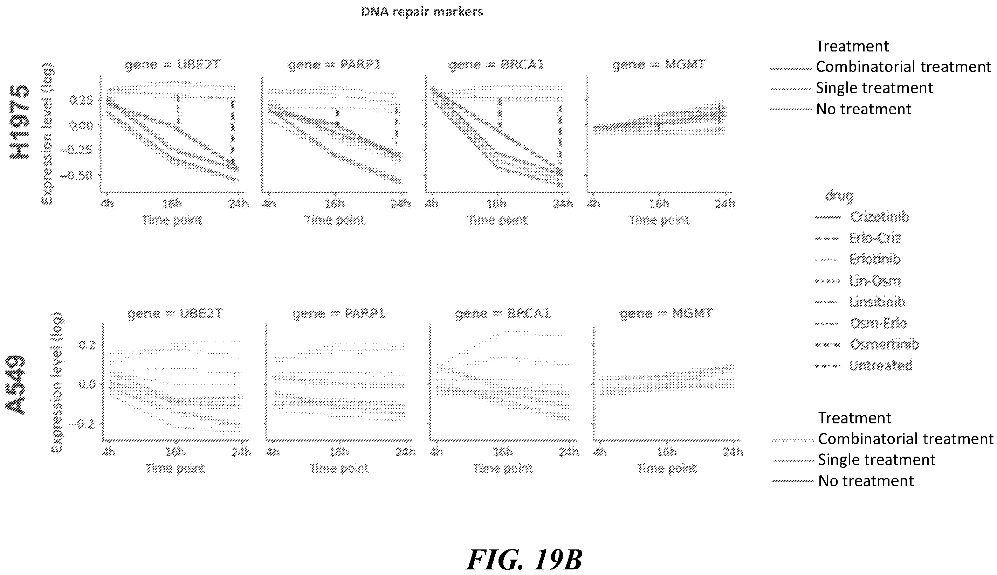

H1975 cells were observed to be more responsive to drug treatments than A549 cells in the cell cycle **(FIG. 19A)** and DNA repair **(FIG. 19B)** pathways.

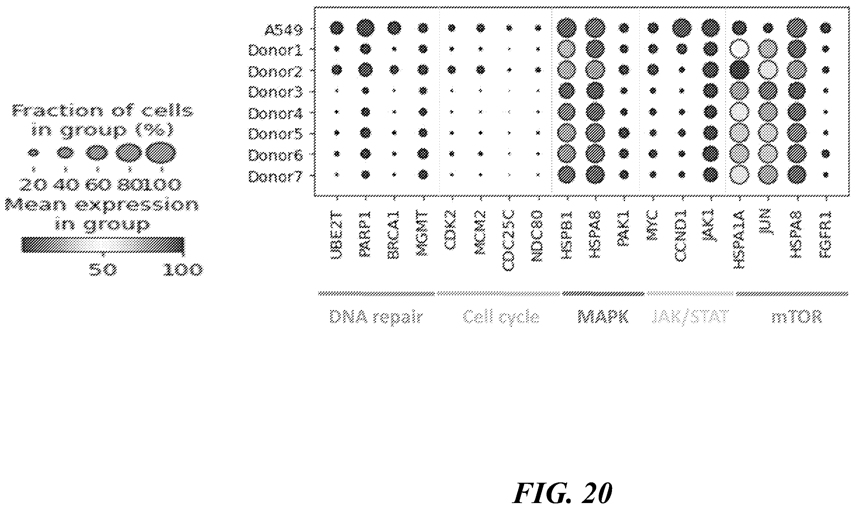

The gene expression pattern for the untreated A549 NSCLC cell line (a KRAS mutated cell line) was compared to the gene expression patterns of NSCLC human donor material from seven different individuals (Dissociated Tumor Cells or DTCs from Discovery Life Sciences), also generated using an HT kit on the Chromium X (10x Genomics). As shown in **FIG. 20,** A549 and the DTCs from seven NSCLC donors have similar gene expression patterns in DNA repair, cell cycle, and MAPK pathways but differ in JAK/STAT and mTOR pathways. The KRAS mutated cell line A549 shares common signaling pathways with primary NSCLC donor material suggests that A549 could be used to screen for targeted drug combinations to identify treatments for KRAS mutated NSCLC.

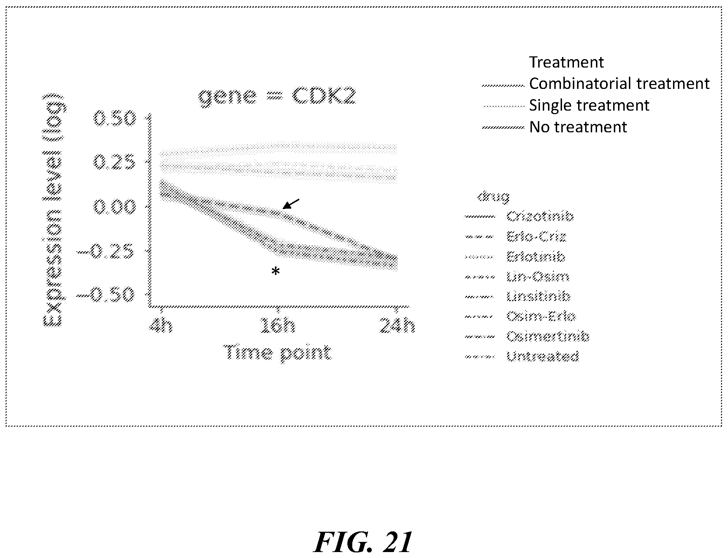

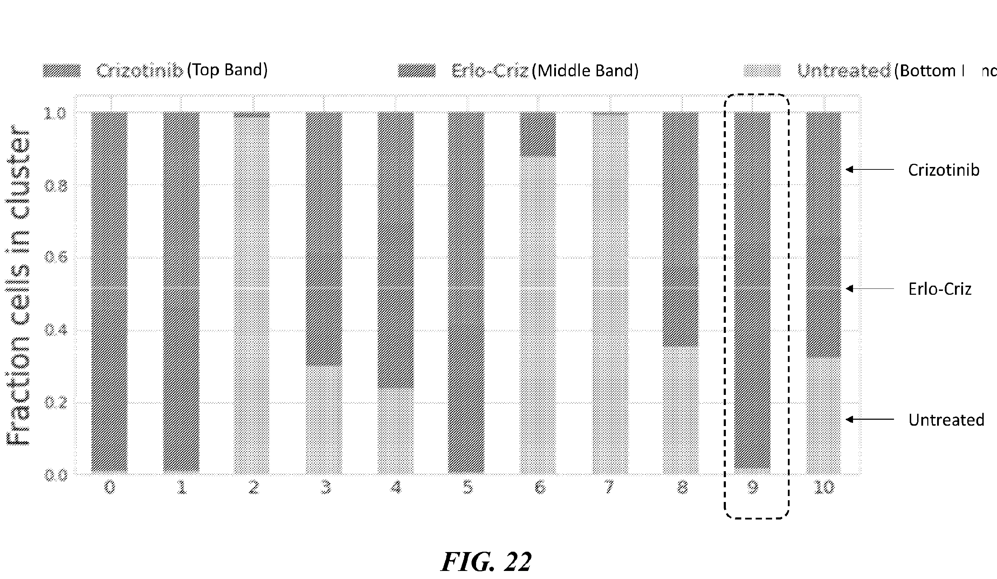

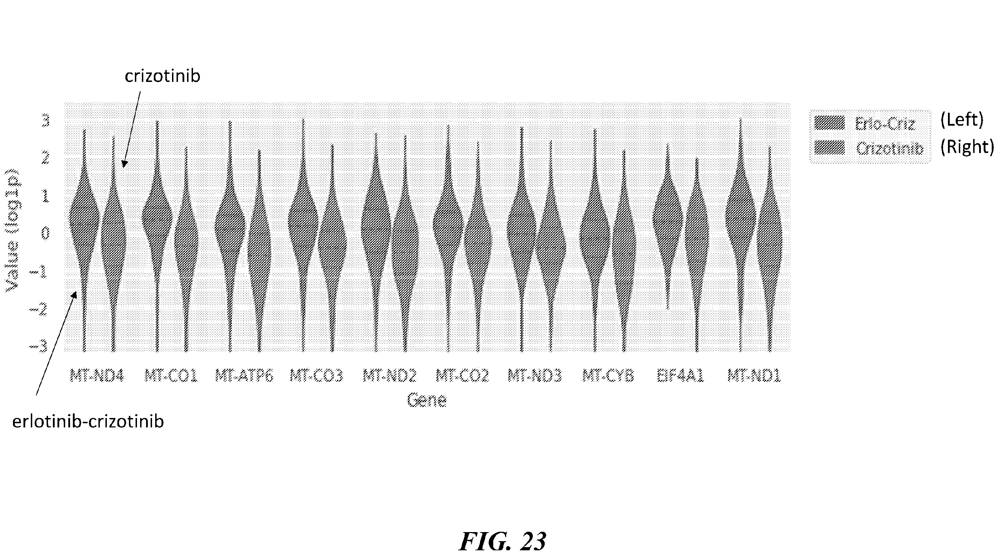

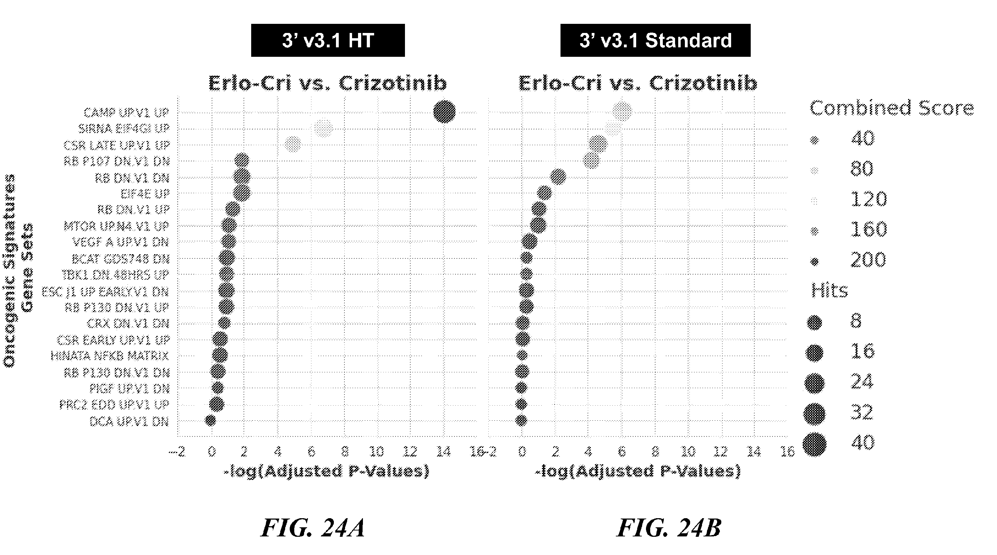

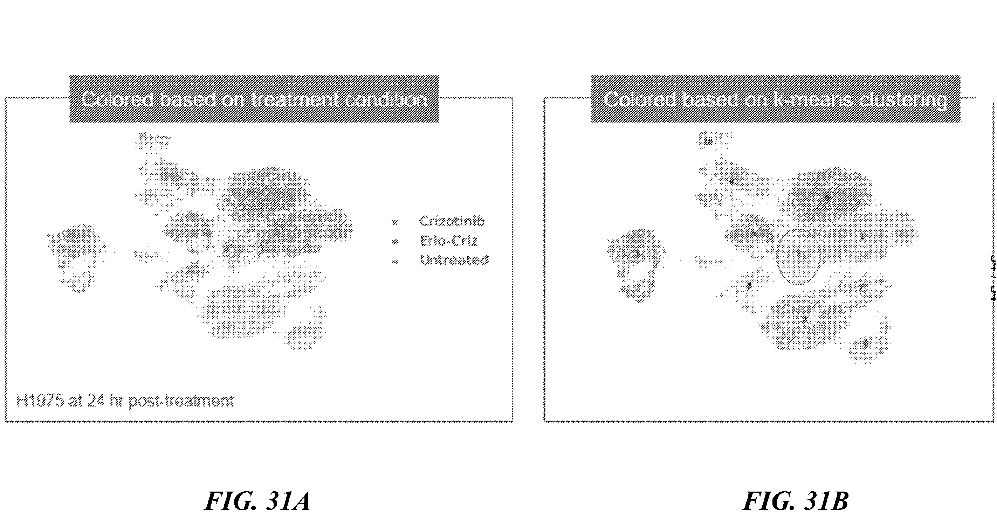

As shown in **FIG. 21,** two of the single drug treatment samples, Crizotinib and Osimertinib, demonstrated downregulation of CDK2 at 16 hours (see asterisk). The same downregulation was achieved at 24 hours using a combination of Crizotinib and Osimertinib. Gene expression for the single treatment with Crizotinib was further analyzed to evaluate the biological differences caused by combinatorial drug effects at 24 hours. Cell clustering analysis was performed for three treatment groups, untreated (as control), Crizotinib (single treatment) and Erlotinib-Crizotinib (combinatorial treatment). UMAP projection of the cells treated with 3 different conditions revealed 11 distinct clusters in HT **(FIG. 31A-B).** The addition of Erlotinib to the treatment might mildly affect cell transcriptome, as most of the crizotinib only-treated cells overlaid with the cells receiving Erlotinib-Crizotinib-combinatorial treatment. As depicted in **FIG. 22,** cluster 9 (dashed line box) of the 11 distinct clusters was observed to be enriched with cells treated with Erlotinib-Crizotinib. **FIG. 23** shows that treatment with Erlotinib-Crizotinib resulted in increased mitochondrial gene expression (per gene from left to right - left plot = Erlotinib-Crizotinib; right plot = Crizotinib). The effect of a combined treatment or a single drug treatment is similar but the impact appears to be stronger with the combined treatment. Cells with more than 25% mitochondrial genes were removed from analysis so it appears that the stronger impact on mitochondrial genes is via the cAMP pathway, which is likely in addition to impact based on cell death. A selection of top rank 1000 gene set enrichment analysis (GSEA) revealed that the cAMP pathway is significantly enriched (see dot plot in **FIG. 24A)** with a combined score of 208.6 and a number of overlapped genes 44. The cAMP pathway regulates cell cycle, cell growth and cell proliferation, indicating that Erlotinib-Crizotinib-combinatorial treatment led to downregulation of cell cycle through cAMP pathway. Furthermore, cAMP can play a role in the functional gene network and thus impacts cell cycle check points and DNA double-strand break repair.

## Example 4 - Multiomic Library Construction

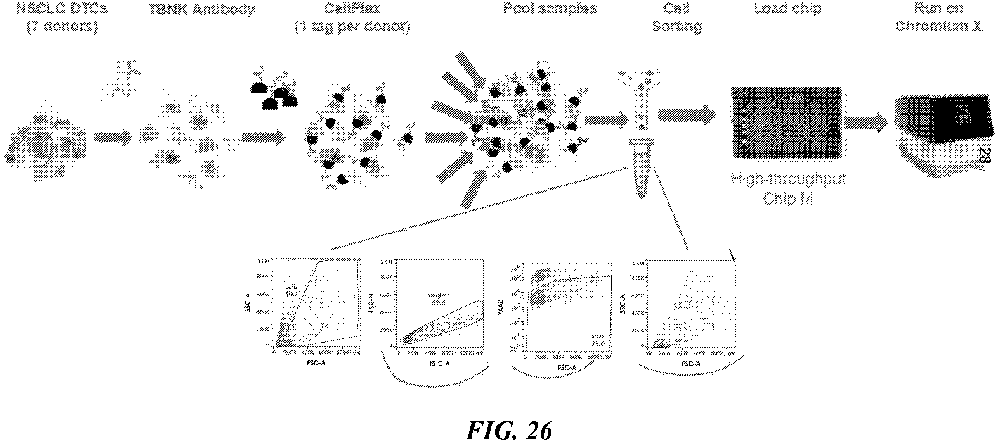

In this experiment, dissociated tumor cells from seven NSCLC patients were acquired. As shown in **FIG. 26,** the samples were labeled with the TotalSeq B Human TBNK cocktail (designed to react with immune cells defined by the expression of surface antigens CD19, CD3, CD16, CD4, CD11c, CD56, CD14, CD8, and CD45) in order to profile cell surface protein expression as well as gene expression with single cell resolution. The samples were labeled using CellPlex, pooled, and run on a FACS sorter. The 7AAD dead cell marker was used to discriminate between viable and dead cells so that viable cells could be selected. The samples were loaded onto a high-throughput Chip M and run on the Chromium X instrument.

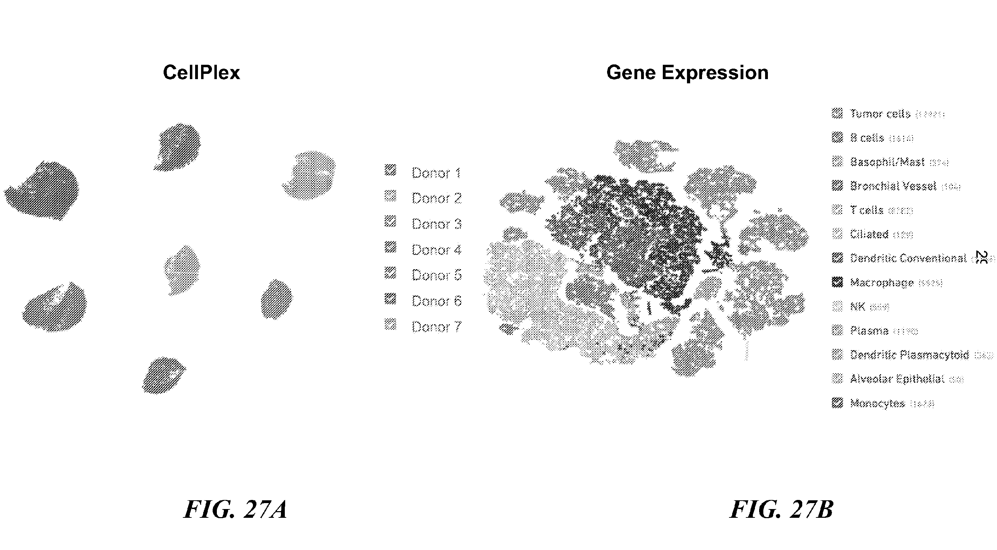

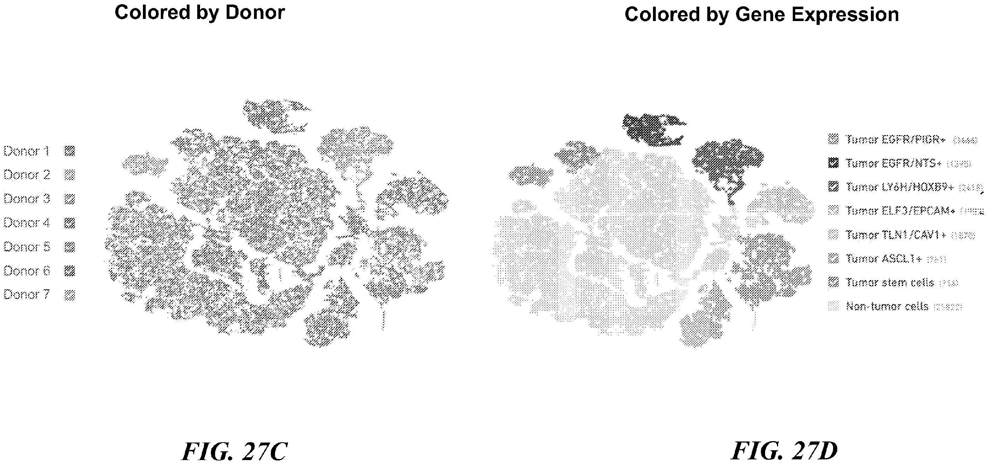

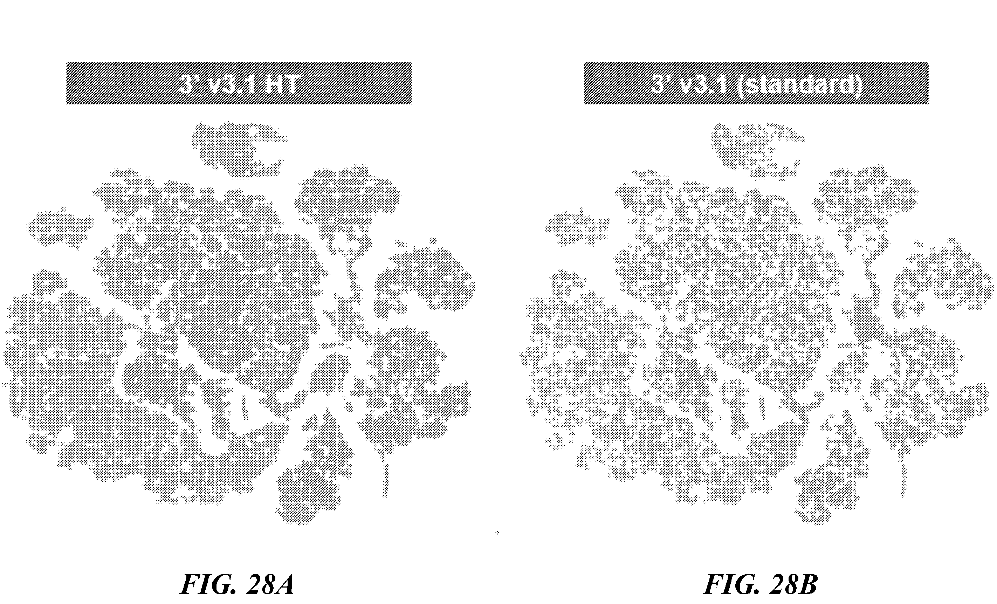

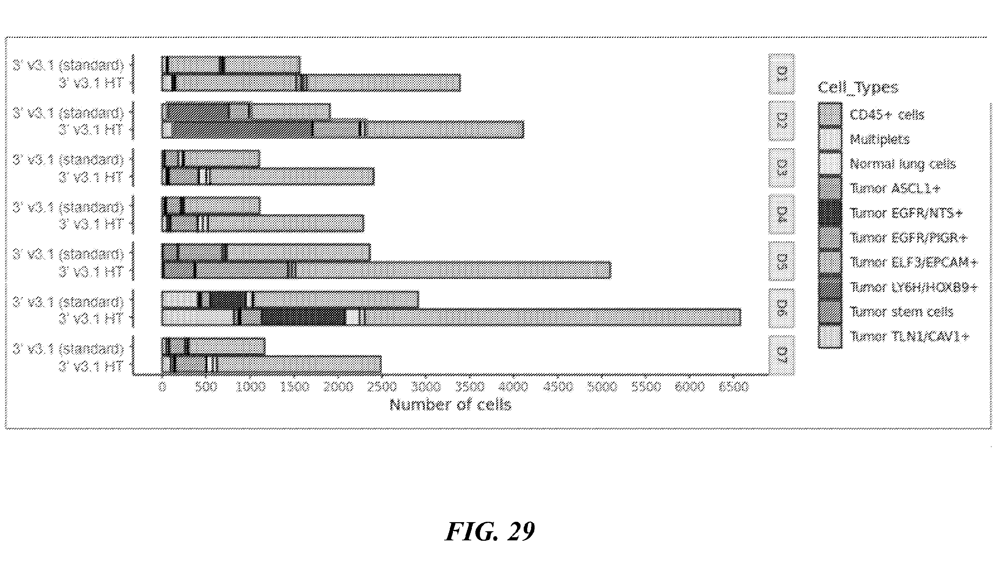

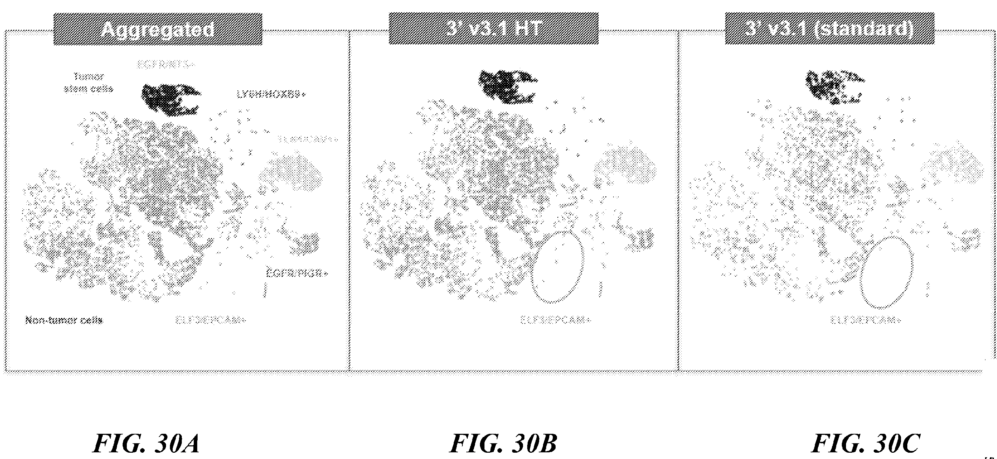

The result was that single cell libraries were generated and sequenced. **FIG. 27A** shows that cells were clustered based on CellPlex oligo labeling, with all detected multiplets removed. The seven donors resolved into separate clusters. **FIG. 27B** shows that cells were also clustered based on gene expression data and were well-resolved into discrete cell types (e.g., B cells, T cells, NK cells, monocytes, healthy lung cells, and tumor cells). The different tumor clusters could be differentiated, and specific genes upregulated in each cluster were identified using Loupe Cell Browser **(FIG. 27C-D).** Similar experiments using standard assays were unable to capture the same level of differentiation **(FIG. 28A-B).** Similarly, with the increased cell throughput per channel, rarer cell types were more easily identified than when using standard assays alone **(FIG. 29 and FIG. 30A-C).**

## Claims

1. A method for single cell drug screening comprising:

   a) contacting a first sample of cells in a first partition with a first drug to provide first treated cells;
   b) contacting a second sample of cells in a second partition with the first drug to provide second treated cells;
   c) contacting the first treated cells with first labelling molecules to generate first labelled treated cells in the first partition, wherein the first labelling molecules comprise a plurality of first barcode sequences;
   d) contacting the second treated cells with the first labelling molecules to generate second labelled treated cells in the second partition, wherein the first labelled treated cells in the first partition and the second labelled treated cells in the second partition comprise (i) a first barcode sequence of the plurality of first barcode sequences and (ii) a first plurality of cellular analytes;
   e) contacting a third sample of cells in a third partition with a second drug to provide third treated cells;
   f) contacting a fourth sample of cells in a fourth partition with the second drug to provide fourth treated cells;
   g) contacting the third treated cells with second labelling molecules to generate third labelled treated cells in the third partition, wherein the second labelling molecules comprises a plurality of second barcode sequences;
   h) contacting the fourth treated cells with the second labelling molecules to generate fourth labelled treated cells in the fourth partition, wherein the third labelled treated cells in the third partition and the fourth labelled treated cells in the fourth partition comprise (i) a second barcode sequence of the plurality of second barcode sequences and (ii) a second plurality of cellular analytes;
   i) removing the first labelled treated cells from the first partition and the third labelled treated cells from the third partition at a first time point;
   j) removing the second labelled treated cells from the second partition and the fourth labelled treated cells from the fourth partition at a second time point;
   k) pooling the first labelled treated cells from the first partition and the third labelled treated cells from the third partition to provide a first pooled sample; and
   l) pooling the second labelled treated cells from the second partition and the fourth labelled treated cells from the fourth partition to provide a second pooled sample.

2. The method of claim 1, wherein the first plurality of cellular analytes and the second plurality of cellular analytes comprise nucleic acid analytes, optionally wherein the nucleic acid analytes comprise ribonucleic acid (RNA) analytes.

3. The method of claim 1, wherein the first labelling molecules comprise a lipophilic moiety, a fluorophore, a cell-penetrating peptide, or a dye.

4. The method of claim 1, wherein:

   (a) the first pooled sample comprises (i) a first labelled treated cell from the first partition, wherein the first labelled treated cell comprises the first barcode sequence and (ii) a third labelled treated cell from the third partition, wherein the third labelled treated cell comprises the second barcode sequence; and/or
   (b) the second pooled sample comprises (i) a second labelled treated cell from the second partition, wherein the second cell comprises the first barcode sequence and (ii) a fourth labelled treated cell from the fourth partition, wherein the fourth cell comprises the second barcode sequence.

5. The method of claim 1, wherein (i) the first drug comprises a single drug or a combination of drugs, and/or (ii) the second drug comprises a single drug or a combination of drugs.

6. The method of claim 1, further comprising:

(m) contacting a fifth sample of cells in a fifth partition with a third drug to provide fifth treated cells and contacting a sixth sample of cells in a sixth partition with the third drug to provide sixth treated cells; and

(n) contacting the fifth treated cells and the sixth treated cells with third labelling molecules to generate fifth labelled treated cells in the fifth partition and sixth labelled treated cells in the sixth partition, wherein the third labelling molecules comprises a plurality of third barcode sequences, and wherein the fifth labelled treated cells in the fifth partition and the sixth labelled cells in the sixth partition comprise (i) a third barcode sequence of the plurality of third barcode sequences and (ii) a third plurality of cellular analytes.

7. The method of claim 6, further comprising:

(o) removing the fifth labelled treated cells from the fifth partition and the sixth labelled treated cells from the sixth partition at a third time point;

(p) pooling the fifth labelled treated cells from the fifth partition with the first pooled sample; and

(q) pooling the sixth labelled treated cells from the sixth partition with the second pooled sample.

8. The method of claim 1, further comprising partitioning (i) labelled treated cells from the first pooled sample and (ii) labelled treated cells from the second pooled sample into a plurality of partitions.

9. The method of claim 8, wherein the partitioning comprises partitioning into (i) a plurality of droplets or (ii) a plurality of wells.

10. The method of claim 8, wherein the plurality of partitions comprises labelled treated cells and nucleic acid barcode molecules.

11. The method of claim 8, wherein a partition of the plurality of partitions comprises a particle comprising a plurality of nucleic acid barcode molecules and a labelled treated cell.

12. The method of claim 11, wherein the particle is a bead, optionally wherein the bead is a gel bead.

13. The method of claim 11, wherein the plurality of nucleic acid barcode molecules comprises (i) partition barcode sequences specific for the partition and (ii) capture sequences configured to couple to cellular analytes, wherein the capture sequences are further configured to generate barcoded nucleic acid molecules from the cellular analytes.

14. The method of claim 13, further comprising generating a plurality of barcoded nucleic acid molecules, wherein a barcoded nucleic acid molecule of the plurality of barcoded nucleic acid molecules comprises:

i) a sequence corresponding to a cellular analyte, a partition barcode sequence, and the first barcode sequence, thereby indicating that the labelled treated cell originates from the first partition or the second partition; or

ii) a sequence corresponding to a cellular analyte, a partition barcode sequence, and the second barcode sequence, thereby indicating that the labelled treated cell originates from the third partition or the fourth partition.

15. The method of any one of claims 1-14, wherein the first sample of cells and/or the second sample of cells comprise perturbed cells.

**Patentansprüche**

1. Verfahren für eine Einzelzell-Arzneimitteluntersuchung, umfassend:

a) Inkontaktbringen einer ersten Probe von Zellen in einer ersten Partition mit einem ersten Arzneimittel, um erste behandelte Zellen bereitzustellen;

b) Inkontaktbringen einer zweiten Probe von Zellen in einer zweiten Partition mit dem ersten Arzneimittel, um zweite behandelte Zellen bereitzustellen;

c) Inkontaktbringen der ersten behandelten Zellen mit ersten Markierungsmolekülen, um erste markierte behandelte Zellen in der ersten Partition zu erzeugen, wobei die ersten Markierungsmoleküle eine Vielzahl von ersten Barcodesequenzen umfassen;

d) Inkontaktbringen der zweiten behandelten Zellen mit den ersten Markierungsmolekülen, um zweite markierte behandelte Zellen in der zweiten Partition zu erzeugen, wobei die ersten markierten behandelten Zellen in der ersten Partition und die zweiten markierten behandelten Zellen in der zweiten Partition (i) eine erste Barcodesequenz der Vielzahl von ersten Barcodesequenzen und (ii) eine erste Vielzahl von zellulären Analyten umfassen;

e) Inkontaktbringen einer dritten Probe von Zellen in einer dritten Partition mit einem zweiten Arzneimittel, um eine dritte behandelte Zelle bereitzustellen;

f) Inkontaktbringen einer vierten Probe von Zellen in einer vierten Partition mit dem zweiten Arzneimittel, um vierte behandelte Zelle bereitzustellen;

g) Inkontaktbringen der dritten behandelten Zellen mit zweiten Markierungsmolekülen, um dritte markierte behandelte Zellen in der dritten Partition zu erzeugen, wobei die zweiten Markierungsmoleküle eine Vielzahl von zweiten Barcodesequenzen umfassen;

h) Inkontaktbringen der vierten behandelten Zellen mit den zweiten Markierungsmolekülen, um vierte markierte behandelte Zellen in der vierten Partition zu erzeugen, wobei die dritten markierten behandelten Zellen in der dritten Partition und die vierten markierten behandelten Zellen in der vierten Partition (i) eine zweite Barcodesequenz der Vielzahl von zweiten Barcodesequenzen und (ii) eine zweite Vielzahl von zellulären Analyten umfassen;

i) Entfernen der ersten markierten behandelten Zellen aus der ersten Partition und der dritten markierten behandelten Zellen aus der dritten Partition zu einem ersten Zeitpunkt;

j) Entfernen der zweiten markierten behandelten Zellen aus der zweiten Partition und der vierten markierten behandelten Zellen aus der vierten Partition zu einem zweiten Zeitpunkt;

k) Vereinigen der ersten markierten behandelten Zellen aus der ersten Partition und der dritten markierten behandelten Zellen aus der dritten Partition, um eine erste vereinigte Probe bereitzustellen; und

l) Vereinigen der zweiten markierten behandelten Zellen aus der zweiten Partition und der vierten markierten behandelten Zellen aus der vierten Partition, um eine zweite vereinigte Probe bereitzustellen.

2. Verfahren nach Anspruch 1, wobei die erste Vielzahl von zellulären Analyten und die zweite Vielzahl von zellulären Analyten Nukleinsäureanalyten umfassen, wobei die Nukleinsäureanalyten optional Ribonukleinsäure(RNA)-Analyten umfassen.

3. Verfahren nach Anspruch 1, wobei die ersten Markierungsmoleküle eine lipophile Einheit, einen Fluorophor, ein zellpenetrierendes Peptid oder einen Farbstoff umfassen.

4. Verfahren nach Anspruch 1, wobei:

(a) die erste vereinigte Probe umfasst: (i) eine erste markierte behandelte Zelle aus der ersten Partition, wobei die erste markierte behandelte Zelle die erste Barcodesequenz umfasst und (ii) eine dritte markierte behandelte Zelle aus der dritten Partition, wobei die dritte markierte behandelte Zelle die zweite Barcodesequenz umfasst; und/oder

(b) die zweite vereinigte Probe umfasst: (i) eine zweite markierte behandelte Zelle aus der zweiten Partition, wobei die zweite Zelle die erste Barcodesequenz umfasst und (ii) eine vierte markierte behandelte Zelle aus der vierten Partition, wobei die vierte Zelle die zweite Barcodesequenz umfasst.

5. Verfahren nach Anspruch 1, wobei (i) das erste Arzneimittel ein einzelnes Arzneimittel oder eine Kombination von Arzneimitteln umfasst, und/oder (ii) das zweite Arzneimittel ein einzelnes Arzneimittel oder eine Kombination von Arzneimitteln umfasst.

6. Verfahren nach Anspruch 1, ferner umfassend:

(m) Inkontaktbringen einer fünften Probe von Zellen in einer fünften Partition mit einem dritten Arzneimittel, um fünfte behandelte Zellen bereitzustellen, und Inkontaktbringen einer sechsten Probe von Zellen in einer sechsten Partition mit dem dritten Arzneimittel, um sechste behandelte Zellen bereitzustellen; und

(n) Inkontaktbringen der fünften behandelten Zellen und der sechsten behandelten Zellen mit dritten Markierungsmolekülen, um fünfte markierte behandelte Zellen in der fünften Partition und sechste markierte behandelte Zellen in der sechsten Partition zu erzeugen, wobei die dritten Markierungsmoleküle eine Vielzahl von dritten Barcodesequenzen umfassen und wobei die fünften markierten behandelten Zellen in der fünften Partition und die sechsten markierten Zellen in der sechsten Partition (i) eine dritte Barcodesequenz der Vielzahl von dritten Barcodesequenzen und (ii) eine dritte Vielzahl von zellulären Analyten umfassen.

**7.** Verfahren nach Anspruch 6, ferner umfassend:

(o) Entfernen der fünften markierten behandelten Zellen aus der fünften Partition und der sechsten markierten behandelten Zellen aus der sechsten Partition zu einem dritten Zeitpunkt;
(p) Vereinigen der fünften markierten behandelten Zellen aus der fünften Partition mit der ersten vereinigten Probe; und
(q) Vereinigen der sechsten markierten behandelten Zellen aus der sechsten Partition mit der zweiten vereinigten Probe.

**8.** Verfahren nach Anspruch 1, ferner umfassend Partitionieren (i) markierter behandelter Zellen aus der ersten vereinigten Probe und (ii) markierter behandelter Zellen aus der zweiten vereinigten Probe in eine Vielzahl von Partitionen.

**9.** Verfahren nach Anspruch 8, wobei das Partitionieren ein Partitionieren in (i) eine Vielzahl von Tröpfchen oder (ii) eine Vielzahl von Vertiefungen umfasst.

**10.** Verfahren nach Anspruch 8, wobei die Vielzahl von Partitionen markierte behandelte Zellen und Nukleinsäure-Barcodemoleküle umfasst.

**11.** Verfahren nach Anspruch 8, wobei eine Partition der Vielzahl von Partitionen ein Partikel umfasst, das eine Vielzahl von Nukleinsäure-Barcodemolekülen und eine markierte behandelte Zelle umfasst.

**12.** Verfahren nach Anspruch 11, wobei das Partikel ein Kügelchen ist, optional wobei das Kügelchen ein Gelkügelchen ist.

**13.** Verfahren nach Anspruch 11, wobei die Vielzahl der Nukleinsäure-Barcodemoleküle (i) Partitions-Barcodesequenzen, spezifisch für die Partition, und
(ii) Fangsequenzen, die dazu ausgebildet sind, an zelluläre Analyten zu koppeln, umfasst, wobei die Fangsequenzen ferner dazu ausgebildet sind, barcodierte Nukleinsäuremoleküle aus den zellulären Analyten zu erzeugen.

**14.** Verfahren nach Anspruch 13, ferner umfassend Erzeugen einer Vielzahl von barcodierten Nukleinsäuremolekülen, wobei ein barcodiertes Nukleinsäuremolekül der Vielzahl von barcodierten Nukleinsäuremolekülen umfasst:

i) eine Sequenz, die einem zellulären Analyten entspricht, eine Partitions-Barcodesequenz und die erste Barcodesequenz, wodurch angegeben wird, dass die markierte behandelte Zelle aus der ersten Partition oder der zweiten Partition stammt; oder
ii) eine Sequenz, die einem zellulären Analyten entspricht, eine Partitions-Barcodesequenz und die zweite Barcodesequenz, wodurch angegeben wird, dass die markierte behandelte Zelle aus der dritten Partition oder der vierten Partition stammt.

**15.** Verfahren nach einem der Ansprüche 1 bis 14, wobei die erste Probe von Zellen und/oder die zweite Probe von Zellen gestörte Zellen umfasst.

**Revendications**

**1.** Procédé de criblage de médicament à cellule unique comprenant :

a) la mise en contact d'un premier échantillon de cellules dans un premier compartiment avec un premier médicament pour fournir des premières cellules traitées ;
b) la mise en contact d'un deuxième échantillon de cellules dans un deuxième compartiment avec le premier médicament pour fournir des deuxièmes cellules traitées ;
c) la mise en contact des premières cellules traitées avec des premières molécules d'étiquetage pour générer des premières cellules traitées étiquetées dans le premier compartiment, dans lequel les premières molécules d'étiquetage comprennent une pluralité de premières séquences de codes à barres ;
d) la mise en contact des deuxièmes cellules traitées avec les premières molécules d'étiquetage pour générer des deuxièmes cellules traitées étiquetées dans le deuxième compartiment, dans lequel les premières cellules traitées étiquetées dans le premier compartiment et les deuxièmes cellules traitées étiquetées dans le deuxième compartiment comprennent (i) une première séquence de code à barres de la pluralité de premières séquences de code à barres et (ii) une première pluralité d'analytes cellulaires ;

e) la mise en contact d'un troisième échantillon de cellules dans un troisième compartiment avec un deuxième médicament pour fournir des troisièmes cellules traitées ;

f) la mise en contact d'un quatrième échantillon de cellules dans un quatrième compartiment avec le deuxième médicament pour fournir des quatrièmes cellules traitées ;

g) la mise en contact des troisièmes cellules traitées avec des deuxièmes molécules d'étiquetage pour générer des troisièmes cellules traitées étiquetées dans le troisième compartiment, dans lequel les deuxièmes molécules d'étiquetage comprennent une pluralité de deuxièmes séquences de codes à barres ;

h) la mise en contact des quatrièmes cellules traitées avec les deuxièmes molécules d'étiquetage pour générer des quatrièmes cellules traitées étiquetées dans le quatrième compartiment, dans lequel les troisièmes cellules traitées étiquetées dans le troisième compartiment et les quatrièmes cellules traitées étiquetées dans le quatrième compartiment comprennent (i) une deuxième séquence de codes à barres de la pluralité de deuxièmes séquences de codes à barres et (ii) une deuxième pluralité d'analytes cellulaires ;

i) l'élimination des premières cellules traitées étiquetées du premier compartiment et des troisièmes cellules traitées étiquetées du troisième compartiment à un premier point temporel ;

j) l'élimination des deuxièmes cellules traitées étiquetées du deuxième compartiment et des quatrièmes cellules traitées étiquetées du quatrième compartiment à un deuxième point temporel ;

k) le regroupement des premières cellules traitées étiquetées du premier compartiment et des troisièmes cellules traitées étiquetées du troisième compartiment pour fournir un premier échantillon regroupé ; et

l) le regroupement des deuxièmes cellules traitées étiquetées du deuxième compartiment et des quatrièmes cellules traitées étiquetées du quatrième compartiment pour fournir un deuxième échantillon regroupé.

2. Procédé selon la revendication 1, dans lequel la première pluralité d'analytes cellulaires et la deuxième pluralité d'analytes cellulaires comprennent des analytes d'acide nucléique, facultativement dans lequel les analytes d'acide nucléique comprennent des analytes d'acide ribonucléique (ARN).

3. Procédé selon la revendication 1, dans lequel les premières molécules d'étiquetage comprennent un fragment lipophile, un fluorophore, un peptide pénétrant les cellules ou un colorant.

4. Procédé selon la revendication 1, dans lequel :

(a) le premier échantillon regroupé comprend (i) une première cellule traitée étiquetée provenant du premier compartiment, dans lequel la première cellule traitée étiquetée comprend la première séquence de code à barres et (ii) une troisième cellule traitée étiquetée provenant du troisième compartiment, dans lequel la troisième cellule traitée étiquetée comprend la deuxième séquence de code à barres ; et/ou

(b) le deuxième échantillon regroupé comprend (i) une deuxième cellule traitée étiquetée provenant du deuxième compartiment, dans lequel la deuxième cellule comprend la première séquence de codes à barres et (ii) une quatrième cellule traitée étiquetée provenant du quatrième compartiment, dans lequel la quatrième cellule comprend la deuxième séquence de codes à barres.

5. Procédé selon la revendication 1, dans lequel (i) le premier médicament comprend un médicament unique ou une combinaison de médicaments, et/ou (ii) le deuxième médicament comprend un médicament unique ou une combinaison de médicaments.

6. Procédé selon la revendication 1, comprenant en outre :

(m) la mise en contact d'un cinquième échantillon de cellules dans un cinquième compartiment avec un troisième médicament pour fournir des cinquièmes cellules traitées et la mise en contact d'un sixième échantillon de cellules dans un sixième compartiment avec le troisième médicament pour fournir des sixièmes cellules traitées ; et

(n) la mise en contact des cinquièmes cellules traitées et des sixièmes cellules traitées avec des troisièmes molécules d'étiquetage pour générer des cinquièmes cellules traitées étiquetées dans le cinquième compartiment et des sixièmes cellules traitées étiquetées dans le sixième compartiment, dans lequel les troisièmes molécules d'étiquetage comprennent une pluralité de troisièmes séquences de codes à barres, et dans lequel les cinquièmes cellules traitées étiquetées dans le cinquième compartiment et les sixièmes cellules étiquetées dans le sixième compartiment comprennent (i) une troisième séquence de codes à barres de la pluralité de troisièmes séquences de codes à barres et (ii) une troisième pluralité d'analytes cellulaires.

**7.** Procédé selon la revendication 6, comprenant en outre :

(o) l'élimination des cinquièmes cellules traitées étiquetées du cinquième compartiment et des sixièmes cellules traitées étiquetées du sixième compartiment à un troisième point temporel ;
(p) le regroupement des cinquièmes cellules traitées étiquetées provenant du cinquième compartiment avec le premier échantillon regroupé ; et
(q) le regroupement des sixièmes cellules traitées étiquetées provenant du sixième compartiment avec le deuxième échantillon regroupé.

**8.** Procédé selon la revendication 1, comprenant en outre le compartimentage (i) des cellules traitées étiquetées provenant du premier échantillon regroupé et (ii) des cellules traitées étiquetées provenant du deuxième échantillon regroupé en une pluralité de compartiments.

**9.** Procédé selon la revendication 8, dans lequel le compartimentage comprend le compartimentage en (i) une pluralité de gouttelettes ou (ii) une pluralité de puits.

**10.** Procédé selon la revendication 8, dans lequel la pluralité de compartiments comprend des cellules traitées étiquetées et des molécules de code à barres d'acide nucléique.

**11.** Procédé selon la revendication 8, dans lequel un compartiment de la pluralité de compartiments comprend une particule comprenant une pluralité de molécules de code à barres d'acide nucléique et une cellule traitée étiquetée.

**12.** Procédé selon la revendication 11, dans lequel la particule est une bille, facultativement dans lequel la bille est une bille de gel.

**13.** Procédé selon la revendication 11, dans lequel la pluralité de molécules de code à barres d'acide nucléique comprend (i) des séquences de code à barres de compartiment spécifiques pour le compartiment et (ii) des séquences de capture configurées pour se coupler à des analytes cellulaires, dans lequel les séquences de capture sont en outre configurées pour générer des molécules d'acide nucléique codées par code à barres à partir des analytes cellulaires.

**14.** Procédé selon la revendication 13, comprenant en outre la génération d'une pluralité de molécules d'acide nucléique codée par code à barres, dans lequel une molécule d'acide nucléique codée par code à barres de la pluralité de molécules d'acide nucléique codées par code à barres comprend :

i) une séquence correspondant à un analyte cellulaire, une séquence de code à barres de compartiment, et la première séquence de code à barres, indiquant ainsi que la cellule traitée étiquetée provient du premier compartiment ou du deuxième compartiment ; ou
ii) une séquence correspondant à un analyte cellulaire, une séquence de code à barres de compartiment, et la deuxième séquence de code à barres, indiquant ainsi que la cellule traitée étiquetée provient du troisième compartiment ou du quatrième compartiment.

**15.** Procédé selon l'une quelconque des revendications 1 à 14, dans lequel le premier échantillon de cellules et/ou le deuxième échantillon de cellules comprennent des cellules perturbées.

FIG. 1

FIG. 2

**FIG. 3**

FIG. 4

**FIG. 5**

FIG. 6

FIG. 7

FIG. 8

FIG. 9A

FIG. 9B

*FIG. 9C*

**FIG. 10**

FIG. 11

FIG. 12

*FIG. 13*

*FIG. 14*

*FIG. 15*

FIG. 16

**GEM Generation**

Partitioning Oil in Well 3A

Partitioning Oil in Well 3B

*FIG. 17*

FIG. 18

FIG. 19A

FIG. 19B

*FIG. 20*

FIG. 21

FIG. 22

EP 4 298 239 B1

FIG. 23

**FIG. 24A**          **FIG. 24B**

FIG. 25

*FIG. 26*

**CellPlex**

**Gene Expression**

Donor 1
Donor 2
Donor 3
Donor 4
Donor 5
Donor 6
Donor 7

Tumor cells
B cells
Basophil/Mast
Bronchial Vessel
T cells
Ciliated
Dendritic Conventional
Macrophage
NK
Plasma
Dendritic Plasmacytoid
Alveolar Epithelial
Monocytes

*FIG. 27A*

*FIG. 27B*

**Colored by Donor**

**Colored by Gene Expression**

Donor 1
Donor 2
Donor 3
Donor 4
Donor 5
Donor 6
Donor 7

Tumor EGFR/PIGR+
Tumor EGFR/NTS+
Tumor LY6H/HOXB9+
Tumor ELF3/EPCAM+
Tumor TLN1/CAV1+
Tumor ASCL1+
Tumor stem cells
Non-tumor cells

*FIG. 27C*

*FIG. 27D*

3' Vδ1 HT

*FIG. 28A*

3' Vδ1 (standard)

*FIG. 28B*

*FIG. 29*

*FIG. 30A*　　　　　　*FIG. 30B*　　　　　　*FIG. 30C*

EP 4 298 239 B1

FIG. 31A

FIG. 31B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2020002764 A
- WO 2019113533 A
- WO 2020167862 A1
- US 20190367997 A
- US 20190064173 A
- US 20140155295
- US 20100105112
- US 20140378345
- WO 2019165181 A1
- US 20150292988 A
- US 20140378345 A

- US 20150376609 A
- US 10550429 B
- US 20190177800 A
- US 20190367969 A
- US 20190323088 A
- US 6265552 B
- US 20180105808 A
- WO 2018075693 A1
- US 10428326 B
- US 20190100632 A

### Non-patent literature cited in the description

- **HEATH et al.** *Nature Reviews Drug Discovery*, 2015, vol. 15 (3), 204-216
- **SHEMBEKAR et al.** *Lab On A Chip*, January 2016, vol. 16 (8), 1313-1331
- **HUGHES L D et al.** *PLoS One*, 04 February 2014, vol. 9 (2), e87649

- **FANG et al.** Fluoride-Cleavable Biotinylation Phosphoramidite for 5'-end-Labelling and Affinity Purification of Synthetic Oligonucleotides. *Nucleic Acids Res.*, 15 January 2003, vol. 31 (2), 708-715